# EUROPEAN PATENT SPECIFICATION

(11) **EP 3 921 423 B1**
(45) Date of publication and mention of the grant of the patent: **04.01.2023**
(21) Application number: 20706944.4
(22) Date of filing: 06.02.2020
(51) Int. Cl.: C12N 15/65

(54) **METHOD FOR DETECTING A SPECIFIC SPLICE EVENT OF A GENE OF INTEREST**
VERFAHREN ZUM NACHWEIS EINES SPEZIFISCHEN SPLEISSEREIGNISSES EINES INTERESSIERENDEN GENS
PROCÉDÉ DE DÉTECTION D'UN ÉVÉNEMENT D'ÉPISSAGE SPÉCIFIQUE D'UN GÈNE D'INTÉRÊT

(30) Priority: 06.02.2019 LU 101118
(43) Date of publication of application: 15.12.2021
(73) Proprietor: Helmholtz Zentrum München - Deutsches Forschungszentrum für Gesundheit und Umwelt (GmbH), 85764 Neuherberg (DE); Klinikum rechts der Isar der Technischen Universität München, 81675 München (DE)
(72) Inventor: WESTMEYER, Gil Gregor, 80804 München (DE); TRUONG, Dong-Jiunn Jeffery, 85356 Freising (DE); WURST, Wolfgang, 80937 München (DE)
(74) Representative: Schiweck Weinzierl Koch Patentanwälte Partnerschaft mbB
(86) International application number: PCT/EP2020/052985
(87) International publication number: WO 2020/161236

(56) References cited:
- WO-A2-2017/132580
- Radhika Borra ET AL: "Protein Chemical Modification Inside Living Cells Using Split Inteins" In: "Methods in Molecular Biology", 7 October 2016 (2016-10-07), Humana Press, Inc., US, XP055574780, ISSN: 1064-3745 vol. 1495, pages 111-130, DOI: 10.1007/978-1-4939-6451-2_8, the whole document
- DONG-JIUNN JEFFERY TRUONG ET AL: "Development of an intein-mediated split-Cas9 system for gene therapy", NUCLEIC ACIDS RESEARCH, INFORMATION RETRIEVAL LTD, vol. 43, no. 13, 1 January 2015 (2015-01-01), pages 6450-6458, XP002758945, ISSN: 0305-1048, DOI: 10.1093/NAR/GKV601 [retrieved on 2015-06-16]
- JOSEF A. GRAMESPACHER ET AL: "Improved protein splicing using embedded split inteins : PTS Using Inteins", PROTEIN SCIENCE, vol. 27, no. 3, 17 January 2018 (2018-01-17), pages 614-619, XP055574553, US ISSN: 0961-8368, DOI: 10.1002/pro.3357
- ADAM J. STEVENS ET AL: "Design of a Split Intein with Exceptional Protein Splicing Activity", JOURNAL OF THE AMERICAN CHEMICAL SOCIETY, vol. 138, no. 7, 8 February 2016 (2016-02-08), pages 2162-2165, XP055421171, ISSN: 0002-7863, DOI: 10.1021/jacs.5b13528

## Description

### TECHNICAL FIELD OF THE INVENTION

The present invention provides a method for detecting a specific splice event of a gene of interest, wherein the specific splice event creates a specific splice product, which comprises an exon of interest, wherein the method comprises inserting a split intein - heterologous polynucleotide construct into the exon of interest, wherein the split intein comprises an N-terminal splicing region upstream of the heterologous polynucleotide and a C-terminal splicing region downstream of the heterologous polynucleotide; and detecting the heterologous polynucleotide and/or the expression product of the heterologous polynucleotide, wherein the expression product of the split intein - heterologous polynucleotide construct excises itself from the expression product of the specific splice product at a position, wherein the amino acid C-terminal to this position is a cysteine, a serine or a threonine. Further, the present invention comprises the use of the split intein - heterologous polynucleotide construct in any of the methods of the present invention, a nucleic acid encoding the split intein - heterologous polynucleotide construct, a host cell comprising the nucleic acid, a vector comprising the nucleic acid or the vector, and a kit for detecting specific splice events.

### BACKGROUND ART

Approximately 86% of all human genes encode more than one protein isoform due to alternative pre-mRNA splicing, making this phenomenon the major source of protein diversity (Wang, Sandberg *et al*.). Moreover, more than 60% of disease-relevant mutations affect alternative splicing (AS) of the pre-mRNA rather than its impact on the coding sequence (Lopez-Bigas *et al*.). As a result, these mutations that affect sequences involved in the splicing mechanism and regulation can evoke severe diseases, of which a lot are of neurological or neuromuscular origin, such as chromosome-linked Parkinson's disease and Spinal Muscular Atrophy (Daguenet *et al*.). For over a decade, research has been conducted to understand and target the splicing machinery and regulators of AS for the development of new therapeutics, e.g., antisense oligonucleotides (ASOs) (Wurster *et al*.) that bind splice enhancer or suppressor sequences, or small molecules targeting splicing factor (Luo *et al*.). Analysis of the effects of such drugs relies to date on laborious methods based on reverse transcription followed by quantitative PCR (RT-qPCR), immunoblotting with isoform-specific antibodies, or in some cases luminescent/ fluorescent protein-fusion based assays and minigene-constructs (Zhang *et al.,* Deshpande *et al.,* Stoilov *et al.,* and Porensky *et al*.)*.* All those methods have limited spatiotemporal resolution and cannot be used for real-time tracking. For multi-cell approaches that serve fundamental research, consumptive end-point methods like mRNA fluorescent-in-situ-hybridization (FISH) are state of the art.

Moreover, RNA-based methods do not always represent the presence of the protein since mRNA may also exist in a translationally-arrested state, e.g., in RNA bodies, stress granules and P-bodies (Anderson *et al*.).

WO 2017/091630 deals with tracking and manipulating cellular RNA via nuclear delivery of CRISPR/CAS9, however, does not track or detect any specific splice event.

WO 2013/045632 describes split inteins and the use thereof, wherein those split inteins are active over a certain temperature range, including temperatures as low as 0 °C, over a certain pH range, and in the presence of chaotropic salts.

WO 2013/158309 deals with non-disruptive gene targeting, providing compositions and methods for integrating one or more genes of interest into cellular DNA without substantially disrupting the expression of the gene at the locus of integration, i.e. the target locus.

Licatalosi *et al.* describes that defects in regulation of splicing may underlie many types of human neurologic diseases. This is also outlined by Poulos *et al.*

Instead, the inventors of the present invention developed a minimally-invasive toolkit based on recently identified fast protein-splicing inteins, allowing tracking of RNA-splicing events in a high-throughput manner and with spatiotemporal resolution.

Thus, the insertion of an intein-flanked reporter protein/ enzyme in proteins enables tracking of alternatively spliced protein isoforms with repeated measurements over time so that monitoring over time is possible. Most importantly, only actively translated mRNA will be detected, excluding those that are in an arrested state.

The information obtained from monitoring the splicing event can also be made useable by the cell as input to a genetically encoded computation that may also result in altered cellular processes including processes that may manipulate the splicing event itself that may be associated with an undesired or pathological state.

One of the major and socially most relevant diseases are the tauopathies associated with an imbalance of tau protein isoforms provoking a different kind of symptoms as it is observable, i.e., in Alzheimer's and Parkinson's disease. Protein tau is normally unfolded and highly soluble, which is mainly expressed in neuronal cells (Bolós *et al.,* and Fitzpatrick *et al.*)*.* Phosphorylated tau binds and supports cytoskeletal microtubules and regulates the stability of assembled and β-tubulin (Ballatore *et al.,* and Lathuilière *et al.*)*.* Point-mutations in the MAPT gene that affect the pre-mRNA splicing evoke an imbalance in isoform distribution (Goedert *et al.*)*.* A higher expression does not necessarily lead to pathological occurrences but, in combination with genetic disorders, it increases the probability of tau aggregation in neurons conducting the slow degeneration of cerebral tissue. Individuals carrying the MAPT H1 haplotype instead of H2 show higher efficiency at driving gene expression and therefore higher susceptibility to develop idiopathic forms of Parkinson's disease (Kwok *et al.*)*.* Mutations in splice-silencing and splice-enhancing or even directly in splice donor or splice acceptor sites give rise to unregulated splicing events. In-trans acting factor, on the other hand, may also conduct dysregulation. Such factor can, for example, represent snRNPs (a complex-assembly between proteins and snRNAs) or other non-snRNP associated factors to form the committed complex on the pre-mRNA.

Before evolving potential therapies to counteract AS dysregulation and protein isoform dysbalance, it is even more important to hold a highly sensitive diagnostic tool to detect early-stage diseases and intervene as soon as possible.

The present invention aims at and adresses these needs.

### SUMMARY OF THE INVENTION

The above mentioned problems are solved by the subject-matter as defined in the claims and as defined herein.

The invention provides a method for detecting a specific splice event of a gene of interest, wherein the specific splice event creates a specific splice product, which comprises an exon of interest, wherein the method comprises:
- (i) Inserting a split intein - heterologous polynucleotide construct into the exon of interest, wherein the split intein comprises an N-terminal splicing region upstream of the heterologous polynucleotide and a C-terminal splicing region downstream of the heterologous polynucleotide;
   and
- (ii) detecting the heterologous polynucleotide and/or the expression product of the heterologous polynucleotide,
wherein the expression product of the split intein - heterologous polynucleotide construct excises itself from the expression product of the specific splice product at a position, wherein the amino acid C-terminal to this position is a cysteine, a serine or a threonine.

The present invention also provides the use of a split intein - heterologous polynucleotide construct as defined herein, wherein the split intein comprises an N-terminal splicing region upstream of the heterologous polynucleotide and a C-terminal splicing region downstream of the heterologous polynucleotide, in any of the methods according to the present invention as described herein.

The present invention also provides a nucleic acid encoding a split intein - heterologous polynucleotide construct as described herein, wherein the split intein comprises an N-terminal splicing region upstream of the heterologous polynucleotide and a C-terminal splicing region downstream of the heterologous polynucleotide.

Further, the present invention also comprises a vector comprising the nucleic acid according to the present invention.

The present invention further provides a host cell comprising the nucleic acid according to the present invention or the vector according to the present invention as described herein.

The present invention also comprises the use of the nucleic acid, the vector or the host cell according to the present invention as described herein for detecting specific splice events.

Further, the present invention provides the nucleic acid, the vector or the host cell according to the present invention as described herein, for use in the treatment or prevention of a disease, wherein the disease is preferably selected from the group consisting of retinopathies, tauopathies, motor neuron diseases, muscular diseases, neurodevelopmental and neurodegenerative diseases, more preferably from the group consisting of cystic fibrosis, retinitis pigmentosa, myotonic dystrophy, Alzheimer's disease and Parkinson's disease.

The present invention further provides a kit for detecting a specific splice event of a gene of interest, which comprises:
- a first plasmid, wherein a split intein-heterologous polynucleotide construct is inserted and wherein the split intein comprises an N-terminal splicing region upstream of the heterologous polynucleotide and a C-terminal splicing region downstream of the heterologous polynucleotide;
- a second plasmid coding for a guided endonuclease, preferably wherein the endonuclease is selected from the group consisting of Cas9, Cas12a, TALENs, ZFNs and meganucleases;
   and
- a third plasmid encoding for Cre/Flp recombinases.

### BRIEF DESCRIPTION OF THE DRAWINGS

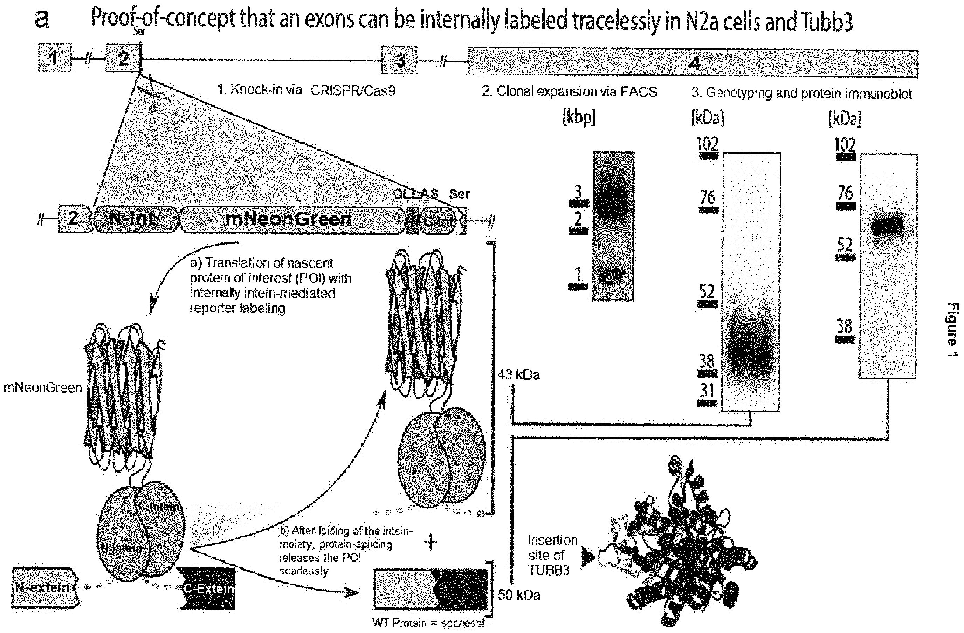

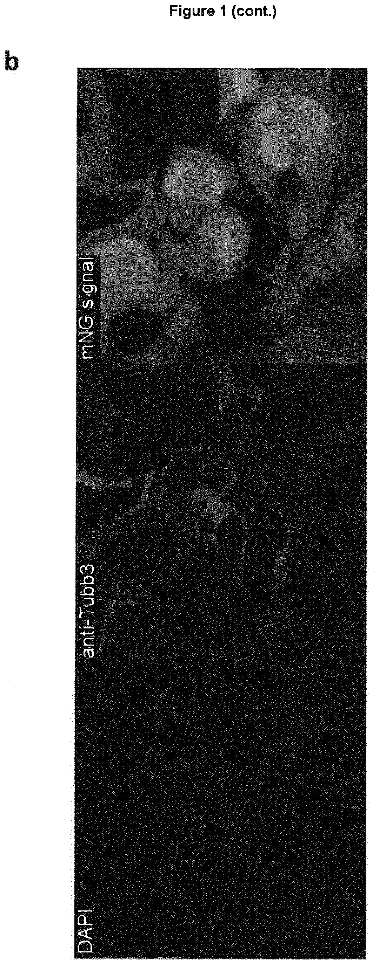

**Figure 1****: Non-invasive exon tagging of Tubb3 in mouse N2a cells using a intein-flanked fluorescent protein.** **Figure 1a** shows the general concept of a minimally-invasive exon tagging system using ultrafast split-inteins inserted into an exon-of-interest (EOI). By using CRISPR/Cas9, the inventors inserted mNeonGreen (mNG) flanked by an N- and C-intein into the second coding exon of mouse Tubb3 gene. After transcription and translation, mNG is posttranslationally spliced out via the flanking split-intein moieties and the remaining exteins are ligated scarlessly. **Figure 1a** also shows that genotyping indicates a successful insertion of intein-mNG (~ 2.8 kbp) with one modified Tubb3 allele (1.1 kbp, WT allele would be 1.6 kbp). **Figure 1a** also shows that immunoblot analysis confirms successful intein splicing of mNG and ligation of the remaining exteins. **Figure 1b** shows that a mouse N2a cell line with the insertion of the intein-mNG reporter into Tubb3 shows typical Tubb3 filaments (middle) indicating functional Tubb3. A fluorescent signal is observed throughout the cell and nucleus indicating successful post-translational splicing of the intein-flanked mNeonGreen.

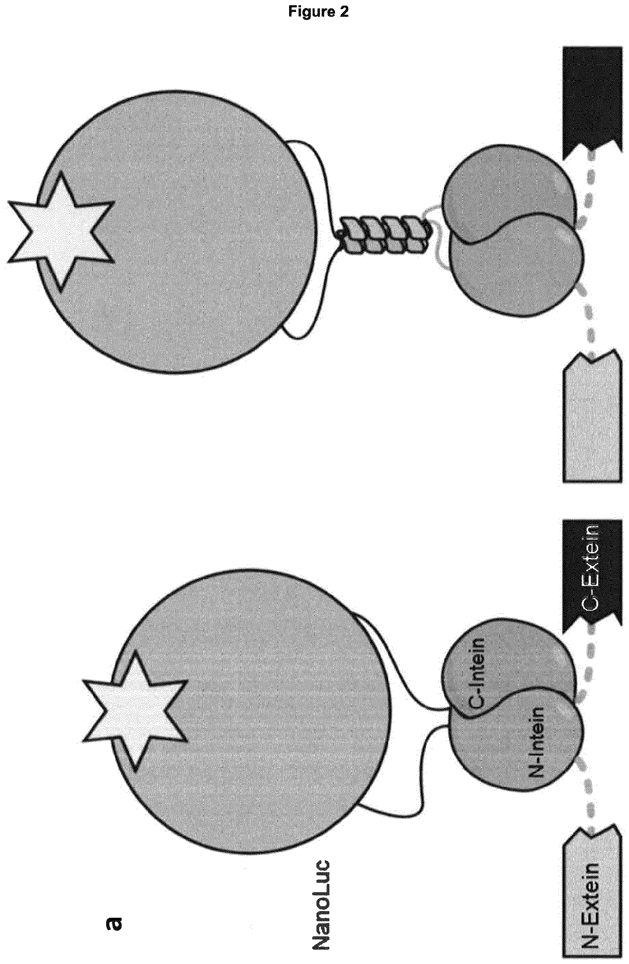

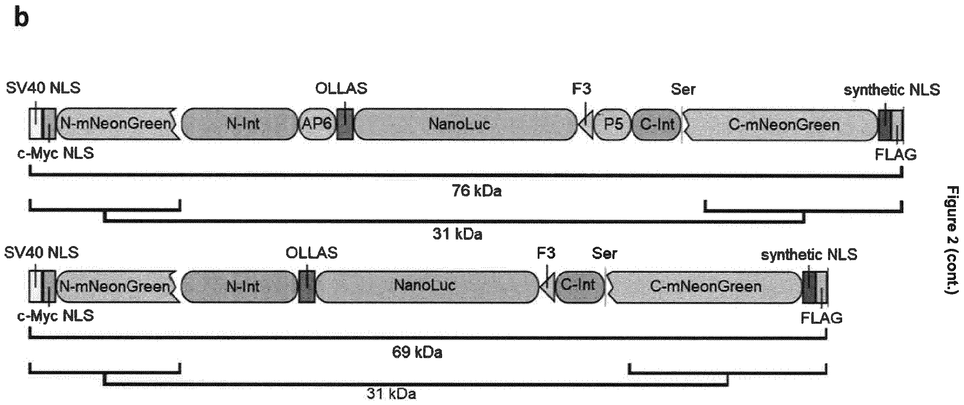

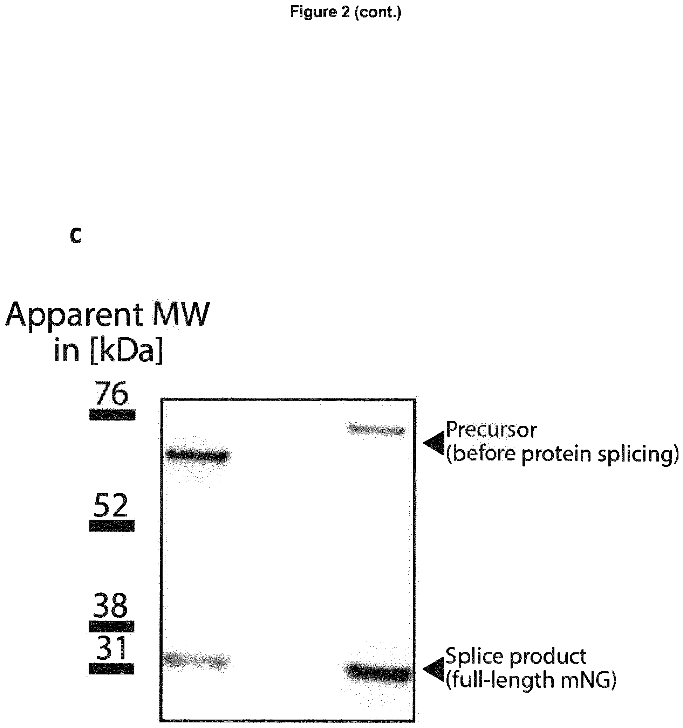

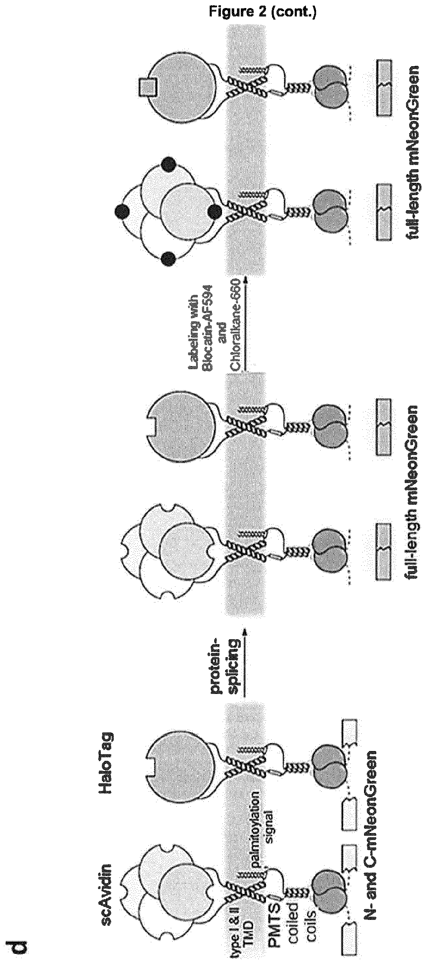

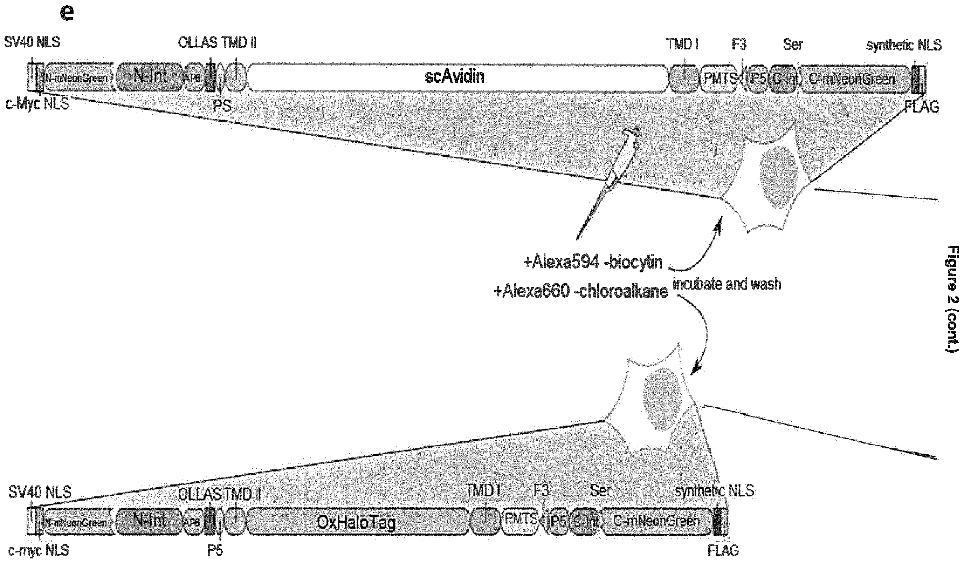

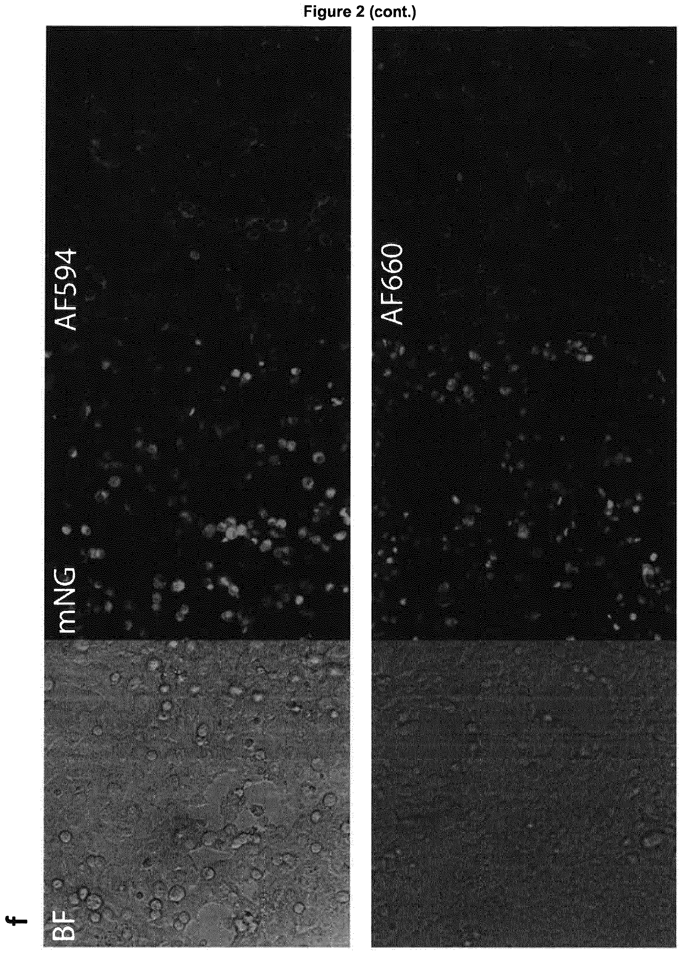

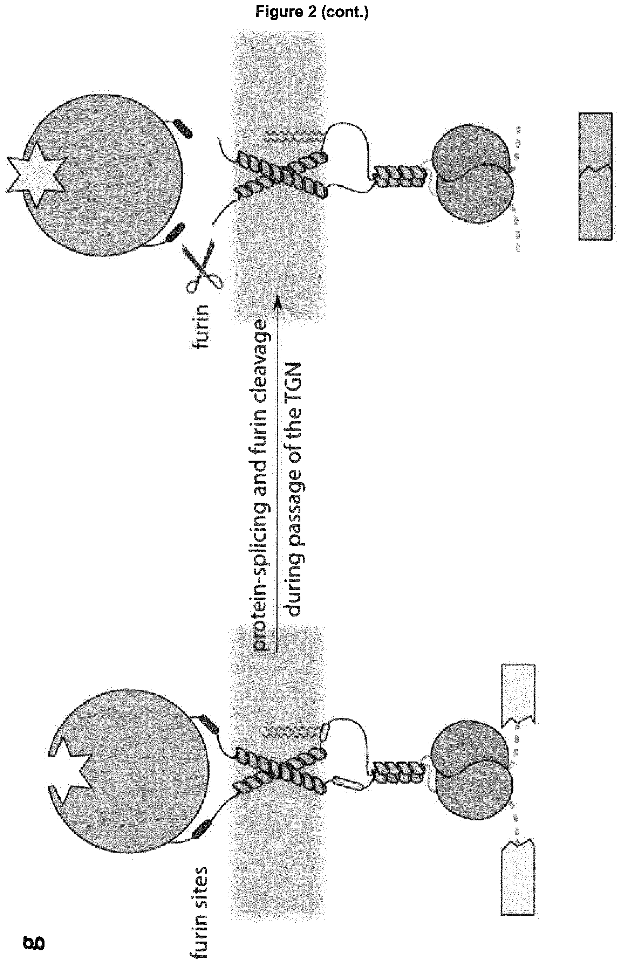

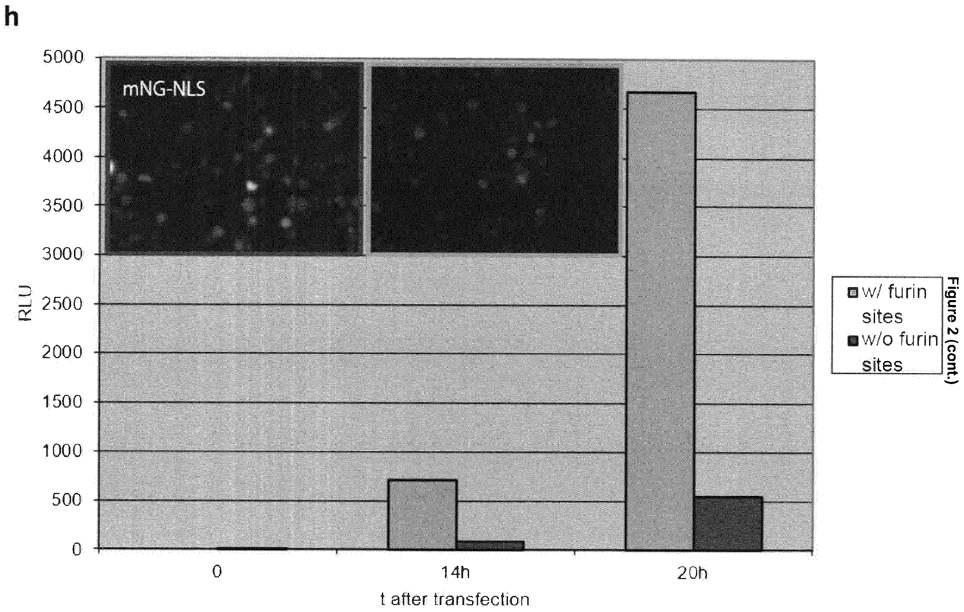

**Figure 2****: Design of an intein-mediated scarless exon-tagging system.** **Figure 2a** and Figure 2b show schematically an example of an intein-mediated scarless reporter enzyme/ protein based on N- and C-mNeonGreen as exteins with and without coiled-coils to increase efficiency of intein-splicing. **Figure 2c** shows that cells were transfected with the respective constructs shown schematically in **Figure 2a** and that protein-splicing efficiency was measured via anti-FLAG immunoblot where the higher MW band indicates the pre-protein-splicing educt and the lower MW band indicates the post-protein-splicing product. **Figure 2d** shows that two strategies were followed using single-chain avidin (scAvidin) and HaloTag as cell-surface markers. Exon-dependent membrane presentation of the binding moiety was achieved similarly as before using intein-coiled-coils and additionally using type II and type I transmembrane domains with an inserted surface marker. **Figure 2e** shows that the aforementioned constructs were flanked with N- and C-mNeonGreen as replacement exteins. After transfection of the test-constructs, cells were labeled with either biocytin-AF594 or chloroalkane-AF660 to check for cell surface functionalization. **Figure 2f** shows membrane-staining with AF594-biocytin and AF660-chloralkane for the corresponding binding moieties scAvidin and HaloTag, which shows successful membrane labeling of the cells transfected with the indicated constructs from **Figure 2e****.** Halotag-construct-transfected cells were only positive for 4F660 and *vice versa.* Uncoupled intracellular mNeonGreen fluorescence signal indicates successful protein ligation of the N- and C-mNG resulting in full-length mNeonGreen formation. **Figure 2g** shows how to enable a non-consumptive monitoring of isoform-specific expression, the binding moiety of **Figure 2D** was changed to a Nanoluc luciferase including flanking furin cleavage sites. Upon translocation of the extracellular section into the ER and passing the trans-Golgi-network, the furin-site flanked Nanoluc is released into the extracellular site. **Figure 2h** shows the Nanoluc signal in the supernatant of cells transfected with constructs from **Figure 2h** with and without furin cleavage sites after indicated time after transfection. Inlet shows the nuclear-localized mNeonGreen after excision of the intein-embedded reporter.

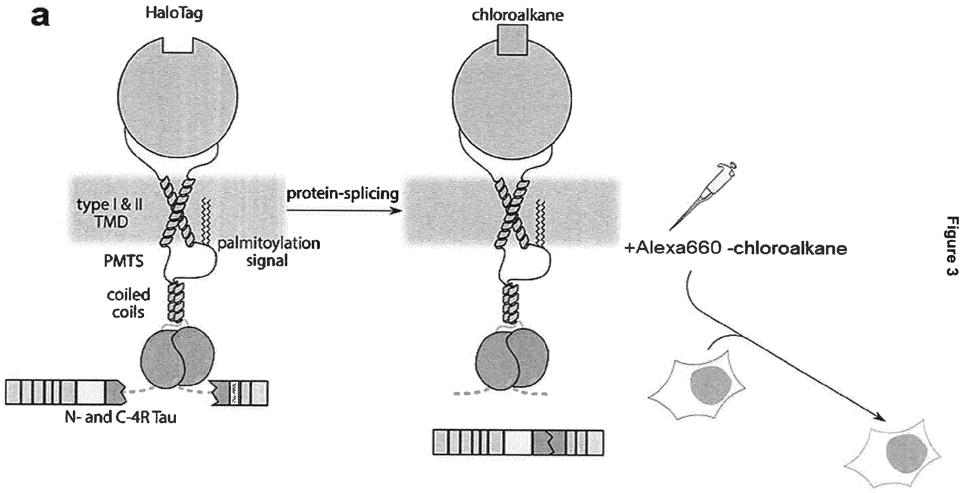

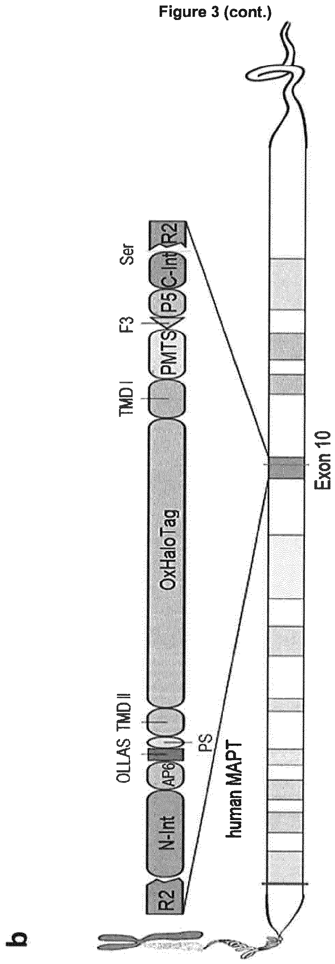

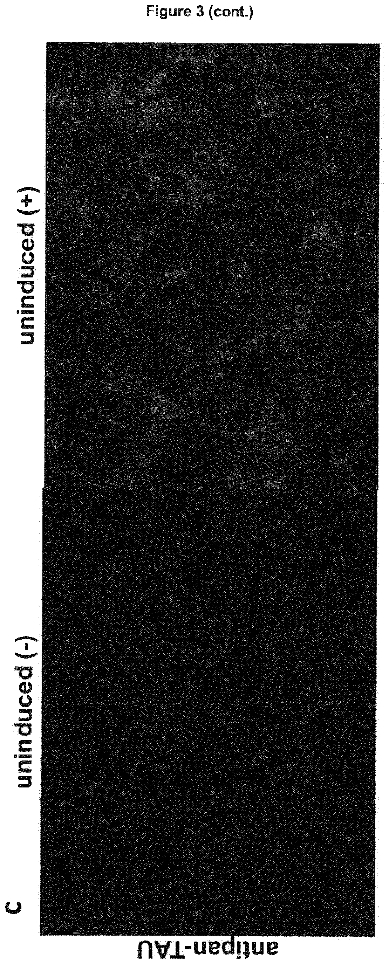

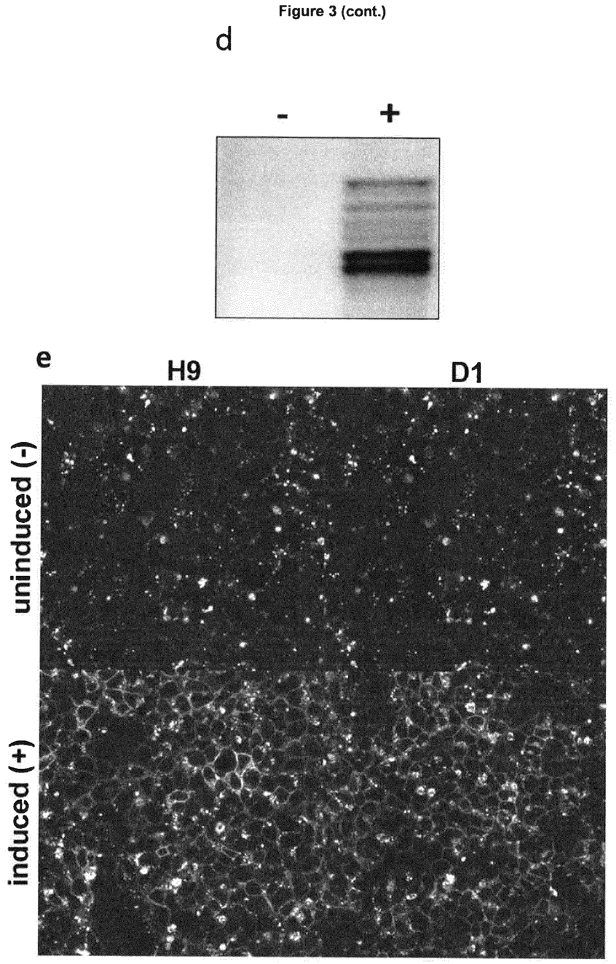

**Figure 3****: Design of an exon-dependent scarless HaloTag-presenting system.** **Figure 3a** shows that an exon-dependent membrane presentation of HaloTag was achieved by insertion of type II and type I transmembrane domains with the surface marker in between within the split-inteins-flanked coiled coils. **Figure 3b** shows a proof-of-concept experiment performed again by targeting MAPT exon 10. **Figure 3c** shows that RNA-guided MAPT induction was achieved again via dCas9-NLS-VPR and anti-pan-TAU staining showed clear TAU staining for the induced condition. In **Figure 3d****,** anti-pan-TAU immunoblot analysis shows all six adult TAU isoforms indicating again the scarless nature of exon tagging. And, also in Figure 2D, 4F660 live-cell-staining showed covalent membrane staining only in the MAPT-induced condition. It can also directly be compared to mNeonGreen with cc - in FACS and fluorescence intensity.

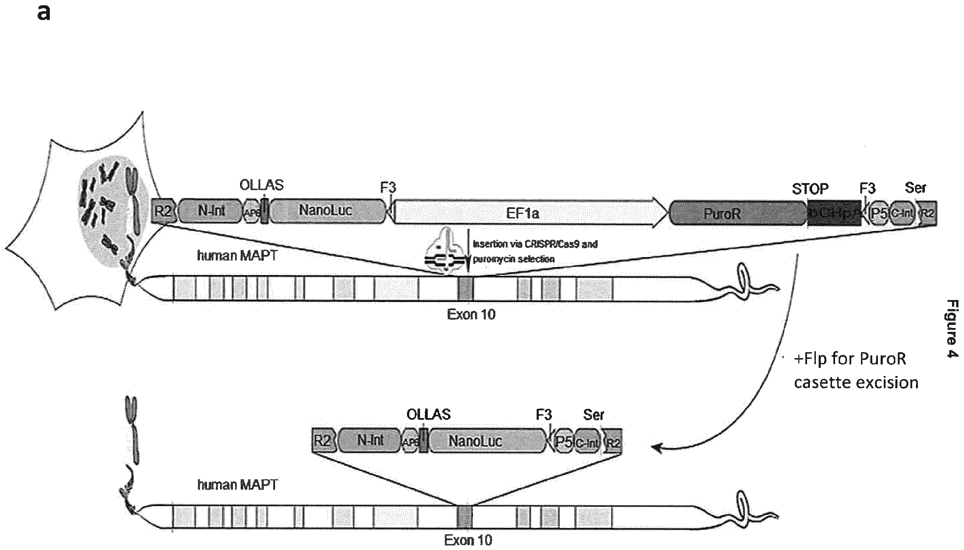

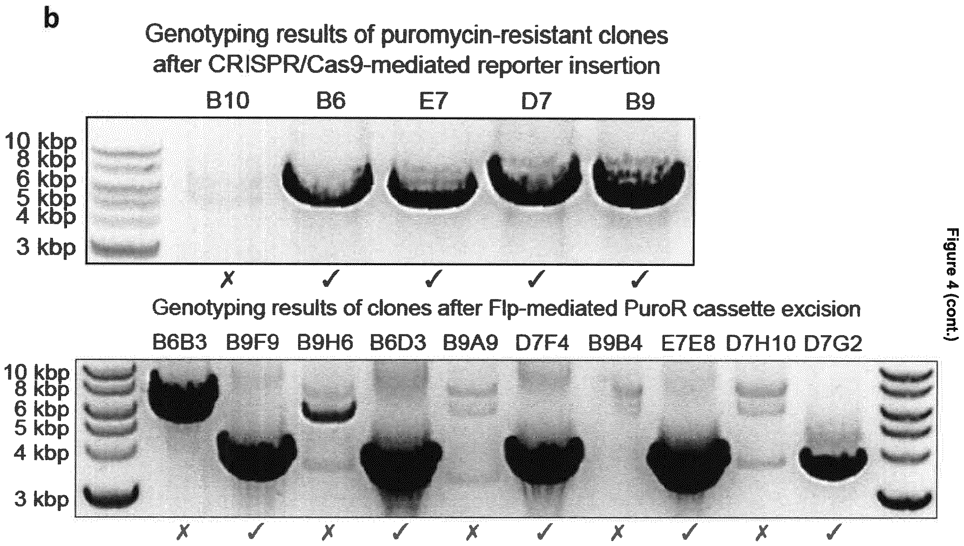

**Figure 4****: Schemata of CRISPR/Cas9-mediated knock-in of the intein-based reporter.** **Figure 4a** shows the FRT-F3-(Flp recombinase site)-flanked puromycin-resistance-cassette was inserted into the intein-flanked reporter via CRISPR/Cas9 targeting exon 10. **Figure 4b** shows that clones were individually tested for puromycin sensitivity after Flp step and revealed that B9F9, D7F4 and E7E8 was completely removed, unexpectedly D7G2 was still resistant even though its genotyping was positive and was not further used.

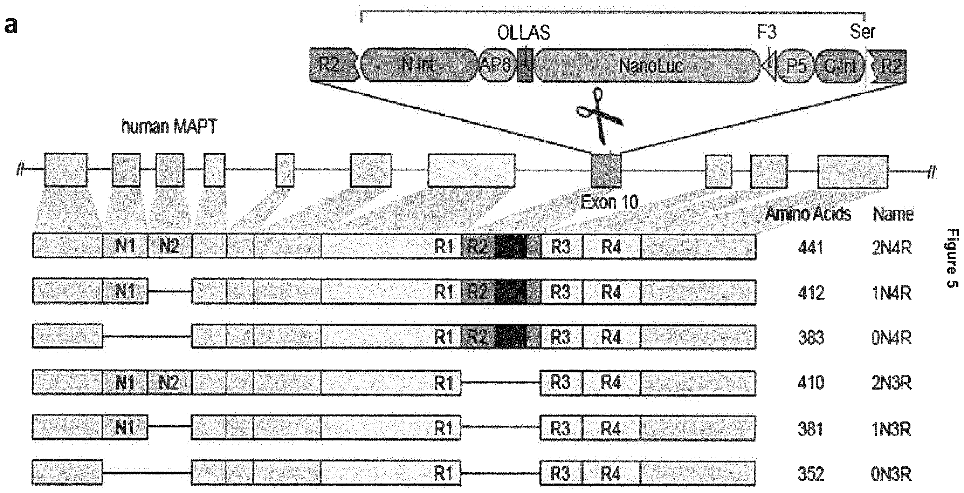

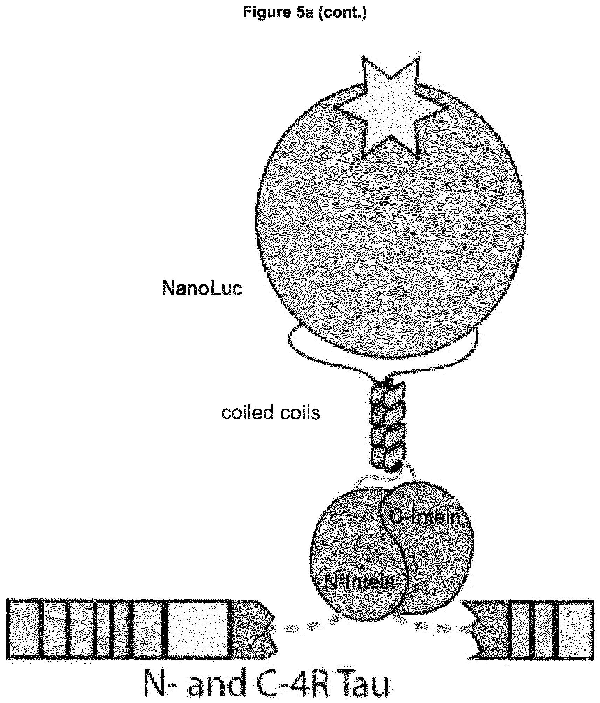

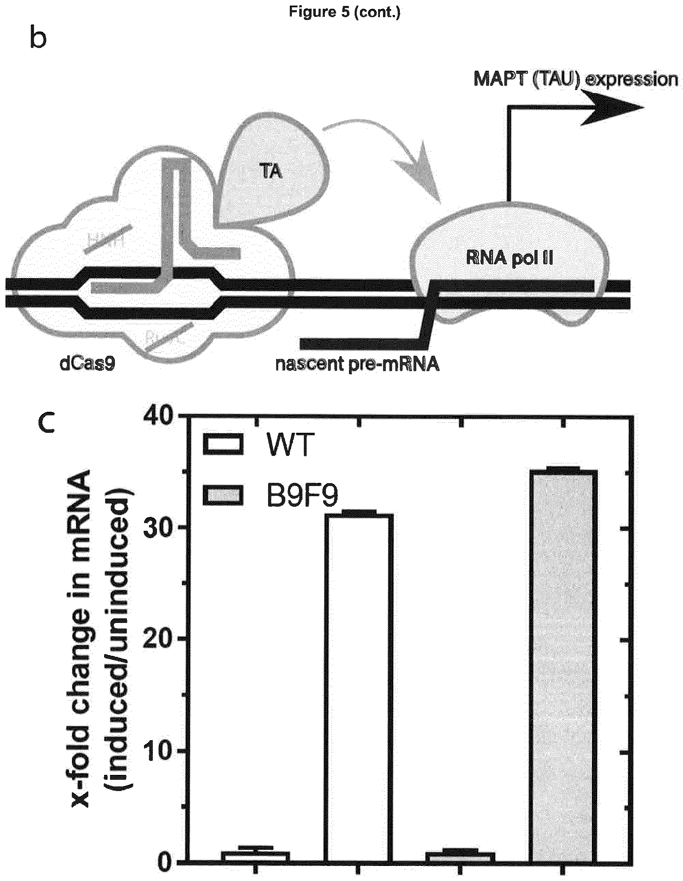

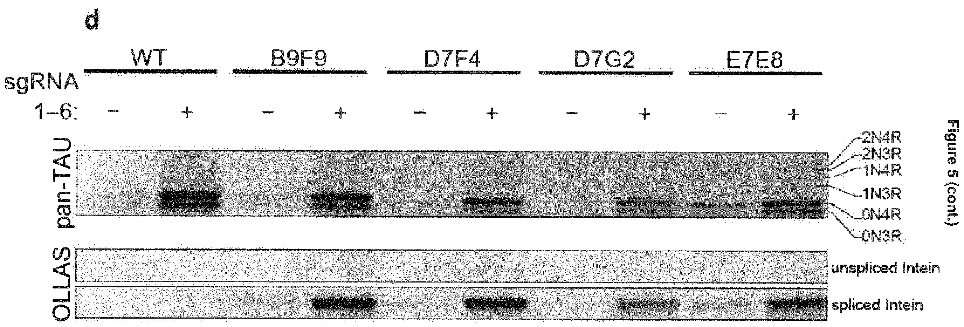

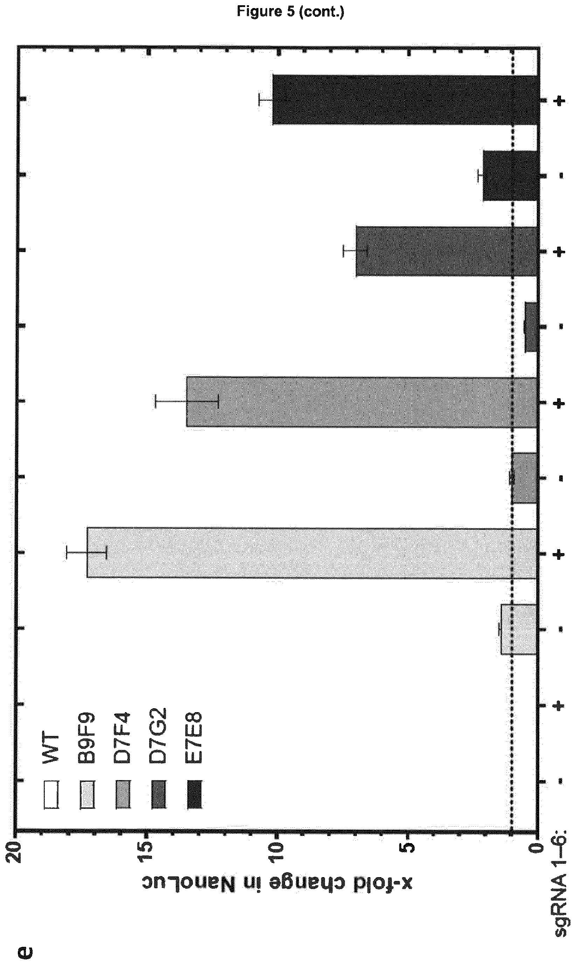

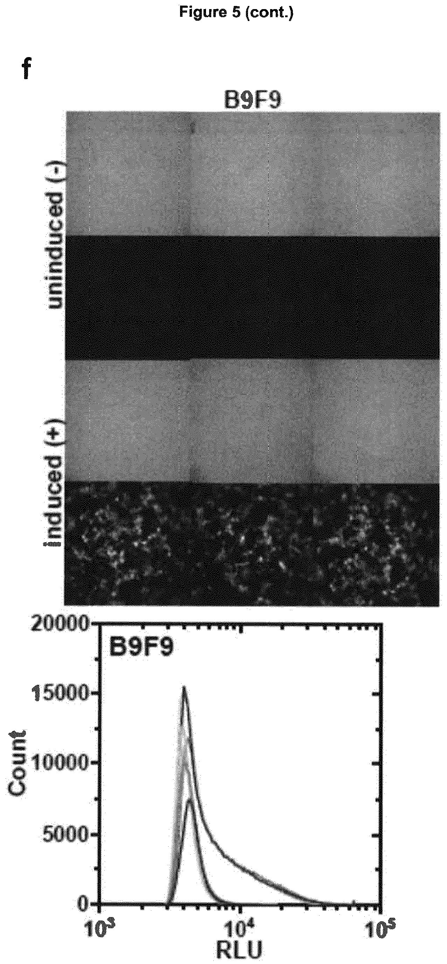

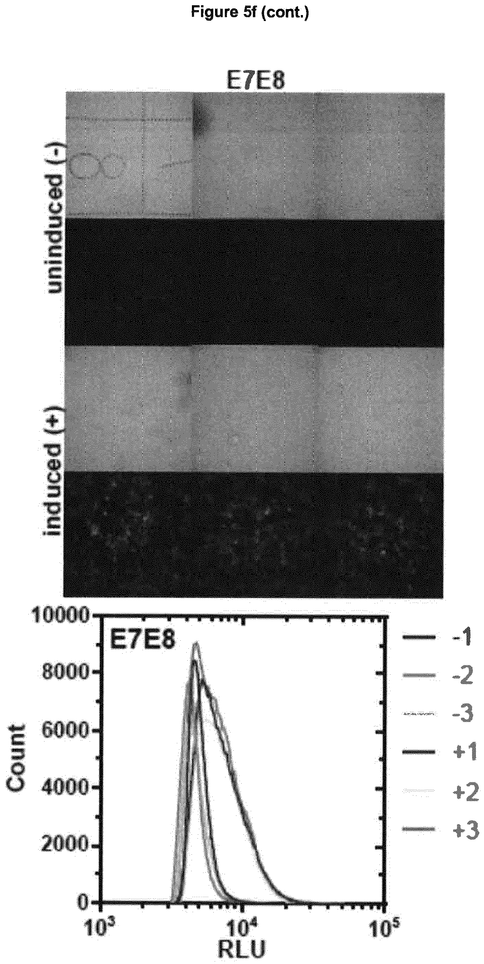

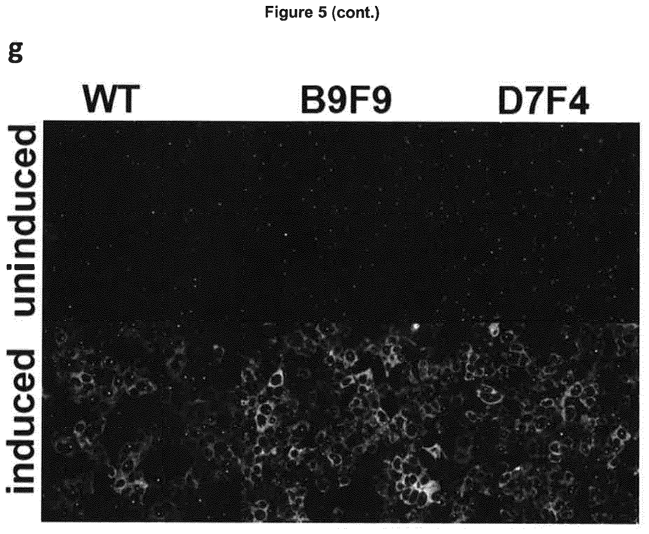

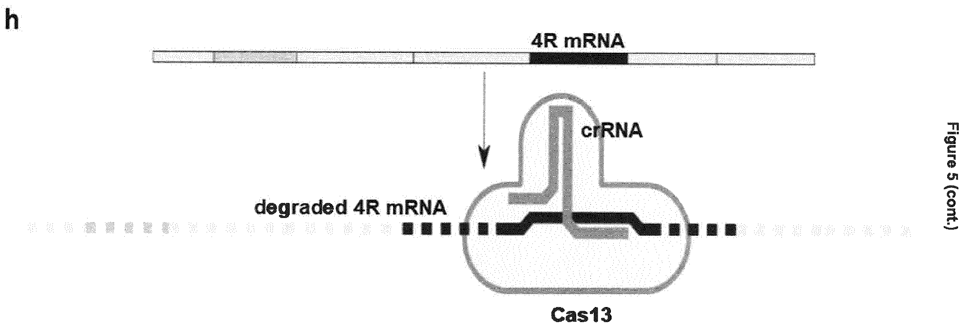

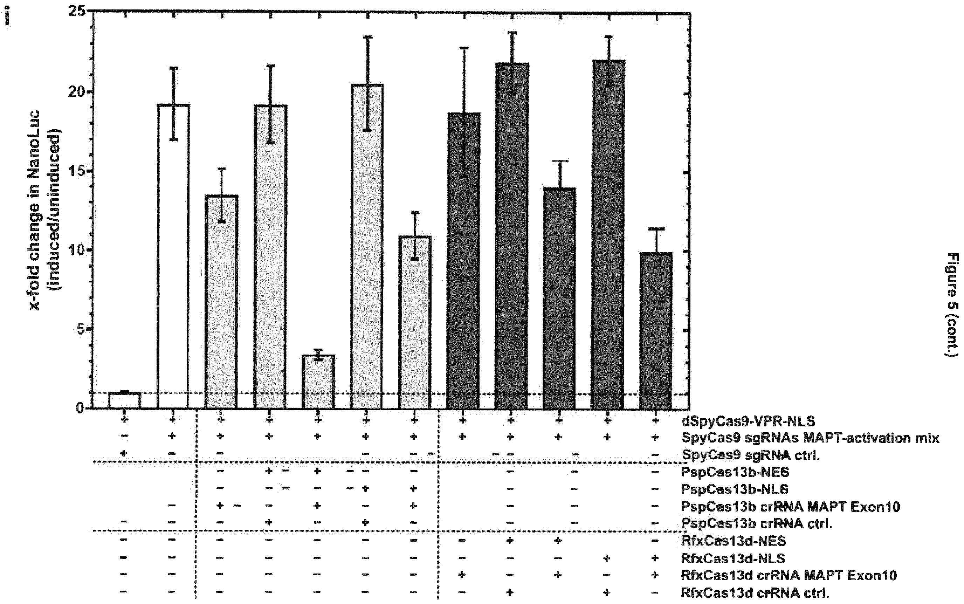

**Figure 5****: Intein-flanked luciferase reporter for non-invasive monitoring of exon-specific isoforms.** **Figure 5a** shows a cell line with the luciferase-based exon-tagging system according to the present invention. **Figure 5b** shows that induction of MAPT was performed using dSpyCas9-NLS-VPR and gRNAs targeting the transcription start site (TSS) of MAPT. **Figure 5c** shows RT-qPCR revealing that MAPT induction was similar in HEK-293 WT cells and also in exon 10 intein-Nluc labeled cells (SD of technical triplicates). **Figure 5d** shows immunoblot analysis, which verifies that the integration of the intein-flanked reporter does not alter the splice pattern of MAPT. All six typical adult isoforms are visible after induction with RNA-guided TFs (+). Clone E7E8 shows somewhat a higher basal expression. Clone D7G2 has one defect allele and is still resistant against Puro and is omitted from further analysis. **Figure 5e** shows a RNA-guided transactivator system (dCas9-VPR), which results in a robust induction of luciferase signal in different clones. The higher basal MAPT expression of clone E7E8 was also observable as increased background signal w/o MAPT induction. **Figure 5f** also shows bioluminescence microscopy of three representative fields of view (FOVs) of clones B9F9 and E7E8 before and after induction. The histograms show the corresponding relative luminescence signals for the 3 FOVs before (-1 to -3) and after induction (+1 to +3). **Figure 5g** shows anti-pan-TAU immunofluorescence revealing that both WT and reporter cell lines show cytosolic TAU staining. **Figure 5h** shows a scheme of the Cas13-mediated mRNA depletion. **Figure 5i** shows CRISPR/Cas13 effectors, especially PspCas13b-NES, which are able to deplete induced 4R TAU expression by greater than 80% tracked via NLuc.

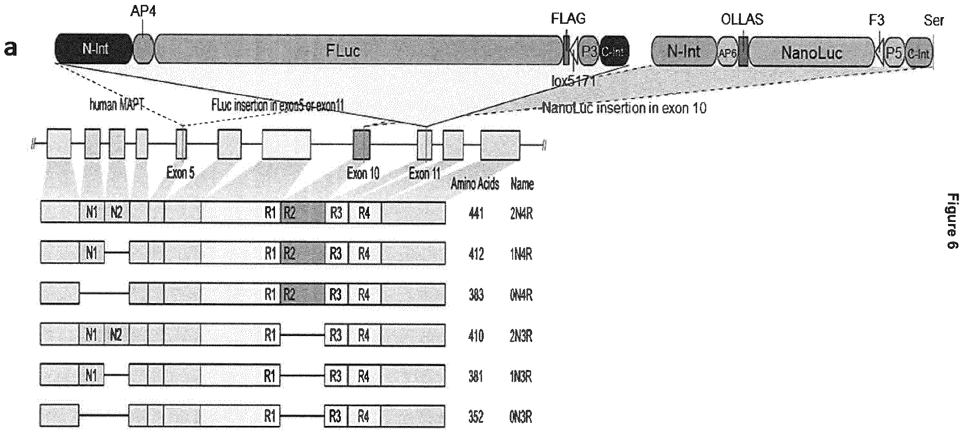

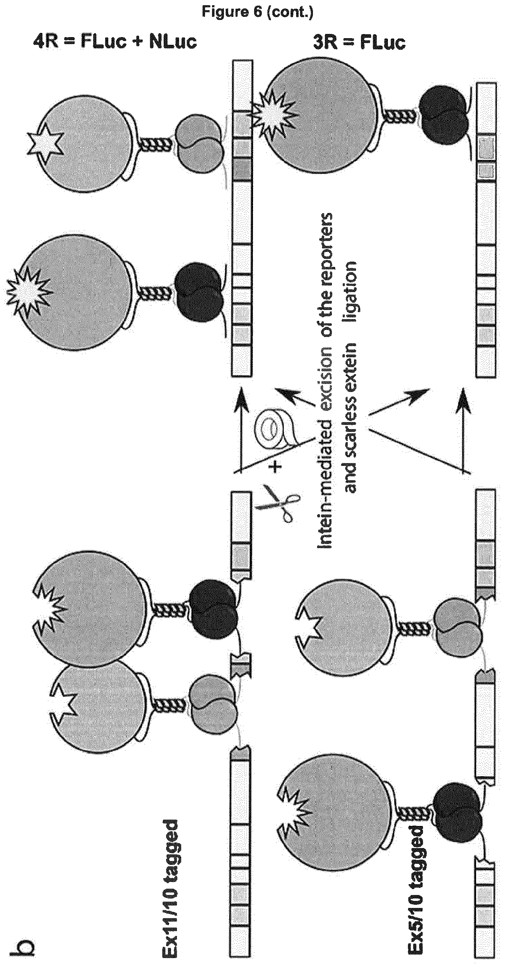

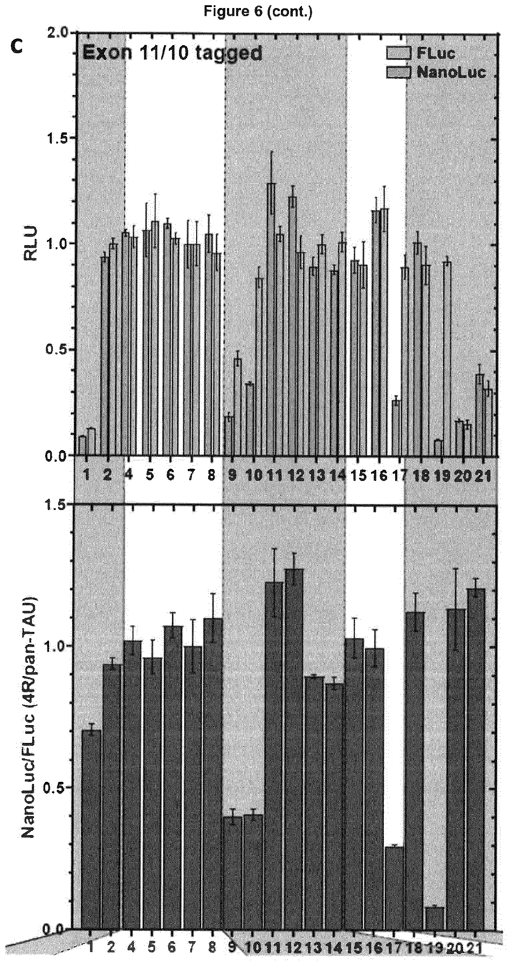

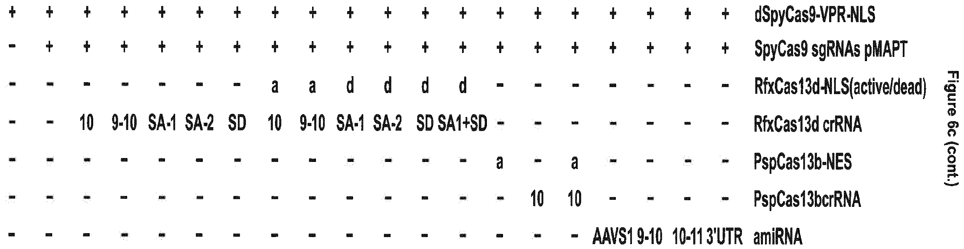

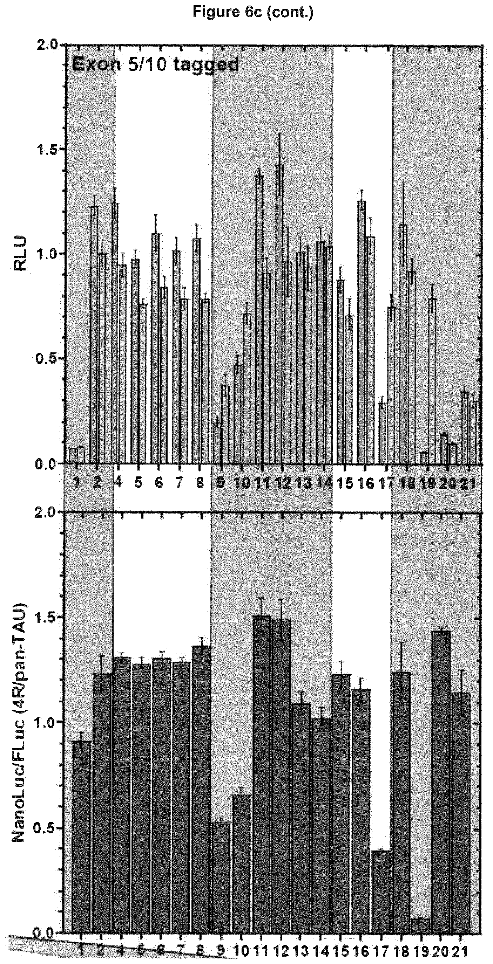

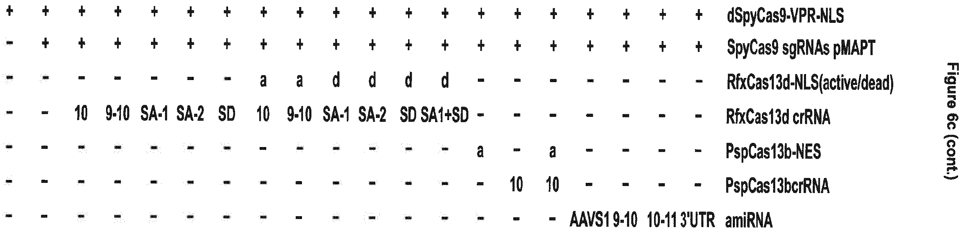

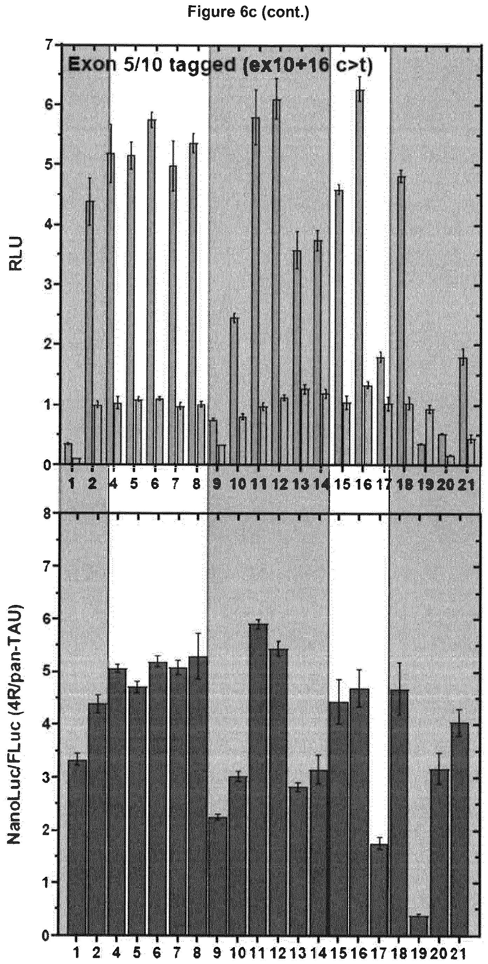

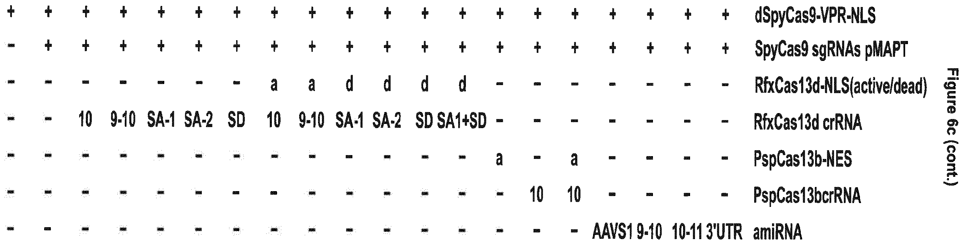

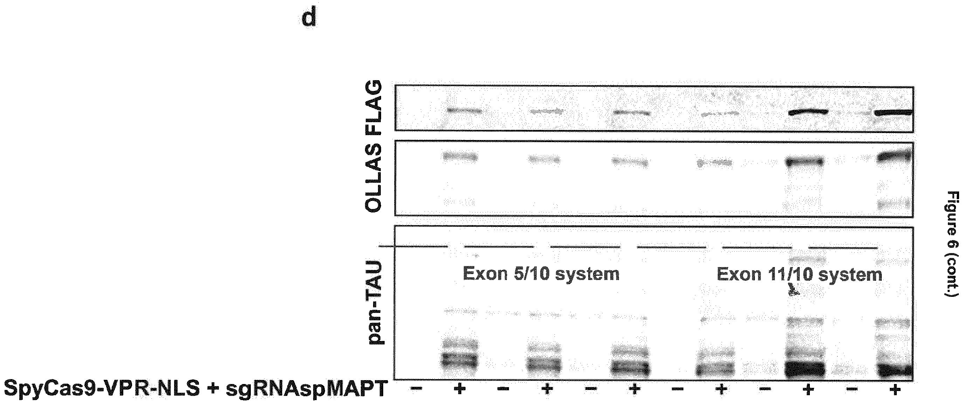

**Figure 6****: Intein-flanked dual-luciferase reporter for ratiometric monitoring of exon-specific** isoforms. **Figure 6a** schematically shows the genetic design to insert a second bioorthogonal reporter Fluc for independent quantification of 4R/pan-TAU expression levels. NrdJ-1 inteins flanking FLuc are introduced into the constitutive exons 5 or exon 11. Nanoluc is flanked by bioorthogonal gp41-1 inteins and coiled coils. **Figure 6b** shows that the Nanoluc signal correlates specifically with exon 10 inclusion whereas FLuc signal indicates the general TAU expression level. The intein-flanked moieties are excised scarlessly from the translation product and can be read out independently (substrate and signal orthogonality: FLuc: D-luciferin (565 nm); Nanoluc (fumirazine, 460 nm). **Figure 6c** shows manipulation of isoform specific-expression with RNAtargeting CRISPR effectors cytosolic PspCas13b-NES, nuclear RfxCas13d-NLS (nuclease-active: "a", and nuclease-defect mutant: "d") and artificial microRNAs (amiRNAs) with indicated targeting crRNAs or regions on the MAPT (pre-)mRNA: 10: ex10; 9-10: ex10/11 junction, 10-11: ex10/11 junction; SA: splice acceptor; SD: splice donor; AAVS1: safe-harbor locus AAVSI intronic region; 3'UTR: 3' untranslated region of MAPT. **Figure 6d** shows immunoblot analysis of individual clones revealing that FLuc (FLAG) and Nanoluc (OLLAS) correlates with MAPT promoter induction with CRISPR/dCas9-VPR-NLS and anti-pan-TAU revealed again the TAU isoforms after induction as shown before.

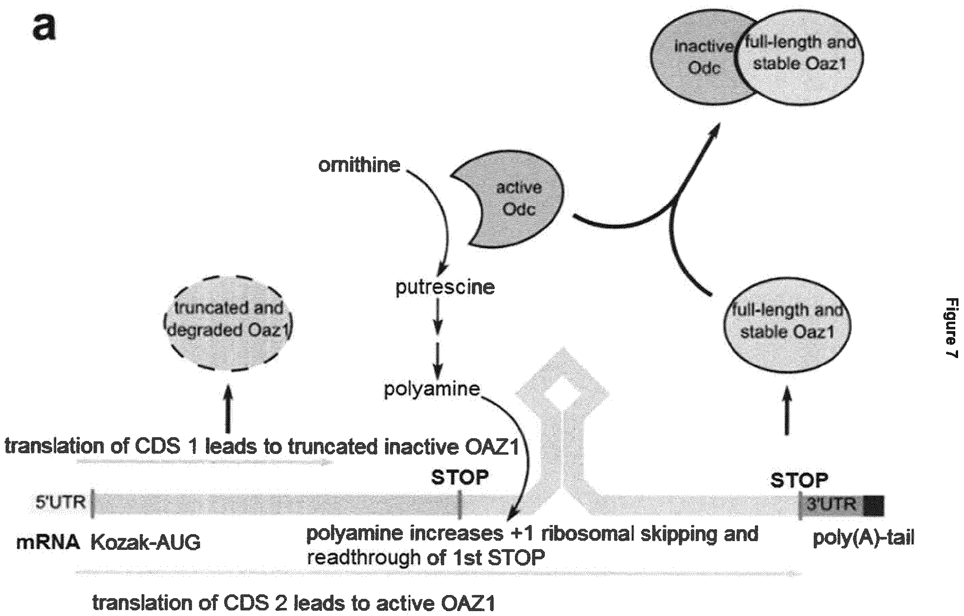

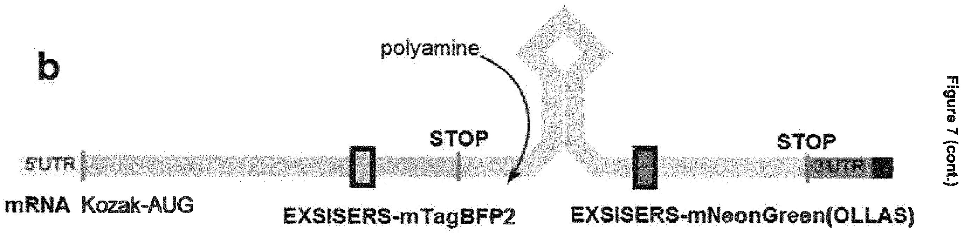

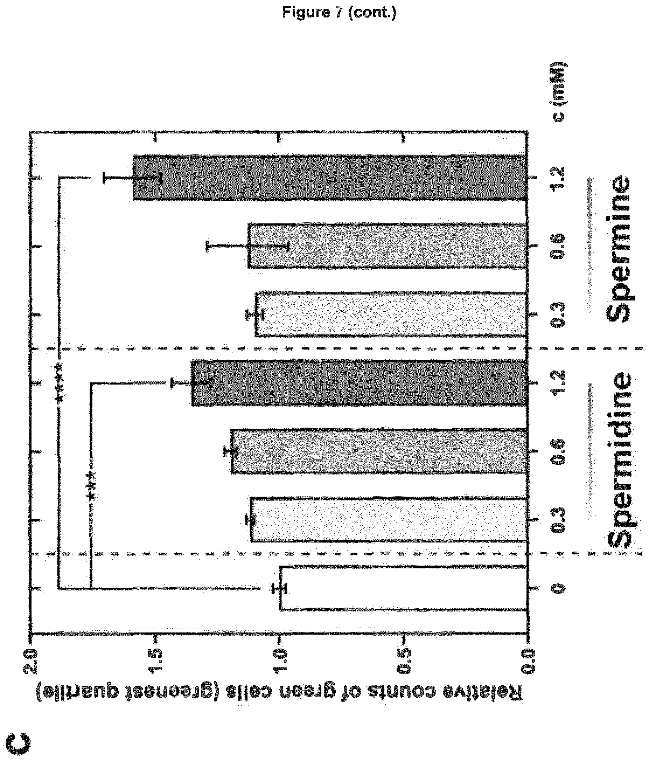

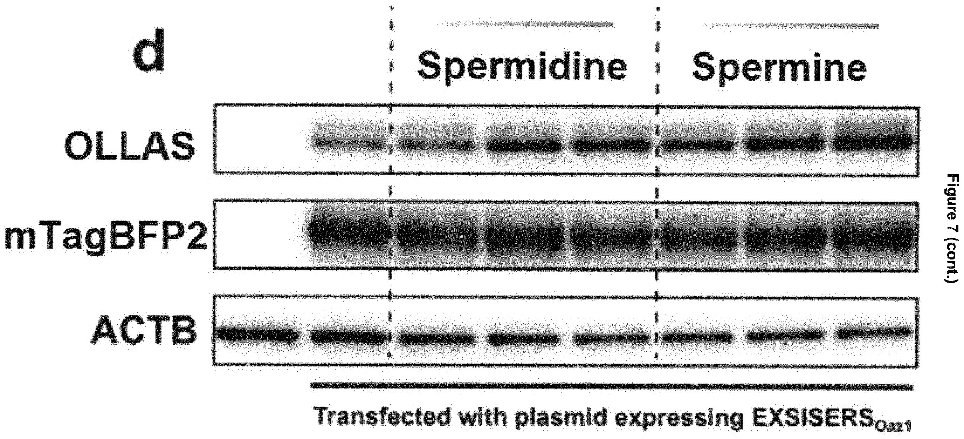

**Figure 7****: Non-invasive protein-level quantification of co-translation regulation.** **Figure 7a** schematically shows: the antizyme Oaz1 is regulated co-translationally by ribosomal frameshifting, which is tightly regulated by polyamines levels such as spermidine and spermine. Rising polyamine levels lead to a +1 frameshift and skipping of the in-frame stop codon leading to the full-length Oaz1 antizyme. The usage of the in-frame stop codon will otherwise lead to truncated non-functional Oaz1. Full-length Oaz1 binds and inactivates the enzyme Odc, the rate-limiting enzyme in the polyamine biosynthesis pathway, resulting in a product-mediated closed-loop homeostatic regulation of polyamine levels. **Figure 7b** schematically shows: gp41-1 split-inteins-flanked mNeonGreen and NrdJ-1-split-inteins-flanked mTagBFP2 were inserted into a plasmid harboring the full-length Oaz1 gene up- and downstream of the regulatory hairpin with the in-frame stop codon. **Figure 7c** shows: FACS analysis of Oaz1-EXSISERS-transfected cells treated with different polyamine levels. Transfected (blue cells) cells were analyzed by counting the fraction of blue cells passing the green gate which is set to contain the 25% greenest cells in the untreated condition (0 mM). **Figure 7d** shows: Immunoblot from the lysate of the corresponding samples shown in Figure 7c. ***, and **** denotes p<0.001, and p<0.0001 of one-way ANOVA post-hoc tests.

### DETAILED DESCRIPTION OF THE INVENTION

The invention provides a method for detecting a specific splice event of a gene of interest, wherein the specific splice event creates a specific splice product, which comprises an exon of interest, wherein the method comprises:
(i) Inserting a split intein - heterologous polynucleotide construct into the exon of interest, wherein the split intein comprises an N-terminal splicing region upstream of the heterologous polynucleotide and a C-terminal splicing region downstream of the heterologous polynucleotide;
   and
(ii) detecting the heterologous polynucleotide and/or the expression product of the heterologous polynucleotide,
wherein the expression product of the split intein - heterologous polynucleotide construct excises itself from the expression product of the specific splice product at a position, wherein the amino acid C-terminal to this position is a cysteine, a serine or a threonine.

The term "specific splice event" relates in the context of the present invention and as used throughout the whole description, to the successfull splicing of an exon of interest of a gene of interest into the mature RNA of the gene of interest. This means, the specific splice event has taken place if the final RNA that will be translated includes the exon of interest. This final RNA that will be translated and which includes the exon of interest is termed "specific splice product" within the context of the present invention.

The term "detecting a specific splice event" can mean in the context of the present invention and as used throughout the whole description, that the "specific splice event" as defined above is identified, traced, tracked, found out, deduced, determined or interrogated. This may also mean in the context of the present invention that in one embodiment in the end by detecting a specific splice event the folding kinetics of the protein of interest can be influenced or modified, e.g. by being slowed down or by being accelerated. This detection step enables the person skilled in the art to immediately feed the information received therefrom into a genetically encoded algorithm, which enables the deduction of properties or characteristics of the relevant isoform of the specific splice product, of the gene of interest or the protein of interest. Thus, the term "detecting a specific splice event" may in the context of the present invention also enable the manipulation and characterization of the specific splice product, of the gene of interest or the protein of interest. Thus, the method of the present invention enables any form of computation of the cell comprising the gene of interest, of the gene of interest or the protein of interest. This also means that the information about the splicing is already encoded in a genetically controlled form, it can be directly converted into outputs other than useful for read-out. The intein splicing event itself can manipulate protein folding whereas the manipulation via a spliced out handle (such as a resistance gene) or an actuator (such as a splice modulator or toxic gene) is a function of the extein.

The invention also provides a method for interrogating a specific splice event of a gene of interest, wherein the specific splice event creates a specific splice product, which comprises an exon of interest, wherein the method comprises:
(i) Inserting a split intein - heterologous polynucleotide construct into the exon of interest, wherein the split intein comprises an N-terminal splicing region upstream of the heterologous polynucleotide and a C-terminal splicing region downstream of the heterologous polynucleotide;
   and
(ii) detecting the heterologous polynucleotide and/or the expression product of the heterologous polynucleotide,
wherein the expression product of the split intein - heterologous polynucleotide construct excises itself from the expression product of the specific splice product at a position, wherein the amino acid C-terminal to this position is a cysteine, a serine or a threonine.
The term "interrogating" may relate in the context of the present invention and as used throughout the whole description, to detection or detection in a least invasive manner, i.e. via ultrafast intein splicing, or changing the dynamics of protein folding while monitoring the event.

The term "gene of interest" means in the context of the present invention and as used throughout the whole description, a specifc segment of DNA, which is desired for investigation, which may be transcribed into RNA, and which may contain an open reading frame and which encodes a protein, and also includes the DNA regulatory elements, which control expression of the transcribed region. A mutation in a gene or in a gene of interest may occur within any region of the DNA which is transcribed into RNA, or outside of the open reading frame and within a region of DNA which regulates expression of the gene (i.e., within a regulatory element). In diploid organisms, a gene is composed of two alleles. "Gene expression" refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include RNAs which are modified, by processes such as capping, polyadenylation, methylation, and editing, and proteins modified by, for example, methylation, acetylation, phosphorylation, ubiquitination, ADP-ribosylation, myristoylation, and glycosylation.

The term "exon of interest" means, in the context of the present invention and as used throughout the whole description, a specific exon, which is desired for investigation, wherein "exon" means a part of a gene that will encode a part of the final mature RNA produced by that gene after introns have been removed by RNA splicing. The term "exon" may refer to both the DNA sequence within a gene and to the corresponding sequence in RNA transcripts. In RNA splicing, introns are removed and exons are covalently joined to one another as part of generating the mature messenger RNA. Just as the entire set of genes for a species constitutes the genome, the entire set of exons constitutes the exome.

The terms "upstream" and "downstream", as used in the context of the present invention and as used throughout the whole description, refers to relative positions of the genetic code in DNA or RNA. Each strand of DNA or RNA has a 5'-end and a 3'-end, so named for the carbon position on the deoxyribose (or ribose) ring. By convention, upstream and downstream relate to the 5'- or 3'-direction, respectively, in which RNA transcription takes place. Upstream is towards the 5'-end of the RNA molecule and downstream is towards the 3'-end. When considering doublestranded DNA, upstream is towards the 5'-end of the coding strand for the gene in question and downstream is towards the 3'-end. Due to the anti-parallel nature of DNA, this means the 3'-end of the template strand is upstream of the gene and the 5'-end is downstream.

The term "expression product" means, in the context of the present invention and as used throughout the whole description, the product received from expression, meaning the process by which information from a gene is used in the synthesis of a functional gene product. These products are often proteins, but in non-protein coding genes, such as transfer RNA (tRNA) or small nuclear RNA (snRNA) genes, the product is a functional RNA.

Since this method according to the present invention is non-consumptive, all preparation steps for RNA-based methods are needless, thus reducing potential bias. As an imaging method (fluorescent & bioluminescent microscopy), it enables AS-quantification measurements *in vivo* to study the effects of the specific splice event at different time points under several conditions. Based on split-inteins, the inventors developed a palette of tools, which facilitate research focused on enlightening mechanisms in alternative splicing.

"Inteins" as used in the context of the present invention and as used throughout the whole description, can be described as protein introns, which are able to autocatalytically splice themselves posttranslationally out of a protein, respectively protein of interest, resulting in covalently linked exteins as a scarless gene product. This process may be termed protein splicing. Exteins on the other hand are the remaining portions of the protein after the intein has excised itself out. "Scarless gene product" means in this context that a gene product is received or gained, which is not influenced or altered in its properties and characteristics, e.g. the kinetic properties have stayed the same, compared to a gene product, which has been received without an intein, splicing itself out of it posttranslationally.

The term "split intein" means in the context of this present invention and as used throughout the whole description, a subset of inteins that are expressed in two separate halves, named in the context of the present invention "N-intein" and "C-intein", respectively "N-terminal splicing region" and "C-terminal splicing region", and catalyze splicing *in trans* upon association of the two domains. The term "two separate halves" does not mean in this context that the two separated domains of the split intein are even or equally split. Instead, the term also includes any split ratio between the two domains of the split intein, which a person skilled in the art can conceive of. The "split intein" may occur naturally and may also been artificially generated by splitting of contiguous ones. With their unique properties, split-inteins offer improved controllability, flexibility and capability to existing tools based on contiguous inteins.

Intein-mediated protein splicing typically occurs after the intein-containing mRNA has been translated into a protein. The process begins with an N-O or N-S shift, when the side chain of the first residue (preferably a serine, threonine, or cysteine) of the (N-terminal split) intein portion of the expression product of the specific splice product nucleophilically attacks the peptide bond of the residue immediately upstream (that is, the final residue of the N-extein) to form a linear ester (or thioester) intermediate. A transesterification occurs when the side chain of the first residue of the C-extein, i.e. the amino acid C-terminal to the C-terminal split intein, attacks the newly formed (thio)ester to free the N-terminal end of the intein. This forms a branched intermediate, in which the N-extein and C-extein are attached, albeit not through a peptide bond. The last residue of the intein preferably is an asparagine, and the amide nitrogen atom of this side chain might cleave apart the peptide bond between the intein and the C-extein, resulting in a free intein segment with a terminal cyclic imide. Finally, the free amino group of the C-extein may now attack the (thio)ester linking the N- and C-exteins together. An O-N or S-N shift therefore preferably produces a peptide bond and the functional, ligated protein.

As soon as N- and C-exteins (flanking the intein) are in spatial proximity to each other, the excision process can be initialized by forming a succinimide intermediate. For this process, the presence of several amino acids in fixed positions may be required: Either a cysteine or a serine residue at the N-terminal side of the intein, an asparagine at the C-terminal side of the intein and another cysteine at the beginning of the C-terminal extein may exist. After splicing has taken place, the resulting protein contains the N-extein linked to the C-extein; this splicing product may be also termed an extein.

Examples for split inteins include the NrdJ-1 intein or the gp41-1 intein - both of which may be split and excise the polypeptide that has been fused between the N- and the C-terminus of the split intein.

In one embodiment of the method of the present invention, the N-terminal splicing region of the split intein comprises or consists of the NrdJ-1 N-terminal region (SEQ ID NO: 1) or the gp41-1 N-terminal region (SEQ ID NO: 2), and/or the C-terminal splicing region of the split intein comprises or consists of the NrdJ-1 C-terminal region (SEQ ID NO: 3) or the gp41-1 C-terminal region (SEQ ID NO: 4). In one embodiment of the method of the present invention, the N-terminal splicing region of the split intein comprises or consists of the NrdJ-1 N-terminal region (SEQ ID NO: 1). In one specific embodiment of the method of the present invention, the N-terminal splicing region of the split intein comprises or consists of the gp41-1 N-terminal region (SEQ ID NO: 2). In another embodiment of the method of the present invention, the C-terminal splicing region of the split intein comprises or consists of the NrdJ-1 C-terminal region (SEQ ID NO: 3). In one further embodiment of the method of the present invention, the C-terminal splicing region of the split intein comprises or consists of the gp41-1 C-terminal region (SEQ ID NO: 4). In one further embodiment of the method of the present invention, the N-terminal splicing region of the split intein consists of the NrdJ-1 N-terminal region (SEQ ID NO: 1). In another embodiment of the method of the present invention, the N-terminal splicing region of the split intein consists of the gp41-1 N-terminal region (SEQ ID NO: 2). In one further embodiment of the method of the present invention, the C-terminal splicing region of the split intein consists of the NrdJ-1 C-terminal region (SEQ ID NO: 3). In one embodiment of the method of the present invention, the C-terminal splicing region of the split intein consists of the gp41-1 C-terminal region (SEQ ID NO: 4).

In a further embodiment of the method of the present invention, the split intein is gp41-1 or NrdJ-1. In one embodiment, the N-terminal splicing region of the split intein comprises or consists of the NrdJ-1 N-terminal region (SEQ ID NO: 1) and the C-terminal splicing region of the split intein comprises or consists of the NrdJ-1 C-terminal region (SEQ ID NO: 3). In another embodiment, the N-terminal splicing region of the split intein comprises or consists of the gp41-1 N-terminal region (SEQ ID NO: 2) and the C-terminal splicing region of the split intein comprises or consists of the gp41-1 C-terminal region (SEQ ID NO: 4). In one embodiment, the N-terminal splicing region of the split intein consists of the NrdJ-1 N-terminal region (SEQ ID NO: 1) and the C-terminal splicing region of the split intein consists of the NrdJ-1 C-terminal region (SEQ ID NO: 3). In another embodiment, the N-terminal splicing region of the split intein consists of the gp41-1 N-terminal region (SEQ ID NO: 2) and the C-terminal splicing region of the split intein consists of the gp41-1 C-terminal region (SEQ ID NO: 4).

In one specific embodiment of the method of the present invention, the expression product of the specific splice product is a single polypeptide chain. The term "polypeptide" is understood to indicate a mature protein or a precursor form thereof as well as a functional fragment thereof which essentially has retained the activity of the mature protein, i.e. exhibits at least the same qualitative activity and preferably also at least a similar quantitative activity as the mature protein. A functional fragment may for instance be an N- and/or C-terminal truncated form of a full-length polypeptide, or an isoform, in particular a native isoform, of a full-length polypeptide.

In a further embodiment of the method of the present invention, the expression product of the N-terminal splicing region of the split intein comprises at its N-terminus a cysteine or a serine. In a further embodiment of the method of the present invention, the expression product of the N-terminal splicing region of the split intein comprises at its N-terminus a cysteine. In another embodiment of the method of the present invention, the expression product of the N-terminal splicing region of the split intein comprises at its N-terminus a serine.

In another embodiment of the method of the present invention, the expression product of the C-terminal splicing region of the split intein comprises at its C-terminus an asparagine.

"Heterologous polynucleotide" as used herein relates to a nucleic acid, which encodes a protein that is not (naturally) present in a host cell. In one specific embodiment of the method of the present invention, the heterologous polynucleotide encodes a protein or enzyme selected from the group consisting of a fluorescent protein, preferably green fluorescent protein; a bioluminescence-generating enzyme, preferably NanoLuc, NanoKAZ, Cypridina, Firefly, Renilla luciferase or mutant derivatives thereof; an enzyme, which is capable of generating a colored pigment, preferably tyrosinase or an enzyme of a multi-enzymatic process, more preferably the violacein or betanidin synthesis process, a genetically encoded receptor for multimodal contrast agents, preferably Avidin, Streptavidin or HaloTag or mutant derivatives thereof; an enzyme, which is capable of converting a non-reporter molecule into a reporter molecule, preferably TEV protease and picornaviral proteases, more preferably rhinoviral 3C proteases and polioviral 3C protease, SUMO proteases and mutant derivatives thereof; an enzyme, which is capable of inactivating a toxic compound, preferably blasticidin-S-deaminase, puromycin-N-acetyltransferase, neomycin phosphotransferase, hygromycin B phosphotransferase and mutant derivatives thereof and an enzyme, which is capable of converting pro-drug/toxin-mediated toxicity, preferably thymidine kinase and mutant derivatives thereof and a small-molecule sensor protein, preferably calmodulin, troponin C, S100 and mutant derivatives thereof. Preferred proteins or enzymes encoded by the heterologous polynucleotide are depicted in SEQ ID NOs: 33 to 40.

In a further embodiment of the method of the present invention, the split intein - heterologous polynucleotide construct further contains at least one polynucleotide encoding for a hetero-dimerizing domain or a homo-dimerizing domain, preferably at least one PDZ-domain or at least one coiled-coil-domain, more preferably two coiled-coil-domains in an antiparallel configuration, for accelerating the specific splice event. The term "heterodimerizing domain" means in the context of this present invention and as used throughout the whole description, association of two non-identical proteins or peptides to a larger complex. The term "homodimerizing domain" means in the context of this present invention and as used throughout the whole description, the domain enabling association of two identical proteins or peptides to a larger complex. The term "PDZ domain" means in the context of the present invention and as used throughout the whole description, a common structural domain of 80-90 amino acids found in the signaling proteins of many bacteria, yeast, plants, viruses and animals. "PDZ" is an initialism combining the first letters of the first three proteins discovered to share the domain. The PDZ domain structure is partially conserved across the various proteins that contain them. They usually have 4 β-strands and one short and one long α-helix. Apart from this conserved fold, the secondary structure differs across PDZ domains. The term "coiled-coil domain" means in the context of the present invention and as used throughout the whole description two alpha-helical peptides dimerized by intertwining the helices. This can occur as homo- or heterodimer, and in parallel or anti-parallel conformation.

Accordingly, the split intein - heterologous polynucleotide construct preferably further contains at least one polynucleotide encoding for a hetero-dimerizing domain or a homo-dimerizing domain, preferably at least one PDZ-domain or at least one coiled-coil-domain, more preferably two coiled-coil-domains in an antiparallel configuration, for accelerating the self-excision of the expression product of the split intein - heterologous polynucleotide construct from the expression product of the specific splice product. The above given definitions also apply for this specific embodiment.

After intersection of the split intein - heterologous polynucleotide construct into the exon of interest in a cell, it might be necessary to enrich or select the cells, in which the construct has been successfully inserted or integrated. One possibility is to include a selection marker into the split intein - heterologous polynucleotide construct. A person skilled in the art is aware how to select selection markers and how to isolate or enrich cells expressing the selection marker. Accordingly, the heterologous polynucleotide of the split intein - heterologous polynucleotide construct preferably further contains a temporary selection marker for stable cell line generation. "Temporary" in this context means that the selection marker does not necessarily need to be permanently integrated into the host cell. An exemplary selection marker, a puromycin resistance gene, is shown in SEQ ID NO: 43.

The method of the present invention includes a step of detecting the heterologous polynucleotide and/or the expression product of the heterologous polynucleotide. This step allows to study the specific splice event and/or the influence of any manipulation on the cell, e.g. by a modulator of the specific splice event. This also may comprise that detecting the heterologous polynucleotide and/or the expression product of the heterologous polynucleotide enables the person skilled in the art to deduce several or any conceivable property or characteristic of the gene of interest or the protein of interest by this specific detection step. For example, if the person skilled in the art knows or detects by said step the population of the heterologous polynucleotide, he/she can also derive the population of the protein of interest therefrom, as usually, the split intein-heterologous polynucleotide is present in the equal ratio as the protein of interest from which it had been spliced out. Many methods for the detection of a heterologous polynucleotide or an expression product thereof are known to a person skilled in the art. Exemplary methods for detecting the heterologous polynucleotide and/or the expression product of the heterologous polynucleotide include, but are not limited to, high-throughput screening, western blotting, mass spectrometry, luciferase-assays, and longitudinal live-imaging, preferably bioluminescence imaging, fluorescence imaging, photoacoustic imaging, MRI and PET. The preferred method for detecting the heterologous polynucleotide and/or the expression product of the heterologous polynucleotide are luciferase systems, respectively luciferase-assays.

One advantage of the present invention is that the expression of the heterologous polynucleotide or the expression product is directly coupled to the specific splice event. This is achieved by integrating the heterologous polynucleotide into the exon of interest. In addition, the heterologous polynucleotide that is flanked by a split intein excises itself from the expression product of the exon of interest. I.e., even though the sequence of the exon of interest has been altered, the inserted sequence excises itself from the expression product, thereby leaving the exon of interest unaltered. This approach can be described as "scarless", "footprint-free" and non- or minimally invasive. Accordingly, the method of the present invention is preferably non- or minimally-invasive for the protein of interest such that a native and/or fully functional protein of interest is expressed compared to the protein of interest without insertion of the split intein - heterologous polynucleotide construct according to any of the methods according to the present invention as described herein. When the protein of interest is non- or minimally invasive, this also means that the folding kinetics of this protein are not altered or are substantially not altered. This aspect regarding folding kinetics can be seen, for example, in Figure 2c of the present invention, wherein the folding kinetics are sufficiently fast enough in the presence of coiled-coil domains to shift the ratio of unspliced protein even in the case of a very rapidly folding protein, such as for mNG (< 10 min at 37 °C, Shaner *et al*.). "Substantially not altered" means in this context that the kinetic of the protein of interest with applying any of the methods according to the present invention thereto, has still at least 95%, at least 96%, at least 97%, at least 98%, at least 99% or even 100% of the kinetic ability of the protein of interest without applying any of the methods according to the present invention.

In many batch detection methods like, e.g., luciferase assays, a normalization is frequently applied. This normalization may be done by comparison of the activity of the reporter protein with the activity of a reference protein. In the context of luciferase assays, a dual luciferase assay is typically used for normalization. In this context, a first luciferase is used as a readout for the actual experiment while a second and different luciferase, which is constitutively expressed, serves for the normalization. This normalization allows for compensation of different cell numbers or other influences. Typically, the first and the second luciferase use a different substrate. This principle is however not limited to luciferases but can be transferred to any reporter protein encoded by the heterologous polynucleotide. Here, one reporter enzyme is encoded by the heterologous polynucleotide - split intein construct. For normalization, a second and different reporter enzyme is integrated. The second reporter enzyme preferably is constitutively expressed, i.e. its expression is preferably not altered by the conditions applied to the cell. Accordingly, a further heterologous polynucleotide encoding for a reporter enzyme, which is preferably selected from the group consisting of a fluorescent protein, a bioluminescence-generating enzyme, more preferably a luciferase enzyme, is preferably inserted into a constitutively expressed exon of the gene of interest, wherein said further heterologous polynucleotide encoding for a reporter enzyme is different from the heterologous polynucleotide as defined above.

In some instances, the specific splice product may only be present in a host cell at low frequency. Thus, an equimolar level of the expression product of the specific splice product and the excised split intein - heterologous polynucleotide construct may not be sufficient to enable a direct detection of a reporter protein, corresponding to the expression product of the heterologous polynucleotide. Thus, an indirect approach may be applied. In this case, a reporter protein is constitutively expressed in a host cell. This constitutively expressed reporter protein is however inactive unless activated by an activator. This activator may be e.g. a protease that modifies the reporter protein to enable fluorescence activity. Since the activator can activate many constitutively expressed reporter proteins, the signal is enhanced and thereby facilitates the detection of the specific splice event. Accordingly, in a further embodiment of the method of the present invention, the split intein - heterologous polynucleotide construct further comprises a polynucleotide encoding for a protein which functions as an activator of the further heterologous polynucleotide encoding for a reporter enzyme or as an activator of the heterologous polynucleotide of the split intein - heterologous polynucleotide construct.

In addition to the detection of the heterologous polynucleotide and/or the expression product thereof in step (ii) of the method of the present invention, the specific splice product of the gene of interest comprising the exon of interest may be quantified by means known to a person skilled in the art. Thus, not only the heterologous polynucleotide and/or expression product thereof may be detected but, of course, the protein comprising the exon of interest itself. Accordingly, the method of the present invention preferably further comprises as a step (iii) the quantification of an isoform population of the protein of interest encoded by the gene of interest. "Isoform" in this context relates to protein isoforms that result after splicing. One isoform is one specific combination of exons.

The specific splice event may not only be monitored or detected by the expression product of the heterologous polynucleotide, e.g. a fluorescent protein as a reporter protein. The detection may also be indirect. E.g., the heterologous polynucleotide could encode an antibiotic resistance gene. This could be related to the concept of computation as defined above since it constitutes a manipulation mediated by the extein and thus is genetically programmable and is in clear distinction to pure detection methods based on e.g. FISH. An assay for the detection of the specific splice event might than include a step for treating the host cell comprising the antibiotic gene in the heterologous polynucleotide - split intein reporter construct. Cells, which survive a treatment with the respective antibiotic, express the specific splice product. This method might also be used for the enrichment or selection of cells showing the specific splice event. Accordingly, the heterologous polypeptide of the split intein - heterologous polynucleotide construct preferably is an antibiotic resistance gene and wherein the method alternatively to step (ii) comprises detecting of the antibiotic resistance of the cells of interest comprising the protein of interest encoded by the gene of interest. "Detecting" within this context and specific embodiment of the present invention relates to the addition of an antibiotic against which the antibiotic resistance gene provides resistance, to the cell and/or culture medium. Survival of the cell is then indicative for the presence of the specific splice event in the cell. In a preferred embodiment of this method of the present invention, the antibiotic resistance gene is selected from the group consisting of blasticidin-S-deaminase, puromycin-N-acetyltransferase, neomycin phosphotransferase, hygromycin B phosphotransferase and mutant derivatives thereof. In a further preferred embodiment of the present invention, the antibiotic resistance gene is selected from the group consisting of blasticidin-S-deaminase, puromycin-N-acetyltransferase, neomycin phosphotransferase and hygromycin B phosphotransferase.

Another option for detection of the specific splice event of a gene of interest in a cell makes use of a cell surface marker. In this case, the heterologous polynucleotide encodes for a cell surface marker. This cell surface marker may then be detected by e.g. fluorescently labelled immunoglobulins, anti-Avidin or anti-HaloTag labelled antibodies or fluorescently labelled small molecules, since Avidin binds the small molecule biotin (coupled to e.g. fluorescent dyes) and also HaloTag binds halogenalkanated molecules (Nordlund et al., Los et al.). In any case, cells presenting the cell surface marker on their surface are cells, in which the specific splice event has taken place. Thus, the use of a cell surface marker encoded by the heterologous polynucleotide may, e.g., be used to monitor the specific splice event and/or for the selection or enrichment of cells, in which the specific splice event takes place. E.g., cells, which present the cell surface marker on their cell surface, may be isolated by flow cytometry and/or magnetic cell separation. Accordingly, the method of the present invention preferably comprises alternatively to step (ii) or additionally to step (ii) the detection of an isoform dependent cell-surface marker. Exemplary split intein - heterologous polynucleotide constructs comprising a cell surface marker are, for example, shown in SEQ ID NO: 11 or 12.

In a further embodiment of the method of the present invention, the method further comprises (iii) manipulation of the folding process of the protein of interest encoded by the gene of interest. The term "manipulation of the folding process" may mean in this regard and in the context of the present invention any form of influencing or alternating the folding process of the protein of interest a person skilled in the art can conceive of. For example, this can comprise that the folding process is accelerated or slowed down for being able to further study the folding process of the protein of interest or it may mean altering the efficiency for a folding sequence involving different protein subdomains (Spencer et al.). For example, some proteins may not fold correctly and are prone to aggregation during the natural folding process. Therefore, naturally, sometimes codons are present between protein subdomains so that each domain could have more or enough time to be able to fold separately. These codons can be replaced by using inteins so that the designer protein domain before the intein domain can fold before the 2^{nd} domain is translated and so on.

In another embodiment of the method of the present invention, the method further comprises (iii) manipulation of the kinetics of the splice event of the gene of interest, preferably wherein the kinetics of the specific splice event is manipulated due to step (ii), i.e. due to the information received from the detection step (ii). The term "manipulation of the kinetics of the splice event" may mean in this regard that the folding kinetics can be tuned to accelerate or also slow down to study the folding kinetics as part of a basic research or to steer the folding process of designer proteins (de novo or variants of wildtype proteins) as to enable the folding of also complex multi-domain proteins that would otherwise need, e.g., chaperones, for folding.

By the method according to the present invention, the kinetics of the splicing process can be influenced such that it does not alter the formation of the protein of interest. Further, it could however also be designed such that the folding of several domains of a designer protein could be influenced beneficially, e.g. such that sequential folding of domains is possible. With the methods according to the present invention, the kinetics of the splicing process can be modulated, e.g. using different inteins, by adding or not adding coiled coil-domains, etc.

As outlined herein, the expression product of the heterologous polynucleotide may be used to detect cells, in which the specific splice event has taken place. This is possible, because the detection of the expression product of the heterologous polynucleotide marks cells, in which the specific splice event takes place and/or has taken place. Thus, those cells can be selected or isolated. Accordingly, the method of the present invention further comprises as step (iii) the enrichment of cells comprising the protein of interest encoded by the gene of interest, preferably the enrichment of cells comprising a specific isoform of the protein of interest.

In another embodiment of the method of the present invention, the method further comprises (iii) modification of the folding process of the protein of interest. The term "modification of the folding process" may mean in this regard and in the context of the present invention any form of influencing or alternating the folding process of the protein of interest a person skilled in the art can conceive of. For example, this can comprise that the folding kinetics are not made maximally fast to provide scarless monitoring as described above, but instead may be slowed down such that individual domains of the protein of interest can fold ,first' as to reduce the complexity of the protein folding. This provides a powerful option besides or additional to chaperones. This embodiment also comprises that the folding process may be accelerated or slowed down for being able to further study the folding process of the protein of interest.

In addition to the detection of the heterologous polynucleotide and/or the expression product thereof in step (ii) of the method of the present invention, the specific splice product of the gene of interest comprising the exon of interest may be quantified by means known to a person skilled in the art. Thus, not only the heterologous polynucleotide and/or expression product thereof may be detected but, of course, also the protein comprising the exon of interest itself. Accordingly, the method of the present invention, preferably further comprises (iii) quantification of the protein of interest encoded by the gene of interest or quantification of the exon of interest.

The method of the present invention may also be used to identify regulators of the inclusion or excision of the exon of interest. Thus, by applying the method of the present invention, it is possible to screen for regulators of the specific splice event. "Regulators" in this context may relate to polypeptides, nucleic acids, lipids or small molecule inhibitors. The regulator may either increase or decrease the rate of the specific splice event. Accordingly, the method of the present invention preferably further comprises (iii) identification of a regulator of the inclusion or exclusion of the exon of interest, preferably identification of a regulator of the inclusion or exclusion of the exon of interest of a pre-mRNA. In that specific embodiment, the regulator may regulate alternative splicing of a non-constitutive exon.

It is also possible, that the method further comprises the application of a CRISPR-library or cDNA library. A "CRISPR-library" in this context relates to a set of host cells, in which in each of the host cells one gene has been knocked out by applying a CRISPR-mediated knockout. A "cDNA library" on the other hand is used for overexpression of a different target protein in each of the set of host cells. In this specific embodiment, the method may further comprise (iv) inactivation or activation of the regulator as defined above, preferably inactivation of the regulator, more preferably inactivation of the regulator by a toxic compound, wherein the toxic compound is selected from the group consisting of puromycin, blasticidin-S, neomycin, hygromycin and derivatives thereof and pro-drug/toxins, preferably ganciclovir, acyclovir or derivatives thereof. In this specific embodiment, the method may further comprise (v) detection of the survival of the cell comprising the protein of interest encoded by the gene of interest. It is also preferred that in this specific embodiment, the survival of the cell is detected by applying toxic compounds, preferably that the toxic compound is selected from the group consisting of puromycin, blasticidin-S, neomycin, hygromycin and derivatives thereof and pro-drug/toxins, more preferably ganciclovir, acyclovir or derivatives thereof.

The present invention also provides the use of a split intein - heterologous polynucleotide construct, wherein the split intein comprises an N-terminal splicing region upstream of the heterologous polynucleotide and a C-terminal splicing region downstream of the heterologous polynucleotide, in any of the methods according to the present invention as described herein. This use according to the present invention also may include that the heterologous polynucleotide encodes for a protein or enzyme selected from the group consisting of a fluorescent protein, preferably green fluorescent protein; a bioluminescence-generating enzyme, preferably NanoLuc, NanoKAZ, Cypridina, Firefly, Renilla luciferase or mutant derivatives thereof; an enzyme, which is capable of generating a colored pigment, preferably tyrosinase or an enzyme of a multi-enzymatic process, more preferably the violacein or betanidin synthesis process; a genetically encoded receptor for multimodal contrast agents, preferably Avidin, Streptavidin or HaloTag or mutant derivatives thereof; an enzyme, which is capable of converting a non-reporter molecule into a reporter molecule, preferably TEV protease and picornaviral proteases, more preferably rhinoviral 3C proteases and polioviral 3C protease, SUMO proteases and mutant derivatives thereof; an enzyme, which is capable of inactivating a toxic compound, preferably blasticidin-S-deaminase, puromycin-N-acetyltransferase, neomycin phosphotransferase, hygromycin B phosphotransferase and mutant derivatives thereof, an enzyme, which is capable of converting pro-drug/toxin-mediated toxicity, preferably thymidine kinase and mutant derivatives thereof and a small-molecule sensor protein, preferably calmodulin, troponin C, S100 and mutant derivatives thereof. Further, it is preferred for this use according to the present invention that the split intein - heterologous polynucleotide construct is set forth in any of the SEQ ID NOs: 5 to 22. In one specific embodiment of the use according to the present invention, the split intein - heterologous polynucleotide construct is set forth in SEQ ID NO: 5. In one specific embodiment of the use according to the present invention, the split intein - heterologous polynucleotide construct is set forth in SEQ ID NO: 6. In one specific embodiment of the use according to the present invention, the split intein - heterologous polynucleotide construct is set forth in SEQ ID NO: 7. In one specific embodiment of the use according to the present invention, the split intein - heterologous polynucleotide construct is set forth in SEQ ID NO: 8. In one specific embodiment of the use according to the present invention, the split intein - heterologous polynucleotide construct is set forth in SEQ ID NO: 9. In one specific embodiment of the use according to the present invention, the split intein - heterologous polynucleotide construct is set forth in SEQ ID NO: 10. In one specific embodiment of the use according to the present invention, the split intein - heterologous polynucleotide construct is set forth in SEQ ID NO: 11. In one specific embodiment of the use according to the present invention, the split intein - heterologous polynucleotide construct is set forth in SEQ ID NO: 12. In one specific embodiment of the use according to the present invention, the split intein - heterologous polynucleotide construct is set forth in SEQ ID NO: 13. In one specific embodiment of the use according to the present invention, the split intein - heterologous polynucleotide construct is set forth in SEQ ID NO: 14. In one specific embodiment of the use according to the present invention, the split intein - heterologous polynucleotide construct is set forth in SEQ ID NO: 15. In one specific embodiment of the use according to the present invention, the split intein - heterologous polynucleotide construct is set forth in SEQ ID NO: 16. In one specific embodiment of the use according to the present invention, the split intein - heterologous polynucleotide construct is set forth in SEQ ID NO: 17. In one specific embodiment of the use according to the present invention, the split intein - heterologous polynucleotide construct is set forth in SEQ ID NO: 18. In one specific embodiment of the use according to the present invention, the split intein - heterologous polynucleotide construct is set forth in SEQ ID NO: 19. In one specific embodiment of the use according to the present invention, the split intein - heterologous polynucleotide construct is set forth in SEQ ID NO: 20. In one specific embodiment of the use according to the present invention, the split intein - heterologous polynucleotide construct is set forth in SEQ ID NO: 21. In one specific embodiment of the use according to the present invention, the split intein - heterologous polynucleotide construct is set forth in SEQ ID NO: 22.

The present invention also provides a nucleic acid encoding a split intein - heterologous polynucleotide construct, wherein the split intein comprises an N-terminal splicing region upstream of the heterologous polynucleotide and a C-terminal splicing region downstream of the heterologous polynucleotide. In this specific embodiment, the heterologous polynucleotide may encode a protein or enzyme selected from the group consisting of a fluorescent protein, preferably a green fluorescent protein; a bioluminescence-generating enzyme, preferably NanoLuc, NanoKAZ, Cypridina, Firefly, Renilla luciferase or mutant derivatives thereof; an enzyme, which is capable of generating a colored pigment, preferably tyrosinase or an enzyme of a multi-enzymatic process, more preferably the violacein or betanidin synthesis process; a genetically encoded receptor for multimodal contrast agents, preferably Avidin, Streptavidin or HaloTag or mutant derivatives thereof; an enzyme, which is capable of converting a non-reporter molecule into a reporter molecule, preferably TEV protease and picornaviral proteases, more preferably rhinoviral 3C proteases and polioviral 3C protease, SUMO proteases and mutant derivatives thereof; an enzyme, which is capable of inactivating a toxic compound, preferably blasticidin-S-deaminase, puromycin-N-acetyltransferase, neomycin phosphotransferase, hygromycin B phosphotransferase and mutant derivatives thereof, an enzyme, which is capable of converting pro-drug/toxin-mediated toxicity, preferably thymidine kinase and mutant derivatives thereof and a small-molecule sensor protein, preferably calmodulin, troponin C, S100 and mutant derivatives thereof.

The nucleic acid according to the present invention also can be a nucleic acid which comprises or consists of any of SEQ ID NOs: 5 to 22. The nucleic acid according to the present invention also can be a nucleic acid which consists of any of SEQ ID NOs: 5 to 22. The nucleic acid according to the present invention can be a nucleic acid according to SEQ ID NO: 5. The nucleic acid according to the present invention can be a nucleic acid according to SEQ ID NO: 6. The nucleic acid according to the present invention can be a nucleic acid according to SEQ ID NO: 7. The nucleic acid according to the present invention can be a nucleic acid according to SEQ ID NO: 8. The nucleic acid according to the present invention can be a nucleic acid according to SEQ ID NO: 9. The nucleic acid according to the present invention can be a nucleic acid according to SEQ ID NO: 10. The nucleic acid according to the present invention can be a nucleic acid according to SEQ ID NO: 11. The nucleic acid according to the present invention can be a nucleic acid according to SEQ ID NO: 12. The nucleic acid according to the present invention can be a nucleic acid according to SEQ ID NO: 13. The nucleic acid according to the present invention can be a nucleic acid according to SEQ ID NO: 14. The nucleic acid according to the present invention can be a nucleic acid according to SEQ ID NO: 15. The nucleic acid according to the present invention can be a nucleic acid according to SEQ ID NO: 16. The nucleic acid according to the present invention can be a nucleic acid according to SEQ ID NO: 17. The nucleic acid according to the present invention can be a nucleic acid according to SEQ ID NO: 18. The nucleic acid according to the present invention can be a nucleic acid according to SEQ ID NO: 19. The nucleic acid according to the present invention can be a nucleic acid according to SEQ ID NO: 20. The nucleic acid according to the present invention can be a nucleic acid according to SEQ ID NO: 21. The nucleic acid according to the present invention can be a nucleic acid according to SEQ ID NO: 22.

Further, the present invention also comprises a vector comprising any of the nucleic acids as described herein above. Exemplary vectors are shown in SEQ ID NOs: 44 to 61. The vector according to the present invention may comprise SEQ ID NO: 44. The vector according to the present invention may comprise SEQ ID NO: 45. The vector according to the present invention may comprise SEQ ID NO: 46. The vector according to the present invention may comprise SEQ ID NO: 47. The vector according to the present invention may comprise SEQ ID NO: 48. The vector according to the present invention may comprise SEQ ID NO: 49. The vector according to the present invention may comprise SEQ ID NO: 50. The vector according to the present invention may comprise SEQ ID NO: 51. The vector according to the present invention may comprise SEQ ID NO: 52. The vector according to the present invention may comprise SEQ ID NO: 53. The vector according to the present invention may comprise SEQ ID NO: 54. The vector according to the present invention may comprise SEQ ID NO: 55. The vector according to the present invention may comprise SEQ ID NO: 56. The vector according to the present invention may comprise SEQ ID NO: 57. The vector according to the present invention may comprise SEQ ID NO: 58. The vector according to the present invention may comprise SEQ ID NO: 59. The vector according to the present invention may comprise SEQ ID NO: 60. The vector according to the present invention may comprise SEQ ID NO: 61. The vector according to the present invention may be according to SEQ ID NO: 44. The vector according to the present invention may be according to SEQ ID NO: 45. The vector according to the present invention may be according to SEQ ID NO: 46. The vector according to the present invention may be according to SEQ ID NO: 47. The vector according to the present invention may be according to SEQ ID NO: 48. The vector according to the present invention may be according to SEQ ID NO: 49. The vector according to the present invention may be according to SEQ ID NO: 50. The vector according to the present invention may be according to SEQ ID NO: 51. The vector according to the present invention may be according to SEQ ID NO: 52. The vector according to the present invention may be according to SEQ ID NO: 53. The vector according to the present invention may be according to SEQ ID NO: 54. The vector according to the present invention may be according to SEQ ID NO: 55. The vector according to the present invention may be according to SEQ ID NO: 56. The vector according to the present invention may be according to SEQ ID NO: 57. The vector according to the present invention may be according to SEQ ID NO: 58. The vector according to the present invention may be according to SEQ ID NO: 59. The vector according to the present invention may be according to SEQ ID NO: 60. The vector according to the present invention may be according to SEQ ID NO: 61.

The present invention further provides a host cell comprising any of the nucleic acids according to the present invention or any of the vectors according to the present invention as described herein.

The present invention also comprises the use of any of the nucleic acids according to the present invention as described herein for detecting a specific splice events as defined herein.

The present invention also comprises the use of any of the vectors according to the present invention as described herein for detecting a specific splice events as defined herein.

The present invention also comprises the use of the host cell according to the present invention as described herein for tracking splice events.

The present invention also comprises any of the uses as described above, wherein the nucleic acid, vector or the host cell is additionally for enriching cells.

Further, the present invention provides the nucleic acid according to the present invention as described herein, for use in the treatment or prevention of a disease, wherein the disease is preferably selected from the group consisting of retinopathies, tauopathies, motor neuron diseases, muscular diseases, neurodevelopmental and neurodegenerative diseases, more preferably from the group consisting of cystic fibrosis, retinitis pigmentosa, myotonic dystrophy, Alzheimer's disease and Parkinson's disease.

Further, the present invention provides the vector according to the present invention as described herein, for use in the treatment or prevention of a disease, wherein the disease is preferably selected from the group consisting of retinopathies, tauopathies, motor neuron diseases, muscular diseases, neurodevelopmental and neurodegenerative diseases, more preferably from the group consisting of cystic fibrosis, retinitis pigmentosa, myotonic dystrophy, Alzheimer's disease and Parkinson's disease.

Further, the present invention provides the host cell according to the present invention as described herein, for use in the treatment or prevention of a disease, wherein the disease is preferably selected from the group consisting of retinopathies, tauopathies, motor neuron diseases, muscular diseases, neurodevelopmental and neurodegenerative diseases, more preferably from the group consisting of cystic fibrosis, retinitis pigmentosa, myotonic dystrophy, Alzheimer's disease and Parkinson's disease.

The present invention further provides a kit for detecting a specific splice event of a gene of interest, which comprises:
- a first plasmid, wherein a split intein-heterologous polynucleotide construct is inserted and wherein the split intein comprises an N-terminal splicing region upstream of the heterologous polynucleotide and a C-terminal splicing region downstream of the heterologous polynucleotide;
- a second plasmid coding for a guided endonuclease, preferably wherein the endonuclease is selected from the group consisting of Cas9, Cas12a, TALENs, ZFNs and meganucleases; and
- a third plasmid encoding for Cre/Flp recombinases.
Further, the kit may provide a plasmid consisting of homology arms, and/or a temporary selection cassette, which may be afterwards removed by site-specific recombinases. Alternative to the second plasmid the kit may contain means for delivering the endonuclease, such as TALENs, ZFNs or meganucleases or RNPs, such as Cas9 or Cas12a (Cpf1) with a protein/RNP delivery method of choice. The second plasmid encoding for Cre/Flp recombinases may be for removing the selection cassette after selection. Alternative thereto recombinant proteins and protein delivery method of choice may be used. Optionally, a further plasmid may be included into the kit coding for Cas9 or encoding for proteins which enhances homology directed repair alias homologous recombination (HDR) or suppresses non-homologous end-joining (NHEJ) (Canny *et al*.).

The present invention further relates to a method (e.g., in vitro, ex vivo method) of protein-level quantification (e.g., non-invasive protein-level quantification) of co-translation regulation (e.g., as described in Fig. 7 and Example 6 described herein below).

### EXAMPLES

### Materials and methods

### Molecular cloning

### PCR for molecular cloning:

Single-stranded primer deoxyribonucleotides were diluted to 100 µM in nuclease-free water (Integrated DNA Technology (IDT)). PCR reaction with plasmid and genomic template was performed with Q5 Hot Start High-Fidelity 2x Master Mix or with 5x High-Fidelity DNA Polymerase and 5x GC-enhancer (New England Biolabs (NEB)) according to manufacturer's protocol. Samples were purified by gel DNA agarose gel-electrophoresis and subsequent purification using Monarch^{®} DNA Gel Extraction Kit (NEB).

### DNA digestion with restriction endonucleases:

Samples were digested with NEB restriction enzymes according to manufacturer's protocol in a total volume of 40 µl with 2-3 µg of plasmid DNA. Afterwards, fragments were gel purified by gel DNA agarose gel-electrophoresis and subsequent purification using Monarch^{®} DNA Gel Extraction Kit (NEB).

### Molecular cloning using DNA ligases and Gibson assembly

Agarose-gel purified DNA fragment concentrations were determined by a spectrophotometer (NanoDrop 1000, Thermo Fisher Scientific). Ligations were carried out with 50-100 ng backbone-DNA (DNA fragment containing the ori) in 20 µl volume, with molar 1:1-3 backbone:insert ratios, using T4 DNA ligase (Quick Ligation^{™} Kit, NEB) at room temperature for 5-10 min. Gibson assemblies were performed with 75 ng backbone DNA in a 15 µl reaction volume and a molar 1:1-5 backbone:insert ratios, using NEBuilder^{®} HiFi DNA Assembly Master Mix (2x) (NEB) for 20-60 min at 50 °C.

### DNA agarose gel-electrophoresis

Gels were prepared with 1% agarose (Agarose Standard, Carl Roth) in 1x TAE-buffer and 1:10.000 SYBR Safe stain (Thermo Fisher Scientific), running for 20-40 min at 120 V. For analysis 1 kb Plus DNA Ladder (NEB) was used. Samples were mixed with Gel Loading Dye (Purple, 6x) (NEB).

### Bacteria strains for molecular cloning

Chemically- and electrocompetent Turbo/Stable cells (NEB) were used for transformation of circular plasmid DNA. For plasmid amplification, carbenicillin (Carl Roth) was used as selection agent at a final concentration at 100 µg/ml. All bacterial cells were incubated in Lysogeny Broth-Medium (LB) and on LB agar plates including proper antibiotic selection agents.

### Bacterial transformation with plasmid DNA

For electroporation, either 5 µl Ligation or Gibson reaction was dialyzed against MilliQ water for 10-20 min on an MF-Millipore membrane filter (Merck). Afterward, 5 µl dialysate was mixed with 50 µl of thawed, electrocompetent cells, transferred to a pre-cooled 2 mm electroporation cuvette (Bio-Rad), shocked at 2.5 kV (Gene Pulser Xcell^{™} Electroporation Systems, Bio-Rad) and immediately mixed with 950 µl SOC-medium (NEB). Chemical transformation was performed by mixing 5 µl of Ligation or Gibson reaction with 50 µl thawed, chemically competent cells and incubated on ice for 30 min. Cells were then heat shocked at 42 °C for 30 s, further incubated on ice for 5 min and finally mixed with 950 µl SOC-medium (NEB). Transformed cells were then plated on agar plates containing the appropriate type of antibiotic and concentrations according to cell supplier's information. Plates were incubated overnight at 37 °C or over the weekend at room temperature.

### Plasmid DNA purification and Sanger-sequencing

Plasmid DNA transformed clones were picked and inoculated from agar plates in 2 ml LB medium with appropriate antibiotics and incubated for about 6 h (NEB Turbo) or overnight (NEB Stable). Plasmid DNA intended for sequencing or molecular cloning was purified with QIAprep Plasmid MiniSpin (QIAGEN) according to manufacturer's protocol. Clones that were intended to be used in cell culture experiments were inoculated in 100 ml antibiotic-medium and grown overnight at 37 °C containing the appropriate antibiotic. Plasmid DNA was purified with Plasmid Maxi Kit (QIAGEN). Plasmids were sent for Sanger-sequencing (GATC-Biotech) and analyzed by Geneious Prime (Biomatters) sequence alignments.

### Mammalian cell culture

### Cell lines and cultivation

All experiments were performed with HEK293T (ECACC: 12022001, Sigma-Aldrich) cells. Cells were maintained at 37 °C, in 5% CO₂, H₂O saturated atmosphere were in advanced Gibco^{™} Advanced DMEM (Gibco^{™}, Thermo Fisher Scientific) supplemented with 10% FBS (Gibco^{™}, Thermo Fisher Scientific), GlutaMAX (Gibco^{™}, Thermo Fisher Scientific) and penicillin-streptomycin (Gibco^{™}, Thermo Fisher Scientific) at 100 µg/ml at 37 °C and 5% CO₂. Cells were passaged at 90% confluency by sucking off the medium, washing with DPBS (Gibco^{™}, Thermo Fisher Scientific) and separating the cell with 2.5 ml of a Accutase^{®} solution (Gibco^{™}, Thermo Fisher Scientific). Cells were then incubated for 5-10 min at room temperature until visible detachment of the cells and subsequently, the Accutase^{™} was inactivated by adding 7.5 ml prewarmed DMEM including 10% FBS and all supplements. Cells were then transferred in appropriate density into a new flask or counted and plated on 96-well, 48-well or 6-well format for plasmid transfection.

### Plasmid transfection

Cells were transfected with X-tremeGENE HP (Roche) according to the protocol of the manufacturer. DNA amounts were kept constant in all transient experiments to yield reproducible complex formation and comparable results. In 96-well plate experiments, a total amount of 100 ng of plasmid DNA was used, in 48-well plates, a total amount of 300 ng of plasmid DNA was used and in 6-well plates, a total amount of 2.4 µg of plasmid DNA was used. Cells were plated one day before transfection (25 000 cells/well in 100 µl for 96-well plates, 75 000 cells/well in 500 µl for 48-well plates, 600 000 cells/well in 3 ml for 6-well plate). 24 h post-transfection, 100 µl fresh medium was added on 96-well transfection per well, 48 h post-transfection 100 µL medium was removed and replaced with fresh medium on 96-well transfections per well.

### Generation of stable cell lines with tagged exons via CRISPR/Cas9

A stable HEK293T cell line was generated with plasmids expressing a mammalian codon-optimized Cas9 from S. pyogenes (SpCas9, SpyCas9) or S. aureus (SaCas9, SauCas9) with a tandem C-terminal SV40 nuclear localization signal (SV40 NLS) or a triple tandem NLS (SV40 NLS + c-myc NLS + synthetic NLS) via a CBh hybrid RNA-polymerase II promoter and human U6 driving a single-guide-RNA (sgRNA, gRNA) for SpyCas9/SauCas9 with a 19-21 bp cloned protospacer targeting the exon 10 of MAPT. The efficiency of CRISPR/Cas9 for a target site was performed by T7 endonuclease I assay (NEB) after manufacturer's protocol after 48-72 h post-transfection of cells with plasmids encoding Cas9 and the targeting sgRNA on a 48-well plate. Optionally, a modified plasmid encoding for SpyCas/SauCas9 system together with i53 expression (a genetically encoded 53bp1 inhibitor) was transfected to enhance homologous recombination (HR) after Cas9-mediated double-strand break at the protospacer-guided genomic site. Donor DNA plasmid contains the intein-flanked moiety including the selection-cassette to select for cells undergoing successful Cas9-mediated HR.

48 hours post-transfection (48-well or 6-well format), the medium was replaced with medium containing 50 µg/ml puromycin, if not otherwise indicated. Cells were daily observed and cells were detached with Accutase^{™} and replated with puromycin when surviving colonies reaches the colony size of about 50 cells. This step was repeated until no significant puromycin-mediated cell death could be observed. Those cells were plated without puromycin on a 48-well plate and were transfected with a CAG-hybrid promoter-driven nuclear-localized Cre (SEQ ID NO: 41) or Flp recombinase (SEQ ID NO: 42) with and a low amount of a green fluorescent protein (Xpa-H62Q) in a 10:1 ratio. The green fluorescent protein was co-transfected in order to enrich cells successfully co-transfected with the recombinase (SEQ ID NO: 42) expressing plasmid. Green cells were enriched with the BD FACSaria II controlled with the BD FACSDiva Software (Version 6.1.3, BD Biosciences) and replated on a suitable dish/plate.

After one week, enriched cells were single-cell-sorted in 96-well plates and grown mono-clonally until colony size were big enough to be duplicated onto a second 96-well plate containing 2 µg/ml puromycin. Cells which underwent successful cassette excision should not survive puromycin treatment indicating that the original clone from which it was duplicated did not anymore contain the puromycin-N-acetyltransferase and was a potential candidate for genotyping for zygosity. Those clones were detached and expanded on 48-well plates until confluency and half of the cell mass was then used subsequently for isolation of genomic DNA using Wizard^{®} Genomic DNA Purification Kit (Promega). Genotyping of the genomic DNA was performed using LongAmp^{®} Hot Start Taq 2x Master Mix (NEB) after manufacturer's protocol with primer deoxynucleotides pairs (IDT) with at least one primer binding outside of the homology arms. The PCR product from clones where the genotyping indicates homozygosity were sent for Sanger-sequencing to verify its sequence integrity.

### Gene expression manipulation with CRISPR/Cas9

Gene expression of MAPT (TAU) was enforced in HEK293T cells by co-transfecting CAG-driven mammalian-codon optimized nuclease-defect *S*. *pyogenes* Cas9 (D10A, H840A) (SEQ ID NO: 65) fused to a tripartite trans-activation domain and SV40 NLS (Chavez et al.) with three protospacer-truncated sgRNAs (14-15 nt protospacer instead of 19-21) targeting the 5'-upstream region of the MAPT transcription start site (TSS).

### mRNA manipulation with CRISPR/Cas13

CAG-driven mammalian codon-optimized RfxCas13d (Cas13d from Ruminococcus flavefaciens XPD3002) (Konermann *et al.)* with a C-terminal triple NLS (SV40 NLS + c-myc NLS + synthetic NLS) or PspCas13b (Cas13b from Prevotella sp. P5-125) (Cox *et al.)* with a C-terminal nuclear export signal from HIV Rev protein were co-transfected with a plasmid encoding for the crRNA of the Cas13 system (human U6 RNA polymerase III driven) targeting the RNA of interest indicated in the figures.

### mRNA manipulation with artificial microRNAs

CAG-driven mammalian codon-optimized iRFP720 were intersected with a modified intron derived from rabbit beta-globin. Within the synthetic intron the artificial mir-30-based synthetic micoRNA backbone containing the critical region for efficient microRNA biogenesis were embedded (Fellmann *et al*.)*.* Guide sequences were designed with the help of SplashRNA (Pelossof *et al*.) and cloned intron-embedded microRNA backbone with type IIS restriction enzymes.

### KO of MBNL1 and MBNL2 with CRISPR/Cas9

To knock-out MBNL1/2 (mucleblind-like protein 1/2, SEQ ID NO: 62 and SEQ ID NO: 63) in HEK293T cell cells which carries a blasticidin resistance gene flanked by inteins within the FOXP1 exon 18b, two plasmids expressing a mammalian codon-optimized Cas9 from *S*. *pyogenes* (SpCas9, SpyCas9) with a tandem C-terminal SV40 nuclear localization signal (SV40 NLS) via a CBh hybrid RNA-polymerase II promoter and human U6 driving a single-guide-RNA (sgRNA, gRNA) for SpyCas9 (SEQ ID NO: 23) with a cloned protospacer targeting MBNL1 and MBNL2 were co-transfected into the cells. 72 h later, cells were replated in a proper format and medium were supplemented with indicated blasticidin concentration. Control condition were transfected with the same conditions but the sgRNA is targeting the control locus AAVS1 (PPP1R12C) (safe-harbor locus AAVSI intronic region, SEQ ID NO: 64). Genomic DNA was isolated from blasticidin-treated surviving colonies with Wizard^{®} Genomic DNA Purification Kit (Promega).

### Proteinbiochemical analysis

### Immunoblot analysis

Cells were lysed with a proper volume of M-PER (Thermo Fisher Scientific) including protease inhibitors (Halt Protease Inhibitor Cocktail, Thermo Fisher Scientific) according to manufacturer's protocol. Cleared lysate were then equalized against the relative protein concentration determined using NanoDrop 1000 (Thermo Fisher Scientific) and diluted with M-PER. Equalized lysates were prepared for SDS-gel-electrophoresis using XT Sample Buffer (Bio-Rad) and XT Reducing Agent (Bio-Rad) and denaturated at 70 °C for 10 min or 95 °C for 5 min. Samples were loaded in 18-well 4-12% Criterion^{™} XT Bis-Tris Protein Gel and electrophoresis was run at 150 V for 1.5 hours in XT MOPS Running Buffer (Bio-Rad). Subsequently, an immunoblot was performed onto a Immobilon^{®}-P PVDF membrane (Merck) with a wet blotting system (Criterion^{™} Blotter, Bio-Rad) in ice-cold Towbin buffer (Bio-Rad) with 20% Methanol (Carl Roth) overnight (15 V, 4 °C). Afterward, the free valences on the PVDF membrane was blocked in blocking buffer containing 5% skimmed milk (Carl Roth) in TBS-T (pH 7.6) with 0.1% Tween-20 (Sigma-Aldrich) at room temperature for 1 h. Antibodies were diluted at 1:1000 (only anti-pan-TAU, PC1C6, Merck, was diluted 1:200) in blocking buffer and either incubated at room temperature for 2 hours or overnight at 4 °C, followed by at least 3 washing steps (room temperature, 5 min, 60 rpm). The HRP-conjugated secondary antibody (Abcam) was also diluted in blocking buffer (1:10 000 - 1:20 000) and subsequently again washed with TBS-T for at least four times. HRP-detection was performed with the SuperSignal^{™} West Femto Maximum Sensitivity Substrate (Thermo Fisher Scientific) on a Fusion FX7/SL advanced imaging system (Vilber Lourmat).

Used primary and secondary antibodies were: Mouse M2 anti-FLAG (Sigma-Aldrich), rat L6 anti-OLLAS (Thermo Fisher Scientific), mouse PC1C6 anti-pan-TAU (Merck), rat EPR4114 anti-FOXP1 (abcam), mouse 32F6 anti-mNeonGreen (ChromoTek), rabbit anti-firefly luciferase (ab21176, abcam), rabbit D71G9 anti-TUBB3 (Cell Signaling Technology (CST)), mouse AC-15 anti-beta-Actin (HRP) (abcam), goat anti-mouse IgG H&L (HRP) (ab97023, abcam), goat anti-rat IgG H&L (HRP) (ab97057, abcam) and goat anti-rabbit IgG H&L (HRP) (ab6721, abcam).

### Fluorescence and chemo/bioluminescence detection

### Immunofluorescence labeling

Medium from cells for immunofluorescence were removed and washed with DPBS (Gibco^{™}, Thermo Fisher Scientifc) and fixed for 15 min in 10% neutral buffered formalin (Sigma-Aldrich) at room temperature. Primary antibodies with indicated concentration were diluted 1:1000 in BSA blocking buffer (only anti-pan-TAU, PC1C6, Merck, was diluted 1:200). Blocking buffer was prepared using DPBS (Gibco^{™}, Thermo Fisher Scientific) with 1% BSA (Sigma-Aldrich) containing 0.5% Triton X-100 (Sigma-Aldrich). Cells were washed 3x after fixation with DPBS (Gibco^{™}, Thermo Fisher Scientific) for 5 min at room temperature and blocking buffer containing the suitable fluorescent dye coupled secondary antibodies (1:1000, Thermo Fisher Scientific) were applied for 2 hours at room temperature or overnight at 4 °C.

Used primary and secondary antibodies were: mouse PC1C6 anti-pan-TAU (Merck), rabbit D71G9 anti-TUBB3 (Cell Signaling Technology (CST), Cy3-conjugated cross-adsorbed goat anti-mouse IgG (H+L) (Thermo Fisher Scientific), Cy5-conjugated cross-adsorbed goat anti-mouse IgG (H+L) (Thermo Fisher Scientific) and Alexa Fluor 633-conjugated cross-adsorbed goat anti-rabbit IgG (H+L) (Thermo Fisher Scientific).

### Epifluorescence microscopy

Epifluorescence microscope images were taken on an Invitrogen^{™} EVOS^{™} FL Auto Cell Imaging System (Thermo Fisher Scientific) under non-saturating conditions and every sample to be compared were taken with the same parameters and saved as uncompressed *.tiff files.

### Confocal microscopy

Confocal microscopy was conducted on a Leica SP5 system (Leica Microsystems) under non-saturating conditions; filters and excitation wavelength were chosen in a way that crosstalk between fluorescent moieties of interest were excluded or minimal. Images were saved as *.tiff files. For life-imaging of cells, medium was changed to warm phenol-red-free DMEM/F12 supplemented with HEPES (Gibco^{™}, Thermo Fisher Scientific) and the 37 °C 5% CO₂ air ventilation system was switched on.

### Bioluminescence microscopy

Bioluminescence life-imaging was performed on a LV200 bioluminescence imaging system (Olympus) under non-saturating conditions in 8-well µ-slides (Ibidi) and images were saved as uncompressed *.tiff files. NanoLuc substrate was delivered with the Nano-Glo^{®} Live Cell Assay System (Promega) after the manufacturer's protocol. Images were analyzed with Fiji ImageJ.

### Bioluminescence quantification

For bioluminescence bulk quantifications, cells were plated and transfected in 96-well format. For NanoLuc bioluminescence on-plate detection, medium was removed until 100 µl/well of medium was remained 72 hours post-transfection and detected with the Nano-Glo^{®} Luciferase Assay System (Promega) on the Centro LB 960 (Berthold Technologies) plate reader with 0.1 s acquisition time. For simultaneous detection of firefly and NanoLuc luciferases, medium was removed until 80 µl/well medium was left. Sequential detection of FLuc and NanoLuc was performed using the Nano-Glo^{®} Dual-Luciferase^{®} Reporter Assay System (Promega) on the Centro LB 960 (Berthold Technologies) plate reader with 0.5 s acquisition time after 10 min of reagent addition for FLuc and 20 min of reagent addition for NanoLuc.

### Example 1: Scarless internal labelling of Tubb3 exon 2 shows proof-of-principle

As a first proof-of-concept, the inventors flanked a green fluorescent protein (mNeonGreen, mNG) with a gp41-1 split-intein pair (SEQ ID NO: 16) corresponding to homology arms and knocked this construct in Tubb3 Exon 2 in mouse neuroblastoma cells (Neuro-2a, N2a) in front of a serine using CRISPR/Cas9 (**Figure 1a**). Tubb3 is highly expressed in N2a cells and cells with the integrated reporter could thus be sorted via FACS for monoclonalization. After genotyping, a hemizygous clone E12 (**Figure 1a**) was selected for further analysis. Anti-OLLAS immunoblot of E12 indicated successful post-translational excision of the integrated gp41-1 intein flanked mNG (43 kDa, **Figure 1a**) and anti-Tubb3 immunoblot shows that Tubb3 gene shows the expected size (50 kDa, **Figure 1a**). No fusion band (83 kDa) could be observed suggesting a fast and efficient protein splicing of the translation product. Since the analyzed clone E12 does not possess a WT allele (all alleles are either knock-in alleles for intein-mNG and did not anymore contain a non-functional deletion allele, N2a cells are highly polyploid), the 50 kDa Tubb3 product, therefore, was a result of intein-mediated protein ligation of the N- and C-extein part of Tubb3. Also, anti-Tubb3 staining of the clone showed typical microtubule pattern and was independent of the strong uniform mNeonGreen signal indicating successful excision of the intein-flanked reporter (**Figure 1b**).

### Example 2: Introduction of anti-parallel orthogonal coiled-coil domains improves the protein ligation

After showing in cellulo with this minimal construct according to Example 1 that an exon can be tagged with an intein-flanked reporter, we sought to optimize the splicing efficiency even more for more challenging exteins (fast folding proteins) by introducing orthogonal synthetic anti-parallel coiled-coil (CC) domains, enabling fast co-folding of the split intein binary complex and thus fast excision of the intein moieties thereby averting potentially disturbance of the tagged protein's folding process (**Figure 1a**).

The most challenging scenario is when the tagged protein folds much faster than the intein-reporter moiety. The native tertiary structure of the tagged protein might prevent the intein part from taking its final active form to excise itself out from the tagged protein and being thus trapped in its fusion form. To mimic this worst case scenario, the inventors tagged the fast folding green fluorescent protein mNeonGreen (~ 10 min) with a Nanoluc luciferase (NLuc) flanked by gp41-1 split inteins with (SEQ ID NO: 22) and without (SEQ ID NO: 21) artificial anti-parallel CCs (**Figure 1a**). The inventors saw for the test constructs besides the product band (full-length mNeonGreen) also higher MW educt bands (mNeonGreen with intein-NLuc still inserted) (**Figure 1a**). The introduction of coiled coil domains improved the splicing efficiency by ~5.5-fold, so that the inventors kept the design with the CCs in all subsequent exon-tagging constructs.

### Example 3: Using type II and type I transmembrane domains with a covalent binding domain to couple exon inclusion with membrane functionalization enabling membrane functionalization and non-consumptive sampling of exon-inclusion

Using imaging modalities, one would be able to observe an exon-of-interest (EOI), but not to enrich the population of cells which expresses a EOI. This is of fundamental importance for questions where it is required to isolate cells expressing the EOI within a heterogeneous cell population upon a specific trigger or an (epi)genetic modification, as an example from a CRISPR-library screening, for subsequent analysis, e.g. transcriptomics or proteomics analysis. For that reason, the inventors created a system, where the translation of an EOI will result in a presentation of a moiety to the extracellular environment which can be subsequently tagged with fluorescent dyes for imaging or fluorescence-activated cell sorting (FACS) or can be harnessed to enrich the EOI-expressing cells via magnetic cell separation systems (MACS). To achieve this, the inventors constructed a reporter protein between the intein-coiled-coil domains which is presented subsequently on the extracellular site.

This was challenging at first sight, since classical single-pass-transmembrane proteins (both type I and type II) have one terminus on the extracellular lumenal site and one on the intracellular cytosolic site. Moreover, type I transmembrane domains require an N-terminal start-transfer signal in form of a signal peptide, which is cleaved off afterwards rendering it useless as an internal tagging system between the inteins. Nevertheless, by combining a type II transmembrane domain, which itself codes for a start-transfer signal and a membrane-anchor (type II TMD), followed by a type I stop-transfer signal (type I TMD), one can translocate a extracellular moiety by embedding it between the two TMDs (**Figure 1a**).

Since the extracellular domain should be able to bind small molecules with exceptional affinity, the inventors tested two different strategies: The first approach was to use a pseudo-tetrameric single-chain avidin (scAvidin) (Nordlund *et al.*)*,* where one chain encodes for four circularly permuted avidins, each one being able to bind one biotin-functionalized ligand with picomolar affinity (in contrast, engineered monomeric variants only have nanomolar affinity (Nordlund *et al*.)*.* The second approach uses a HaloTag, an engineered version of a chloroalkane dehalogenase from Rhodococcus rhodochrous, which is able to bind chloroalkanes covalently (Los *et al.*)*.* The inventors introduced two further mutations C61V and C262A into the HaloTag to remove the cysteins, which might form unwanted disulfide bonds in the ER and in the oxidative extracellular environment and thus will be trapped in the ER due to misfolding and degraded via ERAD as shown before for cysteine-containing fluorescent proteins translocated to the secretory pathways (Costantini *et al*.).

To translocate those binding entities, the desired extracellular region has to be preceded as mentioned before by type II transmembrane domain (TMD), which channels the succeeding protein sequence to the ER until a stop transfer signal in form of a type I transmembrane segment is reached, afterwards the rest of the protein is again translated into the cytosolic compartment (**Figure 1a**). The inventors decided to use the mouse Fcer2 membrane-spanning region as the type II TMD and also adopt the flanking amino acids since the N-terminally positively charged amino acids on the N-terminal (cytoplasmic) site ensure proper domain topology ("positive-inside rule") and two palmitoylable cysteines might also improve membrane association and topology. Human GYPA TMD was used as a prototypical type I TMD since it contains positively charged amino acids C-terminally (cytosolic site) after the TMD and to prevent unwanted homo-dimerization, a G102L mutation was additionally introduced to disrupt the GxxxG TMD-dimerization motif. Also known motifs to enhance plasma membrane trafficking (PMTS) (Gradinaru *et al*.) after ER translocation has been introduced C-terminally after the type I TMD.

To test this complex approach, test constructs were made by insertion of the intein-CC-TMD-flanked scAvidin (SEQ ID NO: 17) or HaloTag (SEQ ID NO: 18) within N- and C-mNeonGreen. Transfection of the test constructs into HEK-293T revealed that both strategies work at least in transient transfections, both constructs could be either live-stained with biotinylated AF594 (scAvidin) or chloroalkane-AF660 (HaloTag) (**Figure 1b**).

Since transient transfection does not always represent the behavior of stably integrated constructs in a low-copy allele-integrated physiological expression level, we knocked-in the HaloTag-based strategy into MAPT exon 10 **(****Figure 2a**). Again, after CRISPR/Cas9-mediated integration, puromycin selection and cassette removal, individual clones were selected which were genotyped as homozygous for reporter integration.

Anti-pan-Tau immunofluorescence shows typical microtubule-associated staining after MAPT induction for HEK-293T WT cells and reporter-embedded cells indicating successful excision of the membrane-anchored constructs **(****Figure 2c**).

Also, anti-pan-Tau immunoblot revealed that despite the huge payload in exon 10 size, no change in pan-Tau splicing could be observed compared to WT HEK-293T cells **(****Figure 2d****).** Again, anti-pan-TAU immunoblot revealed the scarless-nature of our approach, even with a challenging membrane-associated reporter, and showed typical TAU ladder pattern **(****Figure 3**). Cells could be live-stained with chloroalkane-AF66 after MAPT induction indicating successful membrane-trafficking of the reporter. The covalent nature of HaloTag to its chloroalkane-functionalized ligands and its relatively smaller size might contribute to its performance.

In summary, the inventors were able to create a tool that presents a binding moiety on the cell surface upon the expression of a protein isoform, which subsequently can be non-invasively stained with non-membrane-permeable substrates. Those cells can be enriched via commercially available systems (MACS) or by FACS.

This system can be converted into a system for the non-consumptive sampling of the supernatant to measure exon inclusion of a gene of interest (GOI). By first exchanging the binding moiety with a luciferase (here: Nanoluc) and flanking it with furin sites (SEQ ID NO: 20), this translation product should release the furin-sites flanked Nanoluc into the extracellular environment. The release is mediated by furin, a Golgi-network-resident pro-protein-convertase. As shown in **Figure 3**, the test constructs with furin sites show a decent increase in detectable Nanoluc signal in the supernatant compared to the control constructs without furin sites (SEQ ID NO: 19). This allows to measure the integrated signal of exon inclusion during certain windows of observation.

### Example 4: Scarless exon 10 tagging of 4R Tau (MAPT exon 10) shows that disease-relevant isoforms can be tracked quantitatively with cellular resolution and manipulated using CRISPR/Cas13

One of the most prominent cases for the misregulation of alternative splicing resulting in a disease phenotype are tauopathies caused by mutations in the microtubule-associated protein TAU (MAPT). TAU protein is expressed primarily in neurons with their main function to mediate microtubule polymerization and stabilization (Mietelska-Porowska *et al*.).

Structurally, TAU is a natively unfolded highly soluble protein (Bolós *et al.,* and Fitzpatrick *et al*.) with six TAU isoforms, which are expressed in the adult human central nervous system, produced by alternative splicing of exons 2, 3 and 10 (**Figure 2a**). Alternative splicing of exon 10 leads to a protein containing either three (3R; exon 10 exclusion) or four (4R; exon 10 inclusion) tandem repeats of a microtubule-binding motif (Wang, Mandelkow *et al.*)*.* Many mutations in exon 10 or in the intronic region around exon 10 lead to an increase of 4R isoforms containing exon 10, thus causing an imbalance of 4R/3R-ratio. This 4R/3R misbalance leads to a neurodegenerative phenotype classified as tauopathies by aggregated 4R Tau proteins. Thus, the inventors tagged exon 10 of MAPT as a prototype example for alternative splicing and its impact on neurodegenerative diseases to test and establish the splice reporter according to the present invention. The inventors used Nanoluc luciferase as the first-choice reporter instead of a classic fluorescent protein, since bioluminescence detection is linear over several orders of magnitude and additionally has an exceptional signal-to-noise ratio (S/R) and sensitivity and moreover also allow bulk quantification and imaging with single-cell resolution via bioluminescence imaging (BLI).

To integrate the optimized NLuc-based reporter (SEQ ID NO: 8) into exon 10 of MAPT in human HEK-293T cells via CRISPR/Cas9 before Ser-293 (nomenclature of aa positions refers to 2N4R Tau isoform), the inventors also introduced a FRT-F3 sites flanked puromycin resistance cassette into the construct, which is subsequently genetically excised via Flp recombinase after puromycin selection, to enrich cells carrying the reporter construct in MAPT exon 10 **(****Figure 2a**). After genotyping for its zygosity and puromycin cassette excision, three homozygous clones were selected for further analysis (89F9, D7F4, and ETEB; clone D7G2 will not be discussed in the following since it was still resistant after Flp). Since MAPT is not or only weakly expressed in HEK-293T cells, we induced TAU expression by RNA-guided transactivators (TAs) targeting the upstream 5' region of the transcription start site (TSS) of MAPT using dCas9-VPR (Chavez *et al.*) together with 3 gRNAs (**Figure 2b**). Successful induction could be observed via anti-pan-Tau immunoblot showing the 6 main adult isoforms of Tau. Most strikingly, no obvious difference in splicing pattern could be observed when compared to unmodified HEK-293T cells showing that the modification is minimally invasive (**Figure 2d**). Also, anti-OLLAS (indicator for intein-Nluc) immunoblot reveals a band of the expected size of the excised reporter only in the condition where MAPT is induced. The inventors also performed an additional RT-qPCR that induction could also be observed on the mRNA level with similar induction levels for HEK-293T WT cells and the modified B9F9 clone (**Figure 2c**).

Already without RNA-guided induction, ETEB shows the six adult isoforms including the 4R isoforms in contrast to both other clones B9F9 and D7F4 (**Fig**. **2d**). Since the reporter carries an NLuc between the split-intein moieties (**Fig**. **2a**), the inventors could also follow the induction of 4R Tau via luciferase assay, a high-throughput compatible format (**Fig**. **2e**).

Again, the inventors saw that clone E7E8 already showed some NLuc expression without RNA-guided MAPT induction, in accordance to the immunoblot analysis and after induction, the inventors could observe a robust increase in NLuc activity for the clones (**Fig**. **2e**). Additionally, it was demonstrated that by using luciferase it is also possible to measure the bulk signal from a population of cells in a typical luciferase assay format but can also track isoform-specific signal with cellular resolution within a living heterogeneous cell population.

After RNA-guided induction of the MAPT locus, the inventors could observe an increase in NLuc luminescence representing 4R Tau expression with cellular resolution (**Fig. 2f**). Also anti-Tau immunofluorescence staining of the cells revealed that there was no difference in staining pattern (microtubule-associated staining) after MAPT induction in HEK-293T cells and 4R-tagged cells, again emphasizing that the method is minimally invasive (**Fig. 2g**).

Since immunoblot/immunofluorescence analysis of Tau and NLuc activity do correlate with each other, one can conclude that this reporter might enable high-throughput screenings for lead compounds suppressing the disease-relevant MAPT exon 10 inclusion and thus 4R expression.

To verify that indeed by suppressing 4R Tau results in a loss of NLuc signal, the inventors applied the latest generation of RNA-targeting tools using CRISPR/Cas13 effectors whether they were able to decrease 4R Tau level (**Fig. 2h**). The inventors tested the recently discovered Cas13b from Prevotella sp. P5-125 (PspCasl3b) (Cox *et al.*) and Cas13d from Ruminococcus flavefaciens XPD3002 (RfxCasl3d) (Konermann *et al.*) either fused to a nuclear localization signal (NLS) or nuclear export signal (NES) and check for NLuc activity after the induction of MAPT. crRNAs were designed such that it binds to the coding sequence of exon 10 independent from intein-NLuc integration. First, the inventors found out that in accordance to the initial reports from Cas13b (Cox *et al.*) and Cas13d (Konermann *et al*.), that cytosolic PspCasl3b (NES-fusion) is more potent than the nuclear-localized one (NLS-fusion) and that RfxCasl3d-NLS, on the contrary, is better than RfxCasl3d-NES in silencing a targeted RNA (**Fig**. **2i**). Since both RNA targeting activities of the Cas13 system are Mg²⁺-dependent, it might be a preference of both nucleases for a certain concentration of Mg²⁺ in the nuclear or cytosolic environment explaining the discrepancy of the preference for one compartment. It might also be important that we removed a cryptic Pol III termination signal (Gao *et al.*) within the PspCas13b crRNA scaffold (Cox *et al*.) and therefore increased full-length Cas13b crRNA expression.

Summarized, PspCas13b-NES with the improved crRNA scaffold was more potent in silencing 4R MAPT mRNA than PspCas13d-NLS and the inventors could show that RNA-targeting activity of the Cas13 system, in general, can be harnessed to deplete 4R expression. One important note is that if a discrimination between different isoforms is required, one should choose PspCas13b-NES, since only cytosolic effectors are exclusively targeting mature isoform-specific mRNA, whereas nuclear-localized effectors would degrade all pre-mRNAs. Targeting exon-spanning regions might also allow nuclear-localized effectors to discriminate only mature mRNAs.

In order to quantify in detail regarding if pan-TAU is decreased or increased or only a specific isoform such as 4R isoform is solely modified, the inventors used a second orthogonal firefly luciferase in a constitutive exon to monitor pan-TAU level.

The first strategy the inventors tested was knocking in a FLuc-SUMO fusion upstream of the ATG start-codon, which should be subsequently cleaved off scarlessly by ubiquitous expressed Ulps/SENPs (ubiquitin-like protease/sentrin-specific protease). FLuc-SUMO-tagged TAU could not be detected after CRISPR/dCas9-VPR-NLS mediated induction and further analysis revealed a massive destabilization of mRNA since a test construct with FLuc-SUMO2-0N3R-TAU showed a >99% cleavage efficiency expressed transiently from a CAG-promoter-driven plasmid. The reason for destabilization might be a result from detrimental change in the 5'-UTR region, such as secondary structures, resulting in a worsened translation initiation, which will eventuate in NMD of the mRNA and thus low mRNA/protein abundance since the exon-junction complexes are also less efficiently displaced (the main contributor to NMD, (Maquat et al.)).

Encouraged by this observation that knocking-in into a translated region (**Fig**. **2c**) does not obviously change the mRNA half-life, The inventors knocked-in a second orthogonal firefly luciferase (FLuc) into a constitutive exon (exon 5 or exon 11) flanked by a second pair of fast-splicing inteins (N- and C-NrdJ-1 split-intein, SEQ ID NO: 1 and SEQ ID NO: 3) and an orthogonal coiled coil pair (P3 and AP4) to further increase orthogonality (SEQ ID NO: 6).

To show that indeed a isoform-specific signal can be distinguished from pan-TAU signal, the inventors again used PspCas13b-NES, RfxCas13d-NLS variants and artificial microRNAs against different sites on the (pre-)mRNA of MAPT. When using Cas13d-NLS to target the exon 10 in the nucleus, so potentially targeting pre-mRNA and mature mRNAs, all isoforms are knocked-down as the FLuc and Nanoluc signal decreases, whereas Cas13b-NES was able to knock-down 4R specifically in the cytosol. In theory, Cas13d can also be harnessed to target the recent spliced mRNA by using the exon-junction region as a targeting region, but the exon-junction-complexes which reside 20-24 nt upstream of exon-exon-junction might sterically interfere with the binding of Cas13d. Since the Cas13d crRNA targeting the exon 9/10 junction is symmetric and Cas13d targets only the last 15 nt of the exon 9, it should still be targetable. As expected, crRNA targeting this junction using Cas 13d-NLS was able to deplete 4R TAU selectively but not as efficient as Cas13-NES or amiRNAs, where the latter one was the most potent in knocking down selectively the 4R isoform. Surprisingly, the amiRNA targeting the exon 10/11 junction was also knocking down all TAU isoforms. A detailed analysis showed that the microRNA targeting the 10/11 junction is not symmetric (18 nts in exon 11 and 4 nts in exon 10) and thus was able to target the exon 9/11 (3R isoform) junction, since the microRNA's 5' seed region was fully matching.

Summarized, the inventors were able to quantify and modulate protein isoform expression of MAPT using the latest generation of RNA-targeting technology and showed the importance and effects of targeting the pre-mRNA (nucleus) or the mature mRNA in the cytosol.

### Example 5: Coupling the inclusion of an exon with a titratable cell-survival marker using inteins, one can identify new regulators of alternative splicing

An alternative approach, to enrich cells which expresses a certain EOI (exon of interest) might be achieved by coupling isoform expression to the fitness of a cell. As a proof-of-concept, the inventors took advantage of the knowledge that MBNL1 and 2 suppress FOXP1 exon 1Bb stem-cell specific isoforms in non-stem-cells, i.e. also in HEK-293T cells (Gabut *et al.,* and Han *et al*.).

The idea behind coupling the expression of an EOI to the fitness of a cell is that one could apply a whole genome CRISPR KO- or activation-library on the cells and sequence the surviving population which is expected to carry mutations (KO of splice suppressors or activation of splice activators) enabling the expression of the EOI in a direct or indirect manner.

Therefore, the inventors created a cell line for proofing if blasticidin-S resistance was established. The test therefore was negative.

Next, the inventors checked whether the KO of reported suppressors of FOXP1 exon 18b by knocking out their constitutive exon of MBNL1 and MBNL2 are able to confer survival of the cells upon treatment with bs compared to a control condition where gRNA targeting the safe-harbor AAVS1 locus (PPP1R12C) was transfected instead. Upon treatment with bs ranging from 5 to 12.5 µg/mL, colonies were formed in the condition transfected with Cas9 and gRNAs targeting MBNL1 and MBNL2, whereas no survival could be observed in the control condition targeting AAVS1 (**Fig**. **3b**).

Also as expected, no cells survived in unmodified HEK-293T cells regardless targeting MBNL1 and MBNL2 or AAVS1 (**Fig**. **3b**). To further ensure that indeed KO of MBNL1 and MBNL2 is responsible for detoxifying bs via exon 18b inclusion, the inventors performed a semiquantitative RT-PCR of the surviving cells after selection and could clearly see that exon 18b inclusion could only be observed upon KO of MBNL1/2 in a bs-dose dependent manner (**Fig**. **3c**, **upper panel**).

The inventors also analyzed the MBNL1 and MBNL2 locus upon selection with different concentration of antibiotics and could see a clear dose-dependent enrichment of cells carrying MBNL1 and MBNL2 KO. Only 10.2% of WT allele could be observed in the 12.5 µg/mL selection condition whereas 57.6% of the MBNL1 locus was still WT upon treatment with the minimal lethal condition indicating a strong correlation between MBNL1 KO and FOXPI exon 18b inclusion and thus cell survival. For MBNL2 the inventors also observed an enrichment but did not observe a greater enrichment from 12.5 µg/mL compared to 8.5 µg/ml indicating that MBNL1 is that main effector in suppressing exon 18b inclusion (**Fig**. **3d**).

Immunoblot analysis of FOXP1 of the cells showed that there was no difference in WT and bsd-intein reporter cells and anti-OLLAS (indicating the intein-fused reporter) again showed its ability to splice itself from its precursor protein (**Fig**. **3c**). All in all, using this method, we confirmed the reports, that MBNL1 is indeed a major suppressor of FOXP1 exon 18b and showed a proof-of-concept that this method can be in general applied for CRISPR screenings to find direct or indirect regulators of an EOI by insertion of a survival factor flanked by inteins into the EOI, and moreover the stringency of the selection (e.g., the impact of a regulator) could be fine-tuned by simply the concentration of the selection agent.

### Example 6: Non-invasive protein-level quantification of co-translation regulation

Ribosomal frameshift-mediated regulations cannot be monitored by RT-qPCR or other RNA-based quantification methods. Exemplary, Oaz1, the key enzyme in polyamine biosynthesis (**Fig**. **7a**) was chosen, since it was known to be regulated by polyamine. gp41-1 split-inteins-flanked mNeonGreen and NrdJ-1-split-inteins-flanked mTagBFP2 were inserted into the full-length Oaz1 gene and transfected cells were treated with increasing polyamine concentrations to determine whether frameshift regulation could be read out via fluorescence quantification (**Fig**. **7b**). FACS analysis revealed that the stop codon readthrough was stimulated by increasing spermidine or spermine concentrations (**Fig**. **7c**) which could be verified by immunoblot analysis from the bulk lysate of the corresponding conditions (**Fig**. **7d**).

### Example 7: Discussion

In short, the inventors developed a toolkit to label an exon-of-interest using fast-splicing inteins for detailed investigation of alternative splicing. Using a luciferase-based reporter integrated into MAPT exon 10, the inventors showed that one can comfortably measure 4R Tau expression in an HTS-compatible format without changing any amino acids of TAU, thereby not changing TAU's biochemical properties. At the same time, the inventors also show via RT-qPCR, immunoblot, immunofluorescence and luciferase assays that by carefully tagging exon 10 on the DNA/RNA level, no obvious change regarding expression level, splicing pattern, and localization compared to WT cells could be observed. Since luciferase assays are a classic HTS-preferred method due to high sensitivity, excellent S/R-ratio, and scalability, this might enable screening for lead structures of small molecules capable to modulate alternative splicing which seem to play a major role in many neurodegenerative diseases (Luo *et al.,* Bruch, Xu, De Andrade *et al.,* Rottscholl *et al.,* and Bruch, Xu, Rösler *et al*.).

For a more gene-therapeutic approach, the inventors found out that the recently discovered CRISPR/Cas13 RNA-guided RNA-targeting effectors suit to deplete its target-mRNA in our hands and if its activity can be tracked via our 4R reporter-readout. The inventors confirm that the two latest Cas13 effectors PspCas13b and RfxCas13d were able to deplete 4R Tau, in which PspCas13b works best in a cytosolic environment and RfxCas13d in a nuclear environment and PspCas13b-NES was the most effective in our setup. Also, the inventors show that using the same luciferase-based reporter, one can track down the signal with cellular resolution via bioluminescence imaging (BLI), where one can observe potential cell heterogeneity.

However, to isolate the EOI-expressing cells from a heterogeneous cell population for further transcriptomic or proteomic investigation, we created a dual-transmembrane-domain anchored HaloTag-presenting system which is presented on the cell surface upon the inclusion of an EOI. The inventors also confirmed for this reporter system that it is minimally invasive by tagging 4R Tau, followed by MAPT induction and anti-TAU immunoblot/-fluorescence. Additionally, the inventors showed that the Halotag is successfully presented on the cell surface via live-cell staining with a dye-labeled HaloTag ligand. Using commercially available kits, one can pull down those cells due to EOI-dependent cell surface functionalization either by chloroalkane-functionalized affinity matrices or by common streptavidin-functionalized matrices, in which the cells are pre-incubated with chloroalkane-biotin ligands and pulled-down afterward via streptavidin-beads (e.g. MACS systems). To be noted is that the addition of two short furin recognition sites between dual-membrane-anchor flanking the reporter moiety (e.g. NLuc again is the preferred reporter here) on the extracellular site converts this system into an exon-dependent secretable reporter since during trafficking through the ER-Golgi-network, the furin-flanked reporter would be excised and secreted into the extracellular milieu. For whole genome CRISPR-KO or activation approaches to identify regulators of alternative splicing, the inventors showed that by tagging an EOI minimal-invasively with inteins and a detoxifying enzyme, one can couple cell-survival via in-/exclusion of an exon, enabling easy enrichment of the desired cell population for NGS. Proof-of-concept is shown by tagging the stem-cell specific exon 18b of FOXP1, which is normally suppressed in non-stem-cells by MBNL1 and MBNL2, with an intein-flanked blasticidin-S deaminase enzyme (detoxifies blasticidin). After CRISPR/Cas9 KO of MBNL1 and MBNL2 compared to a gRNA targeting an unrelated AAVS1 control locus, surviving cells only could be observed in cells where MBNL1 and MBNL2 were targeted. The exon-dependent survival was verified by blasticidin dose-dependent cell survival and enrichment of MBNL1 and MBNL2 KO alleles.

Hereby, this tool enables genome-wide KO (splice suppressors) or activator (splice activators) screen for regulators of an EOI. This system can be also inverted using pro-toxin converting enzymes, e.g. using thymidine kinases from Herpesviridae (HSV-tk) and titration using ganciclovir which is converted HSV-tk into its toxic form. For example, if a exon-of-interest is expessed in a cell-line, one can knock-in a intein-flanked HSV-tk into the exon and apply a lentiviral CRISPR/cDNA library on the cells. Cells were a true negative regulator (cDNA library) of the EOI is expressed, the cells will skip this exon and will survive pro-drug treatment with ganciclovir. Also, if a splice activator is knocked-out by the CRISPR library, the exon is also skipped and thereby the cells will survive pro-toxin treatment. All in all, the inventors demonstrated a new intein-based toolkit for understanding and manipulating alternative splicing in a minimally-invasive manner.

### REFERENCES

Anderson, P., & Kedersha, N. (2006). RNA granules. J Cell Biol, 172(6), 803-808.
Ballatore, C., Lee, V. M. Y., & Trojanowski, J. Q. (2007). Tau-mediated neurodegeneration in Alzheimer's disease and related disorders. Nature Reviews Neuroscience, 8(9), 663.
Bolós, M., Pallas-Bazarra, N., Terreros-Roncal, J., Perea, J. R., Jurado-Arjona, J., Ávila, J., & Llorens-Martín, M. (2017). Soluble Tau has devastating effects on the structural plasticity of hippocampal granule neurons. Translational psychiatry, 7(12), 1267.
Bruch, J., Xu, H., De Andrade, A., & Hoglinger, G. (2014). Mitochondrial complex 1 inhibition increases 4-repeat isoform tau by SRSF2 upregulation. PloS one, 9(11), e113070.
Bruch, J., Xu, H., Rösler, T. W., De Andrade, A., Kuhn, P. H., Lichtenthaler, S. F., ... & Hoglinger, G. U. (2017). PERK activation mitigates tau pathology in vitro and in vivo. EMBO molecular medicine, 9(3), 371-384.
Canny, M. D., Moatti, N., Wan, L. C., Fradet-Turcotte, A., Krasner, D., Mateos-Gomez, P. A., ... & Noordermeer, S. M. (2018). Inhibition of 53BP1 favors homology-dependent DNA repair and increases CRISPR-Cas9 genome-editing efficiency. Nature biotechnology, 36(1), 95.
Chavez, A., Scheiman, J., Vora, S., Pruitt, B. W., Tuttle, M., Iyer, E. P., ... & Ter-Ovanesyan, D. (2015). Highly efficient Cas9-mediated transcriptional programming. Nature methods, 12(4), 326.
Costantini, L. M., Baloban, M., Markwardt, M. L., Rizzo, M., Guo, F., Verkhusha, V. V., & Snapp, E. L. (2015). A palette of fluorescent proteins optimized for diverse cellular environments. Nature communications, 6, 7670.
Cox, D. B., Gootenberg, J. S., Abudayyeh, O. O., Franklin, B., Kellner, M. J., Joung, J., & Zhang, F. (2017). RNA editing with CRISPR-Cas13. Science, 358(6366), 1019-1027.
Daguenet, E., Dujardin, G., & Valcárcel, J. (2015). The pathogenicity of splicing defects: mechanistic insights into pre-mRNA processing inform novel therapeutic approaches. EMBO reports, 16(12), 1640-1655.
Deshpande, A., Win, K. M., & Busciglio, J. (2008). Tau isoform expression and regulation in human cortical neurons. The FASEB Journal, 22(7), 2357-2367.
Fellmann, C., Hoffmann, T., Sridhar, V., Hopfgartner, B., Muhar, M., Roth, M., ... & Sinha, N. (2013). An optimized microRNA backbone for effective single-copy RNAi. Cell reports, 5(6), 1704-1713.
Fitzpatrick, A. W., Falcon, B., He, S., Murzin, A. G., Murshudov, G., Garringer, H. J., ... & Scheres, S. H. (2017). Cryo-EM structures of tau filaments from Alzheimer's disease. Nature, 547(7662), 185.
Gabut, M., Samavarchi-Tehrani, P., Wang, X., Slobodeniuc, V., O'Hanlon, D., Sung, H. K., ... & Nedelec, S. (2011). An alternative splicing switch regulates embryonic stem cell pluripotency and reprogramming. Cell, 147(1), 132-146.
Gao, Z., Herrera-Carrillo, E., & Berkhout, B. (2018). Delineation of the exact transcription termination signal for type 3 polymerase III. Molecular Therapy-Nucleic Acids, 10, 36-44.
Goedert, M., & Jakes, R. (2005). Mutations causing neurodegenerative tauopathies. Biochimica et Biophysica Acta (BBA)-Molecular Basis of Disease, 1739(2-3), 240-250.
Gradinaru, V., Zhang, F., Ramakrishnan, C., Mattis, J., Prakash, R., Diester, I., ... & Deisseroth, K. (2010). Molecular and cellular approaches for diversifying and extending optogenetics. Cell, 141(1), 154-165.
Han, H., Irimia, M., Ross, P. J., Sung, H. K., Alipanahi, B., David, L., ... & Wang, E. (2013). MBNL proteins repress ES-cell-specific alternative splicing and reprogramming. Nature, 498(7453), 241.
Kwok, J. B., Teber, E. T., Loy, C., Hallupp, M., Nicholson, G., Mellick, G. D., ... & Schofield, P. R. (2004). Tau haplotypes regulate transcription and are associated with Parkinson's disease. Annals of Neurology: Official Journal of the American Neurological Association and the Child Neurology Society, 55(3), 329-334.
Konermann, S., Lotfy, P., Brideau, N. J., Oki, J., Shokhirev, M. N., & Hsu, P. D. (2018). Transcriptome engineering with RNA-targeting type VI-D CRISPR effectors. Cell, 173(3), 665-676.
Lathuilière, A., Valdés, P., Papin, S., Cacquevel, M., Maclachlan, C., Knott, G. W., ... & Schneider, B. L. (2017). Motifs in the tau protein that control binding to microtubules and aggregation determine pathological effects. Scientific reports, 7(1), 13556.
Licatalosi, D. D., and Darnell, R. B., Splicing Regulation in Neurologic Disease, (2006) Neuron 52, 93-101.
Lopez-Bigas, N., Audit, B., Ouzounis, C., Parra, G., & Guigó, R. (2005). Are splicing mutations the most frequent cause of hereditary disease?. FEBS letters, 579(9), 1900-1903.
Los, G. V., Encell, L. P., McDougall, M. G., Hartzell, D. D., Karassina, N., Zimprich, C., ... & Simpson, D. (2008). HaloTag: a novel protein labeling technology for cell imaging and protein analysis. ACS chemical biology, 3(6), 373-382.
Luo, Y., & Disney, M. D. (2014). Bottom-up Design of Small Molecules that Stimulate Exon 10 Skipping in Mutant MAPT Pre-mRNA. Chembiochem, 15(14), 2041-2044.
Maquat, L. E., Tarn, W. Y., & Isken, O. (2010). The pioneer round of translation: features and functions. Cell, 142(3), 368-374.
Mietelska-Porowska, A., Wasik, U., Goras, M., Filipek, A., & Niewiadomska, G. (2014). Tau protein modifications and interactions: their role in function and dysfunction. International journal of molecular sciences, 15(3), 4671-4713.
Nordlund, H. R., Hytönen, V. P., Hörhä, J., Määttä, J. A., White, D. J., Halling, K., ... & Kulomaa, M. S. (2005). Tetravalent single-chain avidin: from subunits to protein domains via circularly permuted avidins. Biochemical journal, 392(3), 485-491.
Pelossof, R., Fairchild, L., Huang, C. H., Widmer, C., Sreedharan, V. T., Sinha, N., ... & Hoffmann, T. (2017). Prediction of potent shRNAs with a sequential classification algorithm. Nature biotechnology, 35(4), 350.
Porensky, P. N., & Burghes, A. H. (2013). Antisense oligonucleotides for the treatment of spinal muscular atrophy. Human gene therapy, 24(5), 489-498.
Poulos, M. G., Batra, R., Charizanis, K., and Swanson, M. S., Developments in RNA splicing and Disease, (2011) Cold Spring Harbor Perspect. Biol., 3:a000778
Rottscholl, R., Haegele, M., Jainsch, B., Xu, H., Respondek, G., Hollerhage, M., ... & Schmitz-Afonso, I. (2016). Chronic consumption of Annona muricata juice triggers and aggravates cerebral tau phosphorylation in wild-type and MAPT transgenic mice. Journal of neurochemistry, 139(4), 624-639.
Shaner, N. C., Lambert, G. G., Chammas, A., Ni, Y., Cranfill, P. J., Baird, M. A., ... & Davidson, M. W. (2013). A bright monomeric green fluorescent protein derived from Branchiostoma lanceolatum. Nature methods, 10(5), 407.
Spencer, P. S., Siller, E., Anderson, J. F., & Barral, J. M. (2012). Silent substitutions predictably alter translation elongation rates and protein folding efficiencies. Journal of molecular biology, 422(3), 328-335.
Stoilov, P., Lin, C. H., Damoiseaux, R., Nikolic, J., & Black, D. L. (2008). A high-throughput screening strategy identifies cardiotonic steroids as alternative splicing modulators. Proceedings of the National Academy of Sciences.
Wang, E. T., Sandberg, R., Luo, S., Khrebtukova, I., Zhang, L., Mayr, C., ... & Burge, C. B. (2008). Alternative isoform regulation in human tissue transcriptomes. Nature, 456(7221), 470.
Wang, Y., & Mandelkow, E. (2016). Tau in physiology and pathology. Nature Reviews Neuroscience, 17(1), 22.
Wurster, C. D., & Ludolph, A. C. (2018). Antisense oligonucleotides in neurological disorders. Therapeutic advances in neurological disorders, 11, 1756286418776932.
Zhang, M. L., Lorson, C. L., Androphy, E. J., & Zhou, J. (2001). An in vivo reporter system for measuring increased inclusion of exon 7 in SMN2 mRNA: potential therapy of SMA. Gene therapy, 8(20), 1532.

## Claims

1. A method for detecting a specific splice event of a gene of interest,
wherein the specific splice event creates a specific splice product, which comprises an exon of interest,
wherein the method comprises:
(i) Inserting a split intein - heterologous polynucleotide construct into the exon of interest,
wherein the split intein comprises an N-terminal splicing region upstream of the heterologous polynucleotide and a C-terminal splicing region downstream of the heterologous polynucleotide; and
(ii) detecting the heterologous polynucleotide and the expression product of the heterologous polynucleotide,
wherein the expression product of the split intein - heterologous polynucleotide construct excises itself from the expression product of the specific splice product at a position, wherein the amino acid C-terminal to this position is a cysteine, a serine or a threonine.

2. Method according to claim 1, wherein the expression product of the specific splice product is a single polypeptide chain, and/or wherein the expression product of the N-terminal splicing region of the split intein comprises at its N-terminus a cysteine or a serine, and/or wherein the expression product of the C-terminal splicing region of the split intein comprises at its C-terminus an asparagine, and/or wherein the heterologous polynucleotide encodes a protein or enzyme selected from the group consisting of a fluorescent protein, preferably green fluorescent protein; a bioluminescence-generating enzyme, preferably NanoLuc, NanoKAZ, Cypridina, Firefly, Renilla luciferase or mutant derivatives thereof; an enzyme, which is capable of generating a colored pigment, preferably tyrosinase or an enzyme of a multi-enzymatic process, more preferably the violacein or betanidin synthesis process, a genetically encoded receptor for multimodal contrast agents, preferably Avidin, Streptavidin or HaloTag or mutant derivatives thereof; an enzyme, which is capable of converting a non-reporter molecule into a reporter molecule, preferably TEV protease and picornaviral proteases, more preferably rhinoviral 3C proteases and polioviral 3C protease, SUMO proteases and mutant derivatives thereof; an enzyme, which is capable of inactivating a toxic compound, preferably blasticidin-S-deaminase, puromycin-N-acetyltransferase, neomycin phosphotransferase, hygromycin B phosphotransferase and mutant derivatives thereof, an enzyme, which is capable of converting pro-drug/toxin-mediated toxicity, preferably thymidine kinase and mutant derivatives thereof and a small-molecule sensor protein, preferably calmodulin, troponin C, S100 and mutant derivatives thereof.

3. Method according to any one of the preceding claims, wherein the split intein - heterologous polynucleotide construct further contains at least one polynucleotide encoding for a hetero-dimerizing domain or a homo-dimerizing domain, preferably at least one PDZ-domain or at least one coiled-coil-domain, more preferably two coiled-coil-domains in an antiparallel configuration, for accelerating the specific splice event, and/or wherein the split intein - heterologous polynucleotide construct further contains at least one polynucleotide encoding for a hetero-dimerizing domain or a homo-dimerizing domain, preferably at least one PDZ-domain or at least one coiled-coil-domain, more preferably two coiled-coil-domains in an antiparallel configuration, for accelerating the self-excision of the expression product of the split intein - heterologous polynucleotide construct from the expression product of the specific splice product, and/or wherein the heterologous polynucleotide of the split intein - heterologous polynucleotide construct further contains a temporary selection marker for stable cell line generation, and/or wherein detecting the heterologous polynucleotide and the expression product of the heterologous polynucleotide is carried out by any method selected from the group consisting of high-throughput screening, western blotting, mass spectrometry, luciferase-assays, and longitudinal live-imaging, preferably bioluminescence imaging, fluorescence imaging, photoacoustic imaging, MRI and PET.

4. Method according to any one of the preceding claims, wherein the method is non- or minimally invasive for the protein of interest such that a native and/or fully functional protein of interest is expressed compared to the protein of interest without insertion of the split intein - heterologous polynucleotide construct according to the method of any one of claims 1 to 3.

5. Method according to any one of the preceding claims, wherein a further heterologous polynucleotide encoding for a reporter enzyme, which is preferably selected from the group consisting of a fluorescent protein, a bioluminescence-generating enzyme, more preferably a luciferase enzyme, is inserted into a constitutively expressed exon of the gene of interest, wherein said further heterologous polynucleotide encoding for a reporter enzyme is different from the heterologous polynucleotide as defined in any one of claims 1 to 4.

6. Method according to claim 5, wherein the split intein - heterologous polynucleotide construct further comprises a polynucleotide encoding for a protein which functions as an activator of the further heterologous polynucleotide encoding for a reporter enzyme or as an activator of the heterologous polynucleotide of the split intein - heterologous polynucleotide construct.

7. Method according to any one of the preceding claims, wherein the method further comprises (iii) quantification of an isoform population of the protein of interest encoded by the gene of interest, and/or wherein the heterologous polypeptide of the split intein - heterologous polynucleotide construct is an antibiotic resistance gene and wherein the method alternatively to step (ii) or additionally to step (ii) comprises detecting the antibiotic resistance of the cells of interest comprising the protein of interest encoded by the gene of interest, and/or wherein the method alternatively to step (ii) or additionally to step (ii) comprises the detection of an isoform dependent cell-surface marker, and/or wherein the method further comprises (iii) manipulation of the folding process of the protein of interest encoded by the gene of interest, and/or wherein the method further comprises (iii) manipulation of the kinetics of the splice event of the gene of interest, preferably wherein the kinetics of the specific splice event is manipulated due to step (ii), and/or wherein the method further comprises (iii) enrichment of cells comprising the protein of interest encoded by the gene of interest, preferably enrichment of cells comprising a specific isoform of the protein of interest, and/or wherein the method further comprises (iii) modification of the folding process of the protein of interest, and/or wherein the method further comprises (iii) quantification of the protein of interest encoded by the gene of interest or quantification of the exon of interest.

8. Method according to any one of the preceding claims, wherein the method further comprises (iii) identification of a regulator of the inclusion or exclusion of the exon of interest, preferably identification of a regulator of the inclusion or exclusion of the exon of interest of a pre-mRNA, preferably wherein the regulator regulates alternative splicing of a non-constitutive exon, and/or preferably wherein the method further comprises the application of a CRISPR-library or cDNA library, and/or preferably wherein the method further comprises (iv) inactivation or activation of the regulator, more preferably inactivation of the regulator, even more preferably inactivation of the regulator by a toxic compound, wherein the toxic compound is selected from the group consisting of puromycin, blasticidin-S, neomycin, hygromycin and derivatives thereof and pro-drug/toxins, more preferably ganciclovir, acyclovir or derivatives thereof, more preferably wherein the method further comprises (v) detection of the survival of the cell comprising the protein of interest encoded by the gene of interest, even more preferably wherein the survival of the cell is detected by applying toxic compounds, even more preferably wherein the toxic compound is selected from the group consisting of puromycin, blasticidin-S, neomycin, hygromycin and derivatives thereof and pro-drug/toxins, even more preferably ganciclovir, acyclovir or derivatives thereof.

9. Method according to any one of the preceding claims, wherein the N-terminal splicing region of the split intein comprises or consists of the NrdJ-1 N-terminal region (SEQ ID NO: 1) or the gp41-1 N-terminal region (SEQ ID NO: 2), and/or wherein the C-terminal splicing region of the split intein comprises or consists of the NrdJ-1 C-terminal region (SEQ ID NO: 3) or the gp41-1 C-terminal region (SEQ ID NO: 4), and/or wherein the split intein is gp41-1 or NrdJ-1.

10. A nucleic acid encoding a split intein - heterologous polynucleotide construct, wherein the split intein comprises an N-terminal splicing region upstream of the heterologous polynucleotide and a C-terminal splicing region downstream of the heterologous polynucleotide,
wherein the heterologous polynucleotide encodes a protein or enzyme selected from the group consisting of a fluorescent protein, preferably a green fluorescent protein; a bioluminescence-generating enzyme, preferably NanoLuc, NanoKAZ, Cypridina, Firefly, Renilla luciferase or mutant derivatives thereof; an enzyme, which is capable of generating a colored pigment, preferably tyrosinase or an enzyme of a multi-enzymatic process, more preferably the violacein or betanidin synthesis process; a genetically encoded receptor for multimodal contrast agents, preferably Avidin, Streptavidin or HaloTag or mutant derivatives thereof; an enzyme, which is capable of converting a non-reporter molecule into a reporter molecule, preferably TEV protease and picornaviral proteases, more preferably rhinoviral 3C proteases and polioviral 3C protease, SUMO proteases and mutant derivatives thereof; an enzyme, which is capable of inactivating a toxic compound, preferably blasticidin-S-deaminase, puromycin-N-acetyltransferase, neomycin phosphotransferase, hygromycin B phosphotransferase and mutant derivatives thereof, an enzyme, which is capable of converting pro-drug/toxin-mediated toxicity, preferably thymidine kinase and mutant derivatives thereof and a small-molecule sensor protein, preferably calmodulin, troponin C, S100 and mutant derivatives thereof, preferably wherein the nucleic acid comprises or consists of any of SEQ ID NOs: 5 to 22.

11. A vector comprising the nucleic acid of claim 10.

12. A host cell comprising the nucleic acid of claim 10 or the vector of claim 11.

13. Use of the nucleic acid of claim 10, the vector of claim 11 or the host cell of claim 12 for detecting splice events, preferably wherein the nucleic acid, vector or the host cell is additionally for enriching cells.

14. The nucleic acid of claim 10, the vector of claim 11 or the host cell of claim 12 for use in the treatment or prevention of a disease, wherein the disease is preferably selected from the group consisting of retinopathies, tauopathies, motor neuron diseases, muscular diseases, neurodevelopmental and neurodegenerative diseases, more preferably from the group consisting of cystic fibrosis, retinitis pigmentosa, myotonic dystrophy, Alzheimer's disease and Parkinson's disease.

15. Kit for detecting a specific splice event of a gene of interest comprising:
- a first plasmid, wherein a split intein-heterologous polynucleotide construct is inserted and wherein the split intein comprises an N-terminal splicing region upstream of the heterologous polynucleotide and a C-terminal splicing region downstream of the heterologous polynucleotide;
- a second plasmid coding for a guided endonuclease, preferably wherein the endonuclease is selected from the group consisting of Cas9, Cas12a, TALENs, ZFNs and meganucleases; and
- a third plasmid encoding for Cre/Flp recombinases.

## Patentansprüche

1. Verfahren zum Nachweisen eines spezifischen Spleißereignisses eines Gens von Interesse, wobei das spezifische Spleißereignis ein spezifisches Spleißprodukt erzeugt, das ein Exon von Interesse umfasst,
wobei das Verfahren umfasst:
(i) Insertieren eines Konstrukts aus gespaltenem Intein und heterologem Polynukleotid in das Exon von Interesse,
wobei das gespaltene Intein eine N-terminale Spleißregion stromaufwärts des heterologen Polynukleotids und eine C-terminale Spleißregion stromabwärts des heterologen Polynukleotids umfasst; und
(ii) Nachweisen des heterologen Polynukleotids und des Expressionsprodukts des heterologen Polynukleotids,
wobei das Expressionsprodukt des Konstrukts aus gespaltenem Intein und heterologem Polynukleotid sich selbst aus dem Expressionsprodukt des spezifischen Spleißprodukts an einer Position ausschneidet, an der die Aminosäure C-terminal zu dieser Position ein Cystein, ein Serin oder ein Threonin ist.

2. Verfahren nach Anspruch 1, wobei das Expressionsprodukt des spezifischen Spleißprodukts eine einzelne Polypeptidkette ist, und/oder wobei das Expressionsprodukt der N-terminalen Spleißregion des gespaltenen Inteins an seinem N-Terminus ein Cystein oder Serin umfasst, und/oder wobei das Expressionsprodukt der C-terminalen Spleißregion des gespaltenen Inteins an seinem C-Terminus ein Asparagin umfasst, und/oder wobei das heterologe Polynukleotid für ein Protein oder Enzym kodiert, das aus der Gruppe, bestehend aus einem fluoreszierenden Protein, vorzugsweise dem grün fluoreszierenden Protein; einem Biolumineszenz erzeugenden Enzym, vorzugsweise NanoLuc-, NanoKAZ-, Cypridina-, Leuchtkäfer-, Renilla-Luciferase oder mutierte Derivate davon; einem Enzym, das ein farbiges Pigment erzeugen kann, vorzugsweise Tyrosinase oder ein Enzym eines multi-enzymatischen Prozesses, bevorzugter des Violacein- oder Betanidin-Syntheseprozesses, einem genetisch kodierten Rezeptor für multimodale Kontrastmittel, vorzugsweise Avidin, Streptavidin oder HaloTag oder mutierte Derivate davon; einem Enzym, das ein Nicht-Reportermolekül in ein Reportermolekül umwandeln kann, vorzugsweise TEV-Protease und picornavirale Proteasen, bevorzugter rhinovirale 3C-Proteasen und poliovirale 3C-Protease, SUMO-Proteasen und mutierte Derivate davon; einem Enzym, das eine toxische Verbindung inaktivieren kann, vorzugsweise Blasticidin-S-Deaminase, Puromycin-N-Acetyltransferase, Neomycin-Phosphotransferase, Hygromycin-B-Phosphotransferase und mutierte Derivate davon; einem Enzym, das Prodrug/Toxinvermittelte Toxizität umwandeln kann, vorzugsweise Thymidin-Kinase und mutierte Derivate davon, und einem niedermolekularen Sensorprotein, vorzugsweise Calmodulin, Troponin C, S100 und mutierte Derivate davon, ausgewählt ist.

3. Verfahren nach einem der vorhergehenden Ansprüche, wobei das Konstrukt aus gespaltenem Intein und heterologem Polynukleotid ferner mindestens ein Polynukleotid enthält, das für eine heterodimerisierende Domäne oder eine homodimerisierende Domäne kodiert, vorzugsweise mindestens eine PDZ-Domäne oder mindestens eine Coiled-Coil-Domäne, bevorzugter zwei Coiled-Coil-Domänen in antiparalleler Konfiguration, zum Beschleunigen des spezifischen Spleißereignisses, und/oder wobei das Konstrukt aus gespaltenem Intein und heterologem Polynukleotid ferner mindestens ein Polynukleotid enthält, das für eine heterodimerisierende Domäne oder eine homodimerisierende Domäne kodiert, vorzugsweise mindestens eine PDZ-Domäne oder mindestens eine Coiled-Coil-Domäne, bevorzugter zwei Coiled-Coil-Domänen in antiparalleler Konfiguration, zur Beschleunigung des Selbstausschneidens des Expressionsprodukts des Konstrukts aus gespaltenem Intein und heterologem Polynukleotid aus dem Expressionsprodukt des spezifischen Spleißprodukts, und/oder wobei das heterologe Polynukleotid des Konstrukts aus gespaltenem Intein und heterologem Polynukleotid ferner einen temporären Selektionsmarker für die Erzeugung einer stabilen Zelllinie enthält, und/oder wobei das Nachweisen des heterologen Polynukleotids und des Expressionsprodukts des heterologen Polynukleotids durch ein beliebiges Verfahren durchgeführt wird, das aus der Gruppe, bestehend aus Hochdurchsatz-Screening, Western Blotting, Massenspektrometrie, Luciferase-Assays und longitudinalem Live-Imaging, vorzugsweise Biolumineszenz-Imaging, Fluoreszenz-Imaging, photoakustischem Imaging, MRT und PET ausgewählt ist.

4. Verfahren nach einem der vorhergehenden Ansprüche, wobei das Verfahren für das Protein von Interesse nicht oder nur minimal invasiv ist, sodass ein natives und/oder voll funktionsfähiges Protein von Interesse verglichen mit dem Protein von Interesse ohne Insertion des Konstrukts aus gespaltenem Intein und heterologem Polynukleotid gemäß dem Verfahren nach einem der Ansprüche 1 bis 3 exprimiert wird.

5. Verfahren nach einem der vorhergehenden Ansprüche, wobei ein weiteres heterologes Polynukleotid, das für ein Reporterenzym kodiert, das vorzugsweise aus der Gruppe, bestehend aus einem fluoreszierenden Protein, einem Biolumineszenz erzeugenden Enzym, bevorzugter einem Luciferase-Enzym ausgewählt ist, in ein konstitutiv exprimiertes Exon des Gens von Interesse insertiert wird, wobei das weitere heterologe Polynukleotid, das für ein Reporterenzym kodiert, sich von dem heterologen Polynukleotid nach einem der Ansprüche 1 bis 4 unterscheidet.

6. Verfahren nach Anspruch 5, wobei das Konstrukt aus gespaltenem Intein und heterologem Polynukleotid ferner ein Polynukleotid umfasst, das für ein Protein kodiert, das als ein Aktivator des weiteren heterologen Polynukleotids, das für ein Reporterenzym kodiert, oder als Aktivator des heterologen Polynukleotids des Konstrukts aus gespaltenem Intein und heterologem Polynukleotid wirkt.

7. Verfahren nach einem der vorhergehenden Ansprüche, wobei das Verfahren ferner (iii) die Quantifizierung einer Isoformpopulation des durch das Gen von Interesse kodierten Proteins von Interesse umfasst, und/oder wobei das heterologe Polypeptid des Konstrukts aus gespaltenem Intein und heterologem Polynukleotid ein Antibiotikaresistenzgen ist, und wobei das Verfahren alternativ zu Schritt (ii) oder zusätzlich zu Schritt (ii) das Nachweisen der Antibiotikaresistenz der Zellen von Interesse umfasst, die das durch das Gen von Interesse kodierte Protein von Interesse umfassen, und/oder wobei das Verfahren alternativ zu Schritt (ii) oder zusätzlich zu Schritt (ii) das Nachweisen eines von der Isoform abhängigen Zelloberflächenmarkers umfasst, und/oder wobei das Verfahren ferner (iii) die Manipulation des Faltungsprozesses des durch das Gen von Interesse kodierten Proteins von Interesse umfasst, und/oder wobei das Verfahren ferner (iii) die Manipulation der Kinetik des Spleißereignisses des Gens von Interesse umfasst, vorzugsweise wobei die Kinetik des spezifischen Spleißereignisses aufgrund von Schritt (ii) manipuliert wird, und/oder wobei das Verfahren ferner (iii) die Anreicherung von Zellen umfasst, die das durch das Gen von Interesse kodierte Protein von Interesse umfassen, vorzugsweise die Anreicherung von Zellen, die eine spezifische Isoform des Proteins von Interesse umfassen, und/oder wobei das Verfahren ferner (iii) die Modifikation des Faltungsprozesses des Proteins von Interesse umfasst, und/oder wobei das Verfahren ferner (iii) die Quantifizierung des durch das Gen von Interesse kodierten Proteins von Interesse oder die Quantifizierung des Exons von Interesse umfasst.

8. Verfahren nach einem der vorhergehenden Ansprüche, wobei das Verfahren ferner (iii) die Identifizierung eines Regulators der Inklusion oder Exklusion des Exons von Interesse umfasst, vorzugsweise die Identifizierung eines Regulators der Inklusion oder Exklusion des Exons von Interesse einer prä-mRNA, vorzugsweise wobei der Regulator vorzugsweise das alternative Spleißen eines nicht-konstitutiven Exons reguliert, und/oder vorzugsweise wobei das Verfahren ferner die Anwendung einer CRISPR-Bibliothek oder cDNA-Bibliothek umfasst, und/oder vorzugsweise wobei das Verfahren ferner (iv) die Inaktivierung oder Aktivierung des Regulators, vorzugsweise die Inaktivierung des Regulators, noch bevorzugter die Inaktivierung des Regulators durch eine toxische Verbindung umfasst, wobei die toxische Verbindung aus der Gruppe, bestehend aus Puromycin, Blasticidin-S, Neomycin, Hygromycin und Derivaten davon und Prodrugs/Toxinen, bevorzugter Ganciclovir, Acyclovir oder Derivaten davon ausgewählt ist, bevorzugter wobei das Verfahren ferner (v) den Nachweis des Überlebens der Zelle umfasst, die das durch das Gen von Interesse kodierte Protein von Interesse umfasst, noch bevorzugter wobei das Überleben der Zelle durch Anwendung toxischer Verbindungen nachgewiesen wird, noch bevorzugter wobei die toxische Verbindung aus der Gruppe, bestehend aus Puromycin, Blasticidin-S, Neomycin, Hygromycin und Derivaten davon und Prodrugs/Toxinen, noch bevorzugter Ganciclovir, Acyclovir oder Derivaten davon ausgewählt ist.

9. Verfahren nach einem der vorhergehenden Ansprüche, wobei die N-terminale Spleißregion des gespaltenen Inteins die N-terminale Region NrdJ-1 (SEQ ID NO: 1) oder die N-terminale Region gp41-1 (SEQ ID NO: 2) umfasst oder daraus besteht, und/oder wobei die C-terminale Spleißregion des gespaltenen Inteins die C-terminale Region NrdJ-1 (SEQ ID NO: 3) oder die C-terminale Region gp41-1 (SEQ ID NO: 4) umfasst oder daraus besteht, und/oder wobei das gespaltene Intein gp41-1 oder NrdJ-1 ist.

10. Nukleinsäure, kodierend für ein Konstrukt aus gespaltenem Intein und heterologem Polynukleotid, wobei das gespaltene Intein eine N-terminale Spleißregion stromaufwärts des heterologen Polynukleotids und eine C-terminale Spleißregion stromabwärts des heterologen Polynukleotids umfasst,
wobei das heterologe Polynukleotid für ein Protein oder Enzym kodiert, das aus der Gruppe, bestehend aus einem fluoreszierenden Protein, vorzugsweise dem grün fluoreszierenden Protein; einem Biolumineszenz erzeugenden Enzym, vorzugsweise NanoLuc-, NanoKAZ-, Cypridina-, Leuchtkäfer-, Renilla-Luciferase oder mutierte Derivate davon; einem Enzym, das ein farbiges Pigment erzeugen kann, vorzugsweise Tyrosinase oder ein Enzym eines multi-enzymatischen Prozesses, bevorzugter des Violacein- oder Betanidin-Syntheseprozesses, einem genetisch kodierten Rezeptor für multimodale Kontrastmittel, vorzugsweise Avidin, Streptavidin oder HaloTag oder mutierte Derivate davon; einem Enzym, das ein Nicht-Reportermolekül in ein Reportermolekül umwandeln kann, vorzugsweise TEV-Protease und picornavirale Proteasen, bevorzugter rhinovirale 3C-Proteasen und poliovirale 3C-Protease, SUMO-Proteasen und mutierte Derivate davon; einem Enzym, das eine toxische Verbindung inaktivieren kann, vorzugsweise Blasticidin-S-Deaminase, Puromycin-N-Acetyltransferase, Neomycin-Phosphotransferase, Hygromycin-B-Phosphotransferase und mutierte Derivate davon; einem Enzym, das Prodrug/Toxinvermittelte Toxizität umwandeln kann, vorzugsweise Thymidin-Kinase und mutierte Derivate davon, und einem niedermolekularen Sensorprotein, vorzugsweise Calmodulin, Troponin C, S100 und mutierte Derivate davon, ausgewählt ist, vorzugsweise wobei die Nukleinsäure eine der SEQ ID NO: 5 bis 22 umfasst oder daraus besteht.

11. Vektor, umfassend die Nukleinsäure nach Anspruch 10.

12. Wirtszelle, umfassend die Nukleinsäure nach Anspruch 10 oder den Vektor nach Anspruch 11.

13. Verwendung der Nukleinsäure nach Anspruch 10, des Vektors nach Anspruch 11 oder der Wirtszelle nach Anspruch 12 zum Nachweisen von Spleißereignissen, vorzugsweise wobei die Nukleinsäure, der Vektor oder die Wirtszelle zusätzlich zum Anreichern von Zellen dient.

14. Nukleinsäure nach Anspruch 10, Vektor nach Anspruch 11 oder Wirtszelle nach Anspruch 12 zur Verwendung bei der Behandlung oder Vorbeugung einer Erkrankung, wobei die Erkrankung vorzugsweise aus der Gruppe, bestehend aus Retinopathien, Tauopathien, Motoneuronenerkrankungen, Muskelerkrankungen, Neuroentwicklungs- und neurodegenerativen Erkrankungen, bevorzugter aus der Gruppe, bestehend aus zystischer Fibrose, Retinitis pigmentosa, myotoner Dystrophie, Morbus Alzheimer und Morbus Parkinson ausgewählt ist.

15. Kit zum Nachweisen eines spezifischen Spleißereignisses eines Gens von Interesse, umfassend:
- ein erstes Plasmid, in das ein Konstrukt aus gespaltenem Intein und heterologem Polynukleotid insertiert wird, und wobei das gespaltene Intein eine N-terminale Spleißregion stromaufwärts des heterologen Polynukleotids und eine C-terminale Spleißregion stromabwärts des heterologen Polynukleotids umfasst;
- ein zweites Plasmid, das für eine geführte Endonuklease kodiert, vorzugsweise wobei die Endonuklease aus der Gruppe bestehend aus Cas9, Cas12a, TALENs, ZFNs und Meganukleasen ausgewählt ist; und
- ein drittes Plasmid, das für Cre/Flp-Rekombinasen kodiert.

## Revendications

1. Procédé pour détecter un événement d'épissage spécifique d'un gène d'intérêt,
dans lequel l'événement d'épissage spécifique crée un produit d'épissage spécifique, qui comprend un exon d'intérêt,
dans lequel le procédé comprend :
(i) l'insertion d'une construction d'intéine divisée - polynucléotide hétérologue dans l'exon d'intérêt,
l'intéine divisée comprenant une région d'épissage N-terminale en amont du polynucléotide hétérologue et une région d'épissage C-terminale en aval du polynucléotide hétérologue ; et
(ii) la détection du polynucléotide hétérologue et du produit d'expression du polynucléotide hétérologue,
le produit d'expression de la construction d'intéine divisée - polynucléotide hétérologue s'excisant lui-même du produit d'expression du produit d'épissage spécifique à une position, l'acide aminé C-terminal à cette position étant une cystéine, une sérine ou une thréonine.

2. Procédé selon la revendication 1, dans lequel le produit d'expression du produit d'épissage spécifique est une chaîne polypeptidique unique, et/ou dans lequel le produit d'expression de la région d'épissage N-terminale de l'intéine divisée comprend à sa terminaison N une cystéine ou une sérine, et/ou dans lequel le produit d'expression de la région d'épissage C-terminale de l'intéine divisée comprend à sa terminaison C une asparagine, et/ou dans lequel le polynucléotide hétérologue code pour une protéine ou une enzyme choisie dans le groupe constitué par une protéine fluorescente, de préférence la protéine fluorescente verte ; une enzyme génératrice de bioluminescence, de préférence la luciférase NanoLuc, NanoKAZ, de Cypridina, de luciole, de Renilla ou des dérivés mutants de celles-ci ; une enzyme qui est capable de générer un pigment coloré, de préférence la tyrosinase ou une enzyme d'un processus multi-enzymatique, plus préférablement le processus de synthèse de la violacéine ou de la bétanidine, un récepteur génétiquement codé pour des agents de contraste multimodaux, de préférence l'avidine, la streptavidine ou HaloTag ou des dérivés mutants de celles-ci ; une enzyme, qui est capable de convertir une molécule non rapporteuse en une molécule rapporteuse, de préférence la protéase TEV et les protéases picornavirales, plus préférablement les protéases 3C rhinovirales et la protéase 3C poliovirale, les protéases SUMO et des dérivés mutants de celles-ci ; une enzyme, qui est capable d'inactiver un composé toxique, de préférence la blasticidine-S-déaminase, la puromycine-N-acétyltransférase, la néomycine phosphotransférase, l'hygromycine B phosphotransférase et des dérivés mutants de celles-ci, une enzyme, qui est capable de convertir la toxicité médiée par les promédicaments/toxines, de préférence la thymidine kinase et des dérivés mutants de celle-ci, et une protéine capteur de petites molécules, de préférence la calmoduline, la troponine C, la S100 et des dérivés mutants de celles-ci.

3. Procédé selon l'une quelconque des revendications précédentes, dans lequel la construction d'intéine divisée - polynucléotide hétérologue contient en outre au moins un polynucléotide codant pour un domaine d'hétérodimérisation ou un domaine d'homodimérisation, de préférence au moins un domaine PDZ ou au moins un domaine de superhélice, plus préférablement deux domaines de superhélice dans une configuration antiparallèle, pour accélérer l'événement d'épissage spécifique, et/ou dans lequel la construction d'intéine divisée - polynucléotide hétérologue contient en outre au moins un polynucléotide codant pour un domaine d'hétérodimérisation ou un domaine d'homodimérisation, de préférence au moins un domaine PDZ ou au moins un domaine de superhélice, plus préférablement deux domaines de superhélice dans une configuration antiparallèle, pour accélérer l'auto-excision du produit d'expression de la construction d'intéine divisée - polynucléotide hétérologue à partir du produit d'expression du produit d'épissage spécifique, et/ou dans lequel le polynucléotide hétérologue de la construction d'intéine divisée - polynucléotide hétérologue contient en outre un marqueur de sélection temporaire pour la génération d'une lignée cellulaire stable, et/ou dans lequel la détection du polynucléotide hétérologue et du produit d'expression du polynucléotide hétérologue est effectuée par toute méthode choisie dans le groupe constitué par le criblage à haut débit, le transfert Western, la spectrométrie de masse, les essais à la luciférase et l'imagerie longitudinale en direct, de préférence l'imagerie par bioluminescence, l'imagerie par fluorescence, l'imagerie photoacoustique, l'IRM et la TEP.

4. Procédé selon l'une quelconque des revendications précédentes, dans lequel le procédé est non ou minimalement invasif pour la protéine d'intérêt de telle sorte qu'une protéine d'intérêt native et/ou pleinement fonctionnelle est exprimée en comparaison de la protéine d'intérêt sans insertion de la construction d'intéine divisée - polynucléotide hétérologue conformément au procédé selon l'une quelconque des revendications 1 à 3.

5. Procédé selon l'une quelconque des revendications précédentes, dans lequel un autre polynucléotide hétérologue codant pour une enzyme rapporteuse, qui est de préférence choisie dans le groupe constitué par une protéine fluorescente, une enzyme génératrice de bioluminescence, plus préférablement une enzyme luciférase, est inséré dans un exon exprimé de manière constitutive du gène d'intérêt, dans lequel ledit autre polynucléotide hétérologue codant pour une enzyme rapporteuse est différent du polynucléotide hétérologue tel que défini dans l'une quelconque des revendications 1 à 4.

6. Procédé selon la revendication 5, dans lequel la construction d'intéine divisée - polynucléotide hétérologue comprend en outre un polynucléotide codant pour une protéine qui fonctionne comme un activateur de l'autre polynucléotide hétérologue codant pour une enzyme rapporteuse ou comme un activateur du polynucléotide hétérologue de la construction d'intéine divisée - polynucléotide hétérologue.

7. Procédé selon l'une quelconque des revendications précédentes, dans lequel le procédé comprend en outre (iii) la quantification d'une population d'isoformes de la protéine d'intérêt codée par le gène d'intérêt, et/ou dans lequel le polypeptide hétérologue de la construction d'intéine divisée - polynucléotide hétérologue est un gène de résistance à des antibiotiques et dans lequel le procédé, au lieu de l'étape (ii) ou en plus de l'étape (ii), comprend la détection de la résistance à des antibiotiques des cellules d'intérêt comprenant la protéine d'intérêt codée par le gène d'intérêt, et/ou dans lequel le procédé, au lieu de l'étape (ii) ou en plus de l'étape (ii), comprend la détection d'un marqueur de surface cellulaire dépendant de l'isoforme, et/ou dans lequel le procédé comprend en outre (iii) la manipulation du processus de repliement de la protéine d'intérêt codée par le gène d'intérêt, et/ou dans lequel le procédé comprend en outre (iii) la manipulation de la cinétique de l'événement d'épissage du gène d'intérêt, de préférence dans lequel la cinétique de l'événement d'épissage spécifique est manipulée en raison de l'étape (ii), et/ou dans lequel le procédé comprend en outre (iii) l'enrichissement de cellules comprenant la protéine d'intérêt codée par le gène d'intérêt, de préférence l'enrichissement de cellules comprenant une isoforme spécifique de la protéine d'intérêt, et/ou dans lequel le procédé comprend en outre (iii) la modification du processus de repliement de la protéine d'intérêt, et/ou dans lequel le procédé comprend en outre (iii) la quantification de la protéine d'intérêt codée par le gène d'intérêt ou la quantification de l'exon d'intérêt.

8. Procédé selon l'une quelconque des revendications précédentes, dans lequel le procédé comprend en outre (iii) l'identification d'un régulateur de l'inclusion ou de l'exclusion de l'exon d'intérêt, de préférence l'identification d'un régulateur de l'inclusion ou de l'exclusion de l'exon d'intérêt d'un pré-ARNm, de préférence dans lequel le régulateur régule l'épissage alternatif d'un exon non-constitutif, et/ou de préférence dans lequel le procédé comprend en outre l'application d'une bibliothèque CRISPR ou d'une bibliothèque d'ADNc, et/ou de préférence dans lequel le procédé comprend en outre (iv) l'inactivation ou l'activation du régulateur, plus préférablement l'inactivation du régulateur, encore plus préférablement l'inactivation du régulateur par un composé toxique, dans lequel le composé toxique est choisi dans le groupe constitué par la puromycine, la blasticidine-S, la néomycine, l'hygromycine et des dérivés de celles-ci et des promédicaments/toxines, plus préférablement le ganciclovir, l'acyclovir ou des dérivés de ceux-ci, plus préférablement dans lequel le procédé comprend en outre (v) la détection de la survie de la cellule comprenant la protéine d'intérêt codée par le gène d'intérêt, encore plus préférablement dans lequel la survie de la cellule est détectée par application de composés toxiques, encore plus préférablement dans lequel le composé toxique est choisi dans le groupe constitué par la puromycine, la blasticidine-S, la néomycine, l'hygromycine et des dérivés de celles-ci et des promédicaments/toxines, encore plus préférablement le ganciclovir, l'acyclovir ou des dérivés de ceux-ci.

9. Procédé selon l'une quelconque des revendications précédentes, dans lequel la région d'épissage N-terminale de l'intéine divisée comprend ou consiste en la région N-terminale de NrdJ-1 (SEQ ID NO : 1) ou la région N-terminale de gp41-1 (SEQ ID NO : 2), et/ou dans lequel la région d'épissage C-terminale de l'intéine divisée comprend ou consiste en la région C-terminale de NrdJ-1 (SEQ ID NO : 3) ou la région C-terminale de gp41-1 (SEQ ID NO : 4), et/ou dans lequel l'intéine divisée est gp41-1 ou NrdJ-1.

10. Acide nucléique codant pour une construction d'intéine divisée - polynucléotide hétérologue, dans lequel l'intéine divisée comprend une région d'épissage N-terminale en amont du polynucléotide hétérologue et une région d'épissage C-terminale en aval du polynucléotide hétérologue,
dans lequel le polynucléotide hétérologue code pour une protéine ou une enzyme choisie dans le groupe constitué par une protéine fluorescente, de préférence la protéine fluorescente verte ; une enzyme génératrice de bioluminescence, de préférence la luciférase NanoLuc, NanoKAZ, de Cypridina, de luciole, de Renilla ou des dérivés mutants de celles-ci ; une enzyme, qui est capable de générer un pigment coloré, de préférence la tyrosinase ou une enzyme d'un processus multi-enzymatique, plus préférablement le processus de synthèse de la violacéine ou de la bétanidine ; un récepteur génétiquement codé pour des agents de contraste multimodaux, de préférence l'avidine, la streptavidine ou HaloTag ou des dérivés mutants de celles-ci ; une enzyme, qui est capable de convertir une molécule non rapporteuse en une molécule rapporteuse, de préférence la protéase TEV et les protéases picornavirales, plus préférablement les protéases 3C rhinovirales et la protéase 3C poliovirale, les protéases SUMO et des dérivés mutants de celles-ci ; une enzyme, qui est capable d'inactiver un composé toxique, de préférence la blasticidine-S-déaminase, la puromycine-N-acétyltransférase, la néomycine phosphotransférase, l'hygromycine B phosphotransférase et des dérivés mutants de celles-ci, une enzyme, qui est capable de convertir la toxicité médiée par les promédicaments/toxines, de préférence la thymidine kinase et des dérivés mutants de celle-ci, et une protéine capteur de petites molécules, de préférence la calmoduline, la troponine C, la S100 et des dérivés mutants de celles-ci, de préférence dans lequel l'acide nucléique comprend ou consiste en l'une quelconque des SEQ ID NO : 5 à 22.

11. Vecteur comprenant l'acide nucléique selon la revendication 10.

12. Cellule hôte comprenant l'acide nucléique selon la revendication 10 ou le vecteur selon la revendication 11.

13. Utilisation de l'acide nucléique selon la revendication 10, du vecteur selon la revendication 11 ou de la cellule hôte selon la revendication 12 pour détecter des événements d'épissage, de préférence dans laquelle l'acide nucléique, le vecteur ou la cellule hôte est en outre destiné(e) à l'enrichissement de cellules.

14. Acide nucléique selon la revendication 10, vecteur selon la revendication 11 ou cellule hôte selon la revendication 12 pour utilisation dans le traitement ou la prévention d'une maladie, la maladie étant de préférence choisie dans le groupe constitué par les rétinopathies, les tauopathies, les maladies des neurones moteurs, les maladies musculaires, les maladies neurodéveloppementales et neurodégénératives, plus préférablement dans le groupe constitué par la mucoviscidose, la rétinite pigmentaire, la dystrophie myotonique, la maladie d'Alzheimer et la maladie de Parkinson.

15. Kit pour détecter un événement d'épissage spécifique d'un gène d'intérêt, comprenant :
- un premier plasmide, dans lequel une construction d'intéine divisée - polynucléotide hétérologue est insérée et l'intéine divisée comprenant une région d'épissage N-terminale en amont du polynucléotide hétérologue et une région d'épissage C-terminale en aval du polynucléotide hétérologue ;
- un deuxième plasmide codant pour une endonucléase guidée, de préférence l'endonucléase étant choisie dans le groupe constitué par Cas9, Cas12a, les TALEN, les ZFN et les méganucléases ; et
- un troisième plasmide codant pour les recombinases Cre/Flp.
